# EUROPÄISCHE PATENTSCHRIFT

(11) **EP 1 280 798 B1**
(45) Veröffentlichungstag und Bekanntmachung des Hinweises auf die Patenterteilung: **03.01.2007**
(21) Anmeldenummer: 01938076.5
(22) Anmeldetag: 31.03.2001
(51) Int. Cl.: C07D 405/12, C07D 405/14, C07D 491/10, C07D 498/10, A61K 31/517, A61K 31/47, C07D 498/04, C07D 413/14, A61P 35/00

(54) **BICYCLISCHE HETEROCYCLEN, DIESE VERBINDUNGEN ENTHALTENDE ARZNEIMITTEL, DEREN VERWENDUNG UND VERFAHREN ZU IHRER HERSTELLUNG**
BICYCLIC HETEROCYLCES, MEDICAMENTS CONTAINING SAID COMPOUNDS, THE USE THEREOF AND METHOD FOR PRODUCING THEM
HETEROCYCLES BICYCLIQUES, MEDICAMENTS CONTENANT CES COMPOSES, LEUR UTILISATION ET LEUR PROCEDE DE PRODUCTION

(30) Priorität: 08.04.2000 DE 10017539; 18.08.2000 DE 10040525
(43) Veröffentlichungstag der Anmeldung: 05.02.2003
(73) Patentinhaber: Boehringer Ingelheim Pharma GmbH & Co.KG, 55218 Ingelheim am Rhein (DE)
(72) Erfinder: HIMMELSBACH, Frank, 88441 Mittelbiberach (DE); LANGKOPF, Elke, 88447 Warthausen (DE); JUNG, Birgit, 55270 Schwabenheim (DE); BLECH, Stefan, 88447 Warthausen (DE); SOLCA, Flavio, A-1230 Wien (AT)
(86) Internationale Anmeldenummer: PCT/EP2001/003694
(87) Internationale Veröffentlichungsnummer: WO 2001/077104

(56) Entgegenhaltungen:
- WO-A-00/18740
- WO-A-97/38983
- WO-A-98/43960
- WO-A-99/06396
- WO-A-99/09016

## Beschreibung

Gegenstand der vorliegenden Erfindung sind bicyclische Heterocyclen der allgemeinen Formel 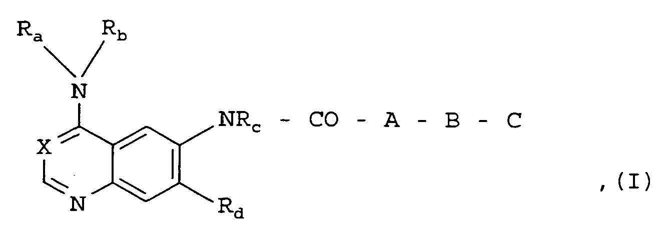 deren Tautomeren, deren Stereoisomere und deren Salze, insbesonders deren physiologisch verträgliche Salze mit anorganischen oder organischen Säuren oder Basen, welche wertvolle pharmakologische Eigenschaften aufweisen, insbesondere eine Hemmwirkung auf die durch Tyrosinkinasen vermittelte Signaltransduktion, deren Verwendung zur Behandlung von Krankheiten, insbesondere von Tumorerkrankungen, von Erkrankungen der Lunge und der Atemwege und deren Herstellung.

In der obigen allgemeinen Formel I bedeutet
X eine durch eine Cyanogruppe substituierte Methingruppe oder ein Stickstoffatom,
Rₐ ein Wasserstoffatom oder eine C₁₋₄-Alkylgruppe,
R_{b} eine Phenyl-, Benzyl- oder 1-Phenylethylgruppe, in denen der Phenylkern jeweils durch die Reste R₁ bis R₃ substituiert ist, wobei
   R₁ und R₂, die gleich oder verschieden sein können, jeweils ein Wasserstoff-, Fluor-, Chlor-, Brom- oder Jodatom,
   eine C₁₋₄-Alkyl-, Hydroxy-, C₁₋₄-Alkoxy-, C₃₋₆-Cycloalkyl-, C₄₋₆-Cycloalkoxy-, C₂₋₅-Alkenyl- oder C₂₋₅-Alkinylgruppe,
   eine Aryl-, Aryloxy-, Arylmethyl- oder Arylmethoxygruppe,
   eine C₃₋₅-Alkenyloxy- oder C₃₋₅-Alkinyloxygruppe, wobei der ungesättigte Teil nicht mit dem Sauerstoffatom verknüpft sein kann,
   eine C₁₋₄-Alkylsulfenyl-, C₁₋₄-Alkylsulfinyl-, C₁₋₄-Alkylsulfonyl-, C₁₋₄-Alkylsulfonyloxy-, Trifluormethylsulfenyl-, Trifluormethylsulfinyl- oder Trifluormethylsulfonylgruppe,
   eine durch 1 bis 3 Fluoratome substituierte Methyl- oder Methoxygruppe,
   eine durch 1 bis 5 Fluoratome substituierte Ethyl- oder Ethoxygruppe,
   eine Cyano- oder Nitrogruppe oder eine gegebenenfalls durch eine oder zwei C₁₋₄-Alkylgruppen substituierte Aminogruppe, wobei die Substituenten gleich oder verschieden sein können, oder
   R₁ zusammen mit R₂, sofern diese an benachbarte Kohlenstoffatome gebunden sind, eine -CH=CH-CH=CH-, -CH=CH-NH- oder -CH=N-NH-Gruppe und
   R₃ ein Wasserstoff-, Fluor-, Chlor- oder Bromatom,
   eine C₁₋₄-Alkyl-, Trifluormethyl- oder C₁₋₄-Alkoxygruppe darstellen,
R_{c} ein Wasserstoffatom oder eine C₁₋₄-Alkylgruppe,
R_{d} ein Wasserstoffatom, eine C₁₋₆-Alkoxy-, C₄₋₇-Cycloalkoxy- oder C₃₋₇-Cycloalkyl-C₁₋₄-alkoxygruppe,
eine C₂₋₆-Alkoxygruppe, die ab Position 2 durch eine Hydroxy-, C₁₋₄-Alkoxy-, C₄₋₇-Cycloalkoxy-, C₃₋₇-Cycloalkyl-C₁₋₃-alkoxy-, Di-(C₁₋₄-alkyl)-amino-, Pyrrolidino-, Piperidino-, Morpholino-, Piperazino- oder 4-(C₁₋₄-Alkyl)-piperazinogruppe substituiert ist, wobei die vorstehend erwähnten cyclischen Iminogruppen durch eine oder zwei C₁₋₂-Alkylgruppen substituiert sein können,
eine Tetrahydrofuran-3-yloxy-, Tetrahydropyran-3-yloxy-, Tetrahydropyran-4-yloxy-, Tetrahydrofuranylmethoxy- oder Tetrahydropyranylmethoxygruppe,
A eine gegebenenfalls durch eine Methyl- oder Trifluormethylgruppe oder durch zwei Methylgruppen substituierte 1,1- oder 1,2-Vinylengruppe,
eine gegebenenfalls durch eine Methyl- oder Trifluormethylgruppe oder durch zwei Methylgruppen substituierte 1,3-Butadien-1,4-ylengruppe oder eine Ethinylengruppe,
B eine C₁₋₆-Alkylengruppe, in der ein oder zwei Wasserstoffatome durch Fluoratome ersetzt sein können, oder, falls B an ein Kohlenstoffatom der Gruppe C gebunden ist, auch eine Bindung,
C eine Pyrrolidinogruppe, in der die beiden Wasserstoffatome in 2-Stellung durch eine Gruppe D ersetzt sind, in der
   D eine gegebenenfalls durch eine oder zwei C₁₋₂-Alkylgruppen substituierte -CH₂-O-CO-CH₂-, -CH₂CH₂-O-CO-, -CH₂-O-CO-CH₂CH₂-, -CH₂CH₂-O-CO-CH₂- oder -CH₂CH₂CH₂-O-CO-Brücke darstellt,
eine Pyrrolidinogruppe, in der die beiden Wasserstoffatome in 3-Stellung durch eine Gruppe E ersetzt sind, in der
   E eine gegebenenfalls durch eine oder zwei C₁₋₂-Alkylgruppen substituierte -O-CO-CH₂CH₂-, -CH₂-O-CO-CH₂-, -CH₂CH₂-O-CO-, -O-CO-CH₂CH₂CH₂-, -CH₂-O-CO-CH₂CH₂-, -CH₂CH₂-O-CO-CH₂-, -CH₂CH₂CH₂-O-CO-, -O-CO-CH₂-NR₄-CH₂-, -CH₂-O-CO-CH₂-NR₄-, -O-CO-CH₂-O-CH₂- oder -CH₂-O-CO-CH₂-O-Brücke darstellt,
   wobei R₄ ein Wasserstoffatom oder eine C₁₋₄-Alkylgruppe bedeutet,
eine Piperidino- oder Hexahydroazepinogruppe, in denen die beiden Wasserstoffatome in 2-Stellung durch eine Gruppe D ersetzt sind, wobei D wie vorstehend erwähnt definiert ist,
eine Piperidino- oder Hexahydroazepinogruppe, in denen jeweils die beiden Wasserstoffatome in 3-Stellung oder in 4-Stellung durch eine Gruppe E ersetzt sind, wobei E wie vorstehend erwähnt definiert ist,
eine Piperazino- oder 4-(C₁₋₄-Alkyl)-piperazinogruppe, in denen die beiden Wasserstoffatome in 2-Stellung oder in 3-Stellung des Piperazinoringes durch eine Gruppe D ersetzt sind, wobei D wie vorstehend erwähnt definiert ist,
eine Pyrrolidino- oder Piperidinogruppe, in denen zwei vicinale Wasserstoffatome durch eine gegebenenfalls durch eine oder zwei C₁₋₂-Alkylgruppen substituierte -O-CO-CH₂-, -CH₂-O-CO-, -O-CO-CH₂CH₂-, -CH₂-O-CO-CH₂-, -CH₂CH₂-O-CO-, -O-CO-CH₂-NR₄- oder -O-CO-CH₂-O-Brücke ersetzt sind, wobei R₄ wie vorstehend erwähnt definiert ist und die Heteroatome der vorstehend erwähnten Brücken nicht an die 2- oder 5-Stellung des Pyrrolidinringes und nicht an die 2- oder 6-Stellung des Piperidinoringes gebunden sind,
eine Piperazino- oder 4-(C₁₋₄-Alkyl)-piperazinogruppe, in denen ein Wasserstoffatom in 2-Stellung zusammen mit einem Wasserstoffatom in 3-Stellung des Piperazinoringes durch eine gegebenenfalls durch eine oder zwei C₁₋₂-Alkylgruppen substituierte -CH₂-O-CO-CH₂- oder -CH₂CH₂-O-CO-Brücke ersetzt sind,
eine Piperazinogruppe, in der ein Wasserstoffatom in 3-Stellung zusammen mit dem Wasserstoffatom in 4-Stellung durch eine gegebenenfalls durch eine oder zwei C₁₋₂-Alkylgruppen substituierte -CO-O-CH₂CH₂- oder -CH₂-O-CO-CH₂-Brücke ersetzt sind, wobei jeweils das linke Ende der vorstehend erwähnten Brücken an die 3-Stellung des Piperazinoringes gebunden ist,
eine durch den Rest R₅ substituierte Pyrrolidino-, Piperidino- oder Hexahydroazepinogruppe, in denen
   R₅ eine gegebenenfalls durch eine oder zwei C₁₋₂-Alkylgruppen substituierte 2-Oxo-tetrahydrofuranyl-, 2-Oxo-tetrahydropyranyl-, 2-Oxo-1,4-dioxanyl- oder 2-Oxo-4-(C₁₋₄-alkyl)-morpholinylgruppe darstellt,
eine in 3-Stellung durch eine 2-Oxo-morpholinogruppe substituierte Pyrrolidinogruppe, wobei die 2-Oxo-morpholinogruppe durch eine oder zwei C₁₋₂-Alkylgruppen substituiert sein kann,
eine in 3- oder 4-Stellung durch eine 2-Oxo-morpholinogruppe substituierte Piperidino- oder Hexahydroazepinogruppe, wobei die 2-Oxo-morpholinogruppe durch eine oder zwei C₁₋₂-Alkylgruppen substituiert sein kann,
eine an einem Ringkohlenstoffatom durch R₅ substituierte 4-(C₁₋₄-Alkyl)-piperazino- oder 4-(C₁₋₄-Alkyl)-homopiperazinogruppe, in denen R₅ wie vorstehend erwähnt definiert ist,
eine in 4-Stellung durch den Rest R₆ substituierte Piperazino- oder Homopiperazinogruppe, in denen
   R₆ eine gegebenenfalls durch eine oder zwei C₁₋₂-Alkylgruppen substituierte 2-Oxo-tetrahydrofuran-3-yl-, 2-Oxo-tetrahydrofuran-4-yl-, 2-Oxo-tetrahydropyran-3-yl-, 2-Oxo-tetrahydropyran-4-yl- oder 2-Oxo-tetrahydropyran-5-yl-Gruppe darstellt,
eine in 3-Stellung durch eine (R₄NR₆)-, R₆O-, R₆S-, R₆SO- oder R₆SO₂-Gruppe substituierte Pyrrolidinogruppe, wobei R₄ und R₆ wie vorstehend erwähnt definiert sind,
eine in 3- oder 4-Stellung durch eine (R₄NR₆)-, R₆O-, R₆S-, R₆SO- oder R₆SO₂-Gruppe substituierte Piperidino- oder Hexahydroazepinogruppe, in denen R₄ und R₆ wie vorstehend erwähnt definiert sind,
eine durch eine R₅-C₁₋₄-alkyl-, (R₄NR₆)-C₁₋₄-alkyl-, R₆O-C₁₋₄-alkyl-, R₆S-C₁₋₄-alkyl-, R₆SO-C₁₋₄-alkyl-, R₆SO₂-C₁₋₄-alkyl- oder R₄NR₆-CO-Gruppe substituierte Pyrrolidino-, Piperidino- oder Hexahydroazepinogruppe, in denen R₄ bis R₆ wie vorstehend erwähnt definiert sind,
eine in 3-Stellung durch eine R₅-CO-NR₄-, R₅-C₁₋₄-alkylen-CONR₄-, (R₄NR₆)-C₁₋₄-alkylen-CONR₄-, R₆O-C₁₋₄-alkylen-CONR₄-, R₆S-C₁₋₄-alkylen-CONR₄-, R₆SO-C₁₋₄-alkylen-CONR₄-, R₆SO₂-C₁₋₄-alkylen-CONR₄-, 2-Oxo-morpholino-C₁₋₄-alkylen-CONR₄-, R₅-C₁₋₄-alkylen-Y- oder C₂₋₄-Alkyl-Y-Gruppe substituierte Pyrrolidinogruppe, wobei der C₂₋₄-Alkylteil der C₂₋₄-Alkyl-Y-Gruppe jeweils ab Position 2 durch eine (R₄NR₆)-, R₆O-, R₆S-, R₆SO- oder R₆SO₂-Gruppe substituiert ist und der 2-Oxo-morpholinoteil durch eine oder zwei C₁₋₂-Alkylgruppen substituiert sein kann, in denen
   R₄ bis R₆ wie vorstehend erwähnt definiert sind und
   Y ein Sauerstoff- oder Schwefelatom, eine Imino-, N-(C₁₋₄-Alkyl)-imino-, Sulfinyl- oder Sulfonylgruppe darstellt,
eine in 3- oder 4-Stellung durch eine R₅-CO-NR₄-, R₅-C₁₋₄-alkylen-CONR₄-, (R₄NR₆)-C₁₋₄-alkylen-CONR₄-, R₆O-C₁₋₄-alkylen-CONR₄-, R₆S-C₁₋₄-alkylen-CONR₄-, R₆SO-C₁₋₄-alkylen-CONR₄-, R₆SO₂C₁₋₄-alkylen-CONR₄-, 2-Oxo-morpholino-C₁₋₄-alkylen-CONR₄-, R₅-C₁₋₄-alkylen-Y- oder C₂₋₄-Alkyl-Y-Gruppe substituierte Piperidino- oder Hexahydroazepinogruppe, in denen Y wie vorstehend erwähnt definiert ist, der 2-Oxo-morpholinoteil durch eine oder zwei C₁₋₂-Alkylgruppen substituiert sein kann und der C₂₋₄-Alkylteil der C₂₋₄-Alkyl-Y-Gruppe jeweils ab Position 2 durch eine (R₄NR₆)-, R₆O-, R₆S-, R₆SO- oder R₆SO₂-Gruppe substituiert ist, wobei R₄ bis R₆ wie vorstehend erwähnt definiert sind,
eine an einem Ringkohlenstoffatom durch eine R₅-C₁₋₄-alkyl-, (R₄NR₆)-C₁₋₄-alkyl-, R₆O-C₁₋₄-alkyl-, R₆S-C₁₋₄-alkyl-, R₆SO-C₁₋₄-alkyl-, R₆SO₂-C₁₋₄-alkyl- oder R₄NR₆-CO-Gruppe substituierte 4-(C₁₋₄-Alkyl)-piperazino- oder 4-(C₁₋₄-Alkyl)-homopiperazinogruppe, in denen R₄ bis R₆ wie vorstehend erwähnt definiert sind,
eine in 4-Stellung durch eine R₅-C₁₋₄-alkyl-, R₅-CO-, R₅-C₁₋₄-alkylen-CO-, (R₄NR₆)-C₁₋₄-alkylen-CO-, R₆O-C₁₋₄-alkylen-CO-, R₆S-C₁₋₄-alkylen-CO-, R₆SO-C₁₋₄-alkylen-CO- oder R₆SO₂-C₁₋₄-alkylen-CO-Gruppe substituierte Piperazino- oder Homopiperazinogruppe, in denen R₄ bis R₆ wie vorstehend erwähnt definiert sind,
eine in 4-Stellung durch eine C₂₋₄-Alkylgruppe substituierte Piperazino- oder Homopiperazinogruppe, in denen die C₂₋₄-Alkylgruppe jeweils ab Position 2 durch eine (R₄NR₆)-, R₆O-, R₆S-, R₆SO- oder R₆SO₂-Gruppe substituiert ist, wobei R₄ und R₆ wie vorstehend erwähnt definiert sind,
eine durch eine 2-Oxo-morpholino-C₁₋₄-alkylgruppe substituierte Pyrrolidino-, Piperidino- oder Hexahydroazepinogruppe, in denen der 2-Oxo-morpholinoteil durch eine oder zwei C₁₋₂-Alkylgruppen substituiert sein kann,
eine in 3-Stellung durch eine C₂₋₄-Alkyl-Y-Gruppe substituierte Pyrrolidinogruppe, in denen Y wie vorstehend erwähnt definiert ist und der C₂₋₄-Alkylteil der C₂₋₄-Alkyl-Y-Gruppe jeweils ab Position 2 durch eine gegebenenfalls durch eine oder zwei C₁₋₂-Alkylgruppen substituierte 2-Oxo-morpholinogruppe substituiert ist,
eine in 3- oder 4-Stellung durch eine C₂₋₄-Alkyl-Y-Gruppe substituierte Piperidino- oder Hexahydroazepinogruppe, in denen Y wie vorstehend erwähnt definiert ist und der C₂₋₄-Alkylteil der C₂₋₄-Alkyl-Y-Gruppe jeweils ab Position 2 durch eine gegebenenfalls durch eine oder zwei C₁₋₂-Alkylgruppen substituierte 2-Oxo-morpholinogruppe substituiert ist,
eine an einem Ringkohlenstoffatom durch eine 2-Oxo-morpholino-C₁₋₄-alkyl-Gruppe substituierte 4-(C₁₋₄-Alkyl)-piperazino- oder 4-(C₁₋₄-Alkyl)-homopiperazinogruppe, in denen der 2-Oxo-morpholinoteil durch eine oder zwei C₁₋₂-Alkylgruppen substituiert sein kann,
eine in 4-Stellung durch eine 2-Oxo-morpholino-C₁₋₄-alkylen-CO-Gruppe substituierte Piperazino- oder Homopiperazinogruppe, in denen der 2-Oxo-morpholinoteil durch eine oder zwei C₁₋₂-Alkylgruppen substituiert sein kann,
eine in 4-Stellung durch eine C₂₋₄-Alkylgruppe substituierte Piperazino- oder Homopiperazinogruppe, in denen der C₂₋₄-Alkylteil jeweils ab Position 2 durch eine gegebenenfalls durch eine oder zwei C₁₋₂-Alkylgruppen substituierte 2-Oxo-morpholinogruppe substituiert ist,
eine in 1-Stellung durch den Rest R₆, durch eine R₅-C₁₋₄-alkyl-, R₅-CO-, R₅-C₁₋₄-alkylen-CO-, (R₄NR₆)-C₁₋₄-alkylen-CO-, R₆O-C₁₋₄-alkylen-CO-, R₆S-C₁₋₄-alkylen-CO-, R₆SO-C₁₋₄-alkylen-CO-, R₆SO₂-C₁₋₄-alkylen-CO- oder 2-Oxo-morpholino-C₁₋₄-alkylen-CO-Gruppe substituierte Pyrrolidinyl- oder Piperidinylgruppe, in denen R₄ bis R₆ wie vorstehend erwähnt definiert sind und der 2-Oxo-morpholinoteil durch eine oder zwei C₁₋₂-Alkylgruppen substituiert sein kann,
eine in 1-Stellung durch eine C₂₋₄-Alkylgruppe substituierte Pyrrolidinyl- oder Piperidinylgruppe, in denen der C₂₋₄-Alkylteil jeweils ab Position 2 durch eine (R₄NR₆)-, R₆O-, R₆S-, R₆SO-, R₆SO₂- oder 2-Oxo-morpholinogruppe substituiert ist, wobei R₄ und R₆ wie vorstehend erwähnt definiert sind und der 2-Oxo-morpholinoteil durch eine oder zwei C₁₋₂-Alkylgruppen substituiert sein kann,
eine jeweils am Ringstickstoffatom durch den Rest R₆, durch eine R₅-C₁₋₄-alkyl-, R₅-CO-, R₅-C₁₋₄-alkylen-CO-, (R₄NR₆)-C₁₋₄-alkylen-CO-, R₆O-C₁₋₄-alkylen-CO-, R₆S-C₁₋₄-alkylen-CO-, R₆SO-C₁₋₄-alkylen-CO-, R₆SO₂-C₁₋₄-alkylen-CO- oder 2-Oxo-morpholino-C₁₋₄-alkylen-CO-Gruppe substituierte Pyrrolidin-3-yl-NR₄-, Piperidin-3-yl-NR₄- oder Piperidin-4-yl-NR₄-Gruppe, in denen R₄ bis R₆ wie vorstehend erwähnt definiert sind und der 2-Oxo-morpholinoteil durch eine oder zwei C₁₋₂-Alkylgruppen substituiert sein kann,
eine jeweils am Ringstickstoffatom durch eine C₂₋₄-Alkylgruppe substituierte Pyrrolidin-3-yl-NR₄-, Piperidin-3-yl-NR₄- oder Piperidin-4-yl-NR₄-Gruppe, in denen der C₂₋₄-Alkylteil jeweils ab Position 2 durch eine (R₄NR₆)-, R₆O-, R₆S-, R₆SO-, R₆SO₂-oder 2-Oxo-morpholinogruppe substituiert ist, wobei R₄ und R₆ wie vorstehend erwähnt definiert sind und der 2-Oxo-morpholinoteil durch eine oder zwei C₁₋₂-Alkylgruppen substituiert sein kann,
eine R₅-C₁₋₄-alkylen-NR₄-Gruppe, in der R₄ und R₅ wie vorstehend erwähnt definiert sind, oder
eine C₂₋₄-Alkyl-NR₄-Gruppe, in denen der C₂₋₄-Alkylteil jeweils ab Position 2 durch eine (R₄NR₆)-, R₆O-, R₆S-, R₆SO-, R₆SO₂- oder 2-Oxo-morpholinogruppe substituiert ist, wobei R₄ und R₆ wie vorstehend erwähnt definiert sind und der 2-Oxo-morpholinoteil durch eine oder zwei C₁₋₂-Alkylgruppen substituiert sein kann, wobei
unter den vorstehend erwähnten Arylteilen eine Phenylgruppe zu verstehen ist, die jeweils durch R' mono- oder disubstituiert sein kann, wobei die Substituenten gleich oder verschieden sein können und
R' ein Fluor-, Chlor-, Brom- oder lodatom, eine C₁₋₂-Alkyl-, Trifluormethyl- oder C₁₋₂-Alkoxygruppe oder
zwei Reste R', sofern sie an benachbarte Kohlenstoffatome gebunden sind, zusammen eine C₃₋₄-Alkylen-, Methylendioxy- oder 1,3-Butadien-1,4-ylengruppe darstellen.

Bevorzugte Verbindungen der obigen allgemeinen Formel I sind diejenigen, in denen
X ein Stickstoffatom,
Rₐ ein Wasserstoffatom,
R_{b} eine Phenyl-, Benzyl- oder 1-Phenylethylgruppe, in denen der Phenylkern jeweils durch die Reste R₁ bis R₃ substituiert ist, wobei
   R₁ und R₂, die gleich oder verschieden sein können, jeweils eine Methylgruppe oder ein Wasserstoff-, Fluor-, Chlor- oder Bromatom und
   R₃ ein Wasserstoffatom darstellen,
R_{c} ein Wasserstoffatom,
R_{d} ein Wasserstoffatom, eine Methoxy-, Ethoxy-, C₄₋₆-Cycloalkoxy-, C₃₋₆-Cycloalkylmethoxy-, 2-Methoxy-ethoxy-, 2-(Cyclobutyloxy)-ethoxy-, 2-(Cyclopentyloxy)-ethoxy-, 2-(Cyclohexyloxy)-ethoxy-, 2-(Cyclopropylmethoxy)-ethoxy-, Tetrahydrofuran-3-yloxy-, Tetrahydropyran-4-yloxy-, Tetrahydrofuran-2-ylmethoxy-, Tetrahydrofuran-3-ylmethoxy-, Tetrahydropyran-2-ylmethoxy- oder Tetrahydropyran-4-ylmethoxygruppe,
A eine 1,2-Vinylengruppe,
B eine Methylen- oder Ethylengruppe oder, falls B an ein Kohlenstoffatom der Gruppe C gebunden ist, auch eine Bindung,
C eine Pyrrolidinogruppe, in der die beiden Wasserstoffatome in 3-Stellung durch eine Gruppe E ersetzt sind, in der
   E eine gegebenenfalls durch eine oder zwei Methylgruppen substituierte -O-CO-CH₂CH₂-, -CH₂-O-CO-CH₂-, -CH₂CH₂-O-CO-, -CH₂CH₂-O-CO-CH₂-, -O-CO-CH₂-NR₄-CH₂-, -CH₂-O-CO-CH₂-NR₄-, -O-CO-CH₂-O-CH₂- oder -CH₂-O-CO-CH₂-O-Brücke darstellt,
   wobei R₄ eine Methyl- oder Ethylgruppe bedeutet,
eine Piperidinogruppe, in der die beiden Wasserstoffatome in 3-Stellung oder in 4-Stellung durch eine Gruppe E ersetzt sind, wobei E wie vorstehend erwähnt definiert ist,
eine Pyrrolidino- oder Piperidinogruppe, in denen zwei vicinale Wasserstoffatome durch eine gegebenenfalls durch eine oder zwei Methylgruppen substituierte -O-CO-CH₂-, -CH₂-O-CO-, -O-CO-CH₂-NR₄- oder -O-CO-CH₂-O-Brücke ersetzt sind, wobei R₄ wie vorstehend erwähnt definiert ist und die Heteroatome der vorstehend erwähnten Brücken nicht an die 2- oder 5-Stellung des Pyrrolidinringes und nicht an die 2- oder 6-Stellung des Piperidinoringes gebunden sind,
eine Piperazinogruppe, in der ein Wasserstoffatom in 3-Stellung zusammen mit dem Wasserstoffatom in 4-Stellung durch eine gegebenenfalls durch eine oder zwei Methylgruppen substituierte -CO-O-CH₂CH₂- oder -CH₂-O-CO-CH₂-Brücke ersetzt sind, wobei jeweils das linke Ende der vorstehend erwähnten Brücken an die 3-Stellung des Piperazinoringes gebunden ist,
eine durch den Rest R₅ substituierte Pyrrolidino- oder Piperidinogruppe, in denen
   R₅ eine gegebenenfalls durch eine oder zwei Methylgruppen substituierte 2-Oxo-tetrahydrofuranyl-, 2-Oxo-1,4-dioxanyl- oder 2-Oxo-4-(C₁₋₄-alkyl)-morpholinylgruppe darstellt,
eine in 3-Stellung durch eine 2-Oxo-morpholinogruppe substituierte Pyrrolidinogruppe, wobei die 2-Oxo-morpholinogruppe durch eine oder zwei Methylgruppen substituiert sein kann,
eine in 3- oder 4-Stellung durch eine 2-Oxo-morpholinogruppe substituierte Piperidinogruppe, wobei die 2-Oxo-morpholinogruppe durch eine oder zwei Methylgruppen substituiert sein kann,
eine in 4-Stellung durch den Rest R₆ substituierte Piperazinogruppe, in der
   R₆ eine gegebenenfalls durch eine oder zwei Methylgruppen substituierte 2-Oxo-tetrahydrofuran-3-yl- oder 2-Oxo-tetrahydrofuran-4-yl-Gruppe darstellt,
eine in 3-Stellung durch eine (R₄NR₆)-, R₆O-, R₆S-, R₆SO- oder R₆SO₂-Gruppe substituierte Pyrrolidinogruppe, wobei R₄ und R₆ wie vorstehend erwähnt definiert sind,
eine in 3- oder 4-Stellung durch eine (R₄NR₆)-, R₆O-, R₆S-, R₆SO- oder R₆SO₂-Gruppe substituierte Piperidinogruppe, wobei R₄ und R₆ wie vorstehend erwähnt definiert sind,
eine durch eine (R₄NR₆)-C₁₋₂-alkyl-, HNR₆-CO- oder R₄NR₆-CO-Gruppe substituierte Pyrrolidino- oder Piperidinogruppe, wobei R₄ und R₆ wie vorstehend erwähnt definiert sind,
eine in 3-Stellung durch eine R₅-CO-NH- oder R₅-CO-NR₄-Gruppe substituierte Pyrrolidinogruppe, wobei R₄ und R₅ wie vorstehend erwähnt definiert sind,
eine in 3- oder 4-Stellung durch eine R₅-CO-NH- oder R₅-CO-NR₄-Gruppe substituierte Piperidinogruppe, wobei R₄ und R₅ wie vorstehend erwähnt definiert sind,
eine in 4-Stellung durch eine R₅-C₁₋₂-alkyl-, R₅-CO-, R₅-C₁₋₂-alkylen-CO-, (R₄NR₆)-C₁₋₂-alkylen-CO-, R₆O-C₁₋₂-alkylen-CO-, R₆S-C₁₋₂-alkylen-CO-, R₆SO-C₁₋₂-alkylen-CO- oder R₆SO₂-C₁₋₂-alkylen-CO-Gruppe substituierte Piperazinogruppe, in der R₄ bis R₆ wie vorstehend erwähnt definiert sind,
eine in 4-Stellung durch eine C₂₋₃-Alkylgruppe substituierte Piperazinogruppe, in der die C₂₋₃-Alkylgruppe jeweils ab Position 2 durch eine (R₄NR₆)-, R₆O-, R₆S-, R₆SO- oder R₆SO₂-Gruppe substituiert ist, wobei R₄ und R₆ wie vorstehend erwähnt definiert sind,
eine durch eine 2-Oxo-morpholino-C₁₋₂-alkylgruppe substituierte Pyrrolidino- oder Piperidinogruppe, in denen der 2-Oxo-morpholinoteil durch eine oder zwei Methylgruppen substituiert sein kann,
eine in 4-Stellung durch eine 2-Oxo-morpholino-C₁₋₂-alkylen-CO-Gruppe substituierte Piperazinogruppe, in der der 2-Oxo-morpholinoteil durch eine oder zwei Methylgruppen substituiert sein kann,
eine in 4-Stellung durch eine C₂₋₃-Alkylgruppe substituierte Piperazinogruppe, in der der C₂₋₃-Alkylteil jeweils ab Position 2 durch eine gegebenenfalls durch eine oder zwei Methylgruppen substituierte 2-Oxo-morpholinogruppe substituiert ist,
eine in 1-Stellung durch den Rest R₆, durch eine R₅-C₁₋₂-alkyl-, R₅-CO-, R₅-C₁₋₂-alkylen-CO-, (R₄NR₆)-C₁₋₂-alkylen-CO-, R₆O-C₁₋₂-alkylen-CO-, R₆S-C₁₋₂-alkylen-CO-, R₆SO-C₁₋₂-alkylen-CO-, R₆SO₂-C₁₋₂-alkylen-CO- oder 2-Oxo-morpholino-C₁₋₂-alkylen-CO-Gruppe substituierte Piperidinylgruppe, wobei R₄ bis R₆ wie vorstehend erwähnt definiert sind und der 2-Oxo-morpholinoteil durch eine oder zwei Methylgruppen substituiert sein kann,
eine in 1-Stellung durch eine C₂₋₃-Alkylgruppe substituierte Piperidinylgruppe, in der der C₂₋₃-Alkylteil jeweils ab Position 2 durch eine (R₄NR₆)-, R₆O-, R₆S-, R₆SO-, R₆SO₂- oder 2-Oxo-morpholinogruppe substituiert ist, wobei R₄ und R₆ wie vorstehend erwähnt definiert sind und der 2-Oxo-morpholinoteil durch eine oder zwei Methylgruppen substituiert sein kann,
eine jeweils am Ringstickstoffatom durch den Rest R₆, durch eine R₅-C₁₋₂-alkyl-, R₅-CO-, R₅-C₁₋₂-alkylen-CO-, (R₄NR₆)-C₁₋₂-alkylen-CO-, R₆O-C₁₋₂-alkylen-CO-, R₆S-C₁₋₂-alkylen-CO-, R₆SO-C₁₋₂-alkylen-CO-, R₆SO₂-C₁₋₂-alkylen-CO- oder 2-Oxo-morpholino-C₁₋₂-alkylen-CO-Gruppe substituierte Pyrrolidin-3-yl-NR₄-, Piperidin-3-yl-NR₄- oder Piperidin-4-yl-NR₄-Gruppe, in denen R₄ bis R₆ wie vorstehend erwähnt definiert sind und der 2-Oxo-morpholinoteil durch eine oder zwei Methylgruppen substituiert sein kann, oder
eine jeweils am Ringstickstoffatom durch eine C₂₋₃-Alkylgruppe substituierte Pyrrolidin-3-yl-NR₄-, Piperidin-3-yl-NR₄- oder Piperidin-4-yl-NR₄-Gruppe, in denen der C₂₋₃-Alkylteil jeweils ab Position 2 durch eine (R₄NR₆)-, R₆O-, R₆S-, R₆SO-, R₆SO₂- oder 2-Oxo-morpholinogruppe substituiert ist, wobei R₄ und R₆ wie vorstehend erwähnt definiert sind und der 2-Oxo-morpholinoteil durch eine oder zwei Methylgruppen substituiert sein kann,
deren Tautomeren, deren Stereoisomere und deren Salze.

Besonders bevorzugte Verbindungen der allgemeinen Formel I sind diejenigen, in denen
X ein Stickstoffatom,
Rₐ ein Wasserstoffatom,
R_{b} eine Phenyl-, Benzyl- oder 1-Phenylethylgruppe, in denen der Phenylkern jeweils durch die Reste R₁ bis R₃ substituiert ist, wobei
   R₁ und R₂, die gleich oder verschieden sein können, jeweils eine Methylgruppe oder ein Wasserstoff-, Fluor-, Chlor- oder Bromatom und
   R₃ ein Wasserstoffatom darstellen,
R_{c} ein Wasserstoffatom,
R_{d} ein Wasserstoffatom, eine Methoxy-, Ethoxy-, C₄₋₆-Cycloalkoxy-, C₃₋₆-Cycloalkylmethoxy-, 2-Methoxy-ethoxy-, 2-(Cyclobutyloxy)-ethoxy-, 2-(Cyclopentyloxy)-ethoxy-, 2-(Cyclohexyloxy)-ethoxy-, 2-(Cyclopropylmethoxy)-ethoxy-, Tetrahydrofuran-3-yloxy-, Tetrahydropyran-4-yloxy-, Tetrahydrofuran-2-ylmethoxy-, Tetrahydrofuran-3-ylmethoxy-, Tetrahydropyran-2-ylmethoxy- oder Tetrahydropyran-4-ylmethoxygruppe,
A eine 1,2-Vinylengruppe,
B eine Methylen- oder Ethylengruppe oder, falls B an ein Kohlenstoffatom der Gruppe C gebunden ist, auch eine Bindung,
C eine Piperidinogruppe, in der die beiden Wasserstoffatome in 4-Stellung durch eine -CH₂-O-CO-CH₂-, -CH₂CH₂-O-CO-, -CH₂CH₂-O-CO-CH₂-, -O-CO-CH₂-NCH₃-CH₂- oder -O-CO-CH₂-O-CH₂-Brücke ersetzt sind,
eine Piperazinogruppe, in der ein Wasserstoffatom in 3-Stellung zusammen mit dem Wasserstoffatom in 4-Stellung durch eine -CO-O-CH₂-CH₂- oder -CH₂-O-CO-CH₂-Brücke ersetzt sind, wobei jeweils das linke Ende der vorstehenden Brücken an die 3-Stellung des Piperazinoringes gebunden ist,
eine durch eine 2-Oxo-tetrahydrofuranylgruppe substituierte Piperidinogruppe,
eine Piperidinogruppe, die in 4-Stellung durch eine 2-Oxo-morpholino- oder 2-Oxo-morpholinomethylgruppe substituiert ist, wobei der 2-Oxo-morpholinoteil jeweils durch eine oder zwei Methylgruppen substituiert sein kann,
eine Piperazinogruppe, die in 4-Stellung durch eine 2-Oxo-tetrahydrofuran-3-yl- oder 2-Oxo-tetrahydrofuran-4-yl-Gruppe substituiert ist,
eine Piperidinogruppe, die in 4-Stellung durch eine CH₃NR₆- oder R₆S-Gruppe substituiert ist, wobei
   R₆ eine 2-Oxo-tetrahydrofuran-3-yl- oder 2-Oxo-tetrahydrofuran-4-yl-Gruppe darstellt,
eine Piperazinogruppe, die in 4-Stellung durch eine 2-Oxo-tetrahydrofuranylmethyl- oder 2-Oxo-tetrahydrofuranylcarbonylgruppe substituiert ist,
eine Piperazinogruppe, die in 4-Stellung durch eine geradkettige C₂₋₃-Alkylgruppe substituiert ist, wobei der C₂₋₃-Alkylteil jeweils endständig durch eine 2-Oxo-tetrahydrofuran-3-ylsulfenylgruppe substituiert ist,
eine Piperidin-4-yl-Gruppe, die in 1-Stellung durch eine 2-Oxo-tetrahydrofuran-3-yl- oder 2-Oxo-tetrahydrofuran-4-yl-Gruppe substituiert ist, oder
eine Piperidin-4-yl-NCH₃-Gruppe, die am Ringstickstoffatom durch eine 2-Oxo-tetrahydrofuran-3-yl-, 2-Oxo-tetrahydrofuran-4-yl- oder 2-Oxo-tetrahydrofuranylcarbonyl-Gruppe substituiert ist, bedeuten,
deren Tautomeren, deren Stereoisomere und deren Salze.
Ganz besonders bevorzugte Verbindungen der obigen allgemeinen Formel I sind diejenigen, in denen
X ein Stickstoffatom,
Rₐ ein Wasserstoffatom,
R_{b} eine 1-Phenylethylgruppe oder eine durch die Reste R₁ bis R₃ substituierte Phenylgruppe, wobei
   R₁ und R₂, die gleich oder verschieden sein können, jeweils eine Methylgruppe oder ein Wasserstoff-, Fluor-, Chlor- oder Bromatom und
   R₃ ein Wasserstoffatom darstellen,
R_{c} ein Wasserstoffatom,
R_{d} ein Wasserstoffatom, eine Methoxy-, Ethoxy-, 2-Methoxy-ethoxy-, Cyclobutyloxy-, Cyclopentyloxy-, Cyclopropylmethoxy-, Cyclobutylmethoxy-, Tetrahydrofuran-3-yloxy-, Tetrahydropyran-4-yloxy- oder Tetrahydrofuran-2-ylmethoxygruppe,
A eine 1,2-Vinylengruppe,
B eine Methylengruppe oder, falls B an ein Kohlenstoffatom der Gruppe C gebunden ist, auch eine Bindung,
C eine Piperidinogruppe, in der die beiden Wasserstoffatome in 4-Stellung durch eine -CH₂-O-CO-CH₂-, -CH₂CH₂-O-CO-, -CH₂CH₂-O-CO-CH₂-, -O-CO-CH₂-NCH₃-CH₂- oder -O-CO-CH₂-O-CH₂-Brücke ersetzt sind,
eine Piperazinogruppe, in der ein Wasserstoffatom in 3-Stellung zusammen mit dem Wasserstoffatom in 4-Stellung durch eine -CO-O-CH₂-CH₂- oder -CH₂-O-CO-CH₂-Brücke ersetzt sind, wobei jeweils das linke Ende der vorstehenden Brücken an die 3-Stellung des Piperazinoringes gebunden ist,
eine durch eine 2-Oxo-tetrahydrofuranylgruppe substituierte Piperidinogruppe,
eine Piperidinogruppe, die in 4-Stellung durch eine 2-Oxo-morpholino- oder 2-Oxomorpholinomethylgruppe substituiert ist, wobei der 2-Oxo-morpholinoteil jeweils durch eine oder zwei Methylgruppen substituiert sein kann,
eine Piperazinogruppe, die in 4-Stellung durch eine 2-Oxo-tetrahydrofuran-3-yl- oder 2-Oxo-tetrahydrofuran-4-yl-Gruppe substituiert ist,
eine Piperidinogruppe, die in 4-Stellung durch eine CH₃NR₆- oder R₆S-Gruppe substituiert ist, wobei
   R₆ eine 2-Oxo-tetrahydrofuran-3-yl- oder 2-Oxo-tetrahydrofuran-4-yl-Gruppe darstellt,
eine Piperazinogruppe, die in 4-Stellung durch eine 2-Oxo-tetrahydrofuranylmethyl- oder 2-Oxo-tetrahydrofuranylcarbonylgruppe substituiert ist,
eine Piperazinogruppe, die in 4-Stellung durch eine [2-(2-Oxo-tetrahydrofuran-3-ylsulfenyl)ethyl]gruppe substituiert ist,
eine Piperidin-4-yl-Gruppe, die in 1-Stellung durch eine 2-Oxo-tetrahydrofuran-3-yl-oder 2-Oxo-tetrahydrofuran-4-yl-Gruppe substituiert ist, oder
eine Piperidin-4-yl-NCH₃-Gruppe, die am Ringstickstoffatom durch eine 2-Oxo-tetrahydrofuran-3-yl-, 2-Oxo-tetrahydrofuran-4-yl- oder 2-Oxo-tetrahydrofuranylcarbonyl-Gruppe substituiert ist, bedeuten,
deren Tautomeren, deren Stereoisomere und deren Salze.

Als ganz besonders bevorzugte Verbindungen der allgemeinen Formel I seien beispielsweise folgende erwähnt:
(a) 4-[(3-Chlor-4-fluor-phenyl)amino]-6-({4-[4-(2-oxo-tetrahydrofuran-3-yl)-piperazin-1-yl]-1-oxo-2-buten-1-yl}amino)-7-cyclopropylmethoxy-chinazolin,
(b) 4-[(3-Chlor-4-fluor-phenyl)amino]-6-({4-[4-(2-oxo-tetrahydrofuran-4-yl)-piperazin-1-yl]-1-oxo-2-buten-1-yl}amino)-7-cyclopropylmethoxy-chinazolin,
(c) 4-[(3-Chlor-4-fluor-phenyl)amino]-6-{[4-(4-{2-[(2-oxo-tetrahydrofuran-3-yl)sulfanyl]-ethyl}-piperazin-1-yl)-1-oxo-2-buten-1-yl]amino}-7-cyclopropylmethoxy-chinazolin,
(d) 4-[(3-Chlor-4-fluor-phenyl)amino]-6-{[4-(3-oxo-perhydro-pyrazino-[2,1-c][1,4]oxazin-8-yl)-1-oxo-2-buten-1-yl]amino}-7-cyclopropylmethoxy-chinazolin,
(e) (S)-4-[(3-Chlor-4-fluor-phenyl)amino]-6-[(4-{4-[(5-oxo-tetrahydrofuran-2-yl)carbonyl]-piperazin-1-yl}-1-oxo-2-buten-1-yl)amino]-7-cyclopropylmethoxy-chinazolin,
(f) 4-[(3-Chlor-4-fluor-phenyl)amino]-6-{[4-(1-oxo-perhydro-pyrazino[2,1-c][1,4]oxazin-8-yl)-1-oxo-2-buten-1-yl]amino}-7-cyclopropylmethoxy-chinazolin und
(g) 4-[(3-Chlor-4-fluor-phenyl)amino]-6-[(4-{4-[(2-oxo-tetrahydrofuran-3-yl)sulfanyl]-piperidin-1-yl}-1-oxo-2-buten-1-yl)amino]-7-cyclopropylmethoxy-chinazolin
sowie deren Salze.

Die Verbindungen der allgemeinen Formel I lassen sich beispielsweise nach folgenden Verfahren herstellen:
a. Umsetzung einer Verbindung der allgemeinen Formel 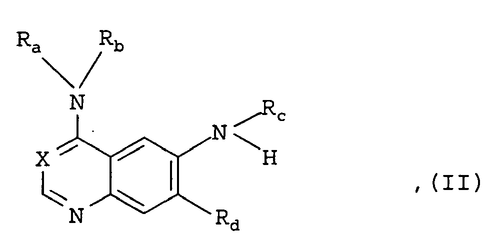 in der
   Rₐ bis R_{d} und X wie eingangs erwähnt definiert sind,
   mit einer Verbindung der allgemeinen Formel

   HO-CO-A-B-C, (III)

   in der
   A bis C wie eingangs erwähnt definiert sind, oder mit deren reaktionsfähigen Derivaten.

   Die Umsetzung wird gegebenenfalls in einem Lösungsmittel oder Lösungsmittelgemisch wie Methylenchlorid, Dimethylformamid, Benzol, Toluol, Chlorbenzol, Tetrahydrofuran oder Dioxan gegebenenfalls in Gegenwart in Gegenwart eines wasserentziehenden Mittels, z.B. in Gegenwart von Chlorameisensäureisobutylester, Thionylchlorid, Trimethylchlorsilan, Phosphortrichlorid, Phosphorpentoxid, N,N'-Dicyclohexylcarbodiimid, N,N'-Dicyclohexylcarbodiimid/N-Hydroxysuccinimid, N,N'-Carbonyldiimidazol, Triphenyl-phosphin/Tetrachlorkohlenstoff oder O-(Benzotriazol-1-yl)-N,N,N',N'-tetramethyluronium-tetrafluorborat oder mit einem entsprechenden reaktionsfähigen Derivat wie einem entsprechenden Ester, Säurehalogenid oder -anydrid gegebenenfalls unter Zusatz einer anorganischen oder organischen Base, vorzugsweise unter Zusatz einer organischen Base wie Triethylamin, N-Ethyl-diisopropylamin oder 4-Dimethylamino-pyridin, zweckmäßigerweise bei Temperaturen zwischen 0 und 150°C, vorzugsweise bei Temperaturen zwischen 0 und 80°C, durchgeführt.
b. Umsetzung einer gegebenenfalls im Reaktionsgemisch gebildeten Verbindung der allgemeinen Formel 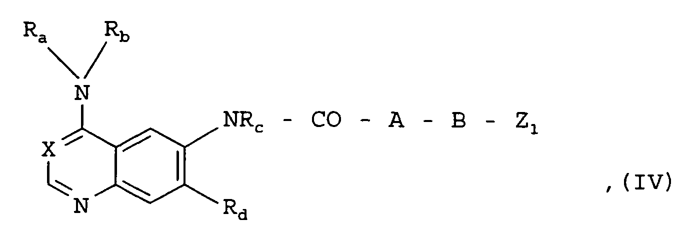 in der
   Rₐ bis R_{d}, A, B und X wie eingangs erwähnt definiert sind und
   Z₁ eine austauschbare Gruppe wie ein Halogenatom oder eine substituierte Sulfinyl- oder Sulfonylgruppe, z. B. ein Chlor- oder Bromatom, eine Methylsulfinyl-, Propylsulfinyl-, Phenylsulfinyl-, Benzylsulfinyl-, Methylsulfonyl-, Propylsulfonyl-, Phenylsulfonyl- oder Benzylsulfonylgruppe bedeutet,
   mit einer Verbindung der allgemeinen Formel

   H-G, (V)

   in der
   G einen der für C eingangs erwähnten Reste darstellt, der über ein Stickstoffatom mit dem Rest B verknüpft ist.

   G kann jedoch auch eine durch Lactonisierung in einen der für C eingangs erwähnten Reste, die über ein Stickstoffatom mit dem Rest B verknüpft sind, überführbare Gruppe (z.B. eine entsprechend substituierte gamma- oder delta-Hydroxycarbonsäureester-Gruppe) darstellen.
   Die Umsetzung wird zweckmäßigerweise in einem Lösungsmittel wie Isopropanol, Butanol, Tetrahydrofuran, Dioxan, Toluol, Chlorbenzol, Dimethylformamid, Dimethylsulfoxid, Methylenchlorid, Ethylenglycolmonomethylether, Ethylenglycoldiethylether oder Sulfolan gegebenenfalls in Gegenwart einer anorganischen oder tertiären organischen Base, z.B. Natriumcarbonat oder Kaliumhydroxid, einer tertiären organischen Base, z.B. Triethylamin, oder in Gegenwart von N-Ethyl-diisopropylamin (Hünig-Base), wobei diese organischen Basen gleichzeitig auch als Lösungsmittel dienen können, und gegebenenfalls in Gegenwart eines Reaktionsbeschleunigers wie einem Alkalihalogenid bei Temperaturen zwischen -20 und 150°C, vorzugsweise jedoch bei Temperaturen zwischen -10 und 100°C, durchgeführt. Die Umsetzung kann jedoch auch ohne Lösungsmittel oder in einem Überschuß der eingesetzten Verbindung der allgemeinen Formel V durchgeführt werden.
   Stellt G eine durch Lactonisierung in einen der für C eingangs erwähnten Reste, die über ein Stickstoffatom mit dem Rest B verknüpft sind, überführbare Gruppe dar, so schließt sich gegebenenfalls die Cyclisierung zum entsprechenden Lacton an. Die Cyclisierung zum entsprechenden Lacton wird gegebenenfalls in einem Lösungsmittel wie Acetonitril, Methylenchlorid, Tetrahydrofuran, Dioxan oder Toluol in Gegenwart einer Säure wie Salzsäure, p-Toluolsulfonsäure oder Trifluoressigsäure bei Temperaturen zwischen -10 und 120°C durchgeführt.
c. Zur Herstellung einer Verbindung der allgemeinen Formel I, in der C eine der für C eingangs erwähnten Gruppen darstellt, die eine durch R₆ oder durch eine R₅-C₁₋₄-alkyl-Gruppe substituierte lmino- oder HNR₄-Gruppe enthält, wobei R₄ bis R₆ wie eingangs erwähnt definiert sind:
   Umsetzung einer Verbindung der allgemeinen Formel 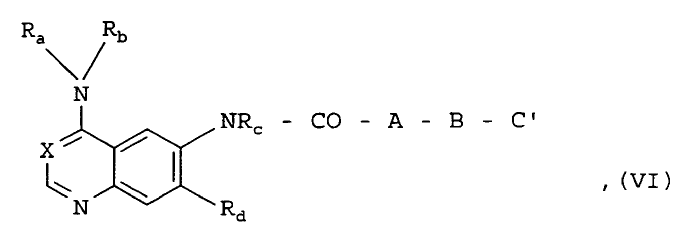 in der
   Rₐ bis R_{d}, A und B wie eingangs erwähnt definiert sind und
   C' eine der für C eingangs erwähnten Gruppen darstellt, die eine entsprechende unsubstituierte lmino- oder HNR₄-Gruppe enthält, wobei R₄ wie eingangs erwähnt definiert ist, darstellt,
   mit einer Verbindung der allgemeinen Formel

   Z₂-U, (VII)

   in der
   U den Rest R₆ oder eine R₅-C₁₋₄-alkyl-Gruppe bedeutet, wobei R₅ und R₆ wie eingangs erwähnt definiert sind, und
   Z₂ eine austauschbare Gruppe wie ein Halogenatom oder eine substituierte Sulfonyloxygruppe, z. B. ein Chlor- oder Bromatom, eine Methylsulfonyloxy-, Propylsulfonyloxy-, Phenylsulfonyloxy- oder Benzylsulfonyloxygruppe, oder
   Z₂ zusammen mit einem benachbarten Wasserstoffatom eine weitere Kohlenstoff-Kohlenstoffbindung, die mit einer Carbonylgruppe verbunden ist, bedeutet.

   Die Umsetzung wird zweckmäßigerweise in einem Lösungsmittel wie Methanol, Ethanol, Isopropanol oder Acetonitril und gegebenenfalls in Gegenwart einer Base wie Triethylamin, N-Ethyl-diisopropylamin oder 2-Dimethylamino-pyridin bei Temperaturen zwischen 0 und 100°C, vorzugsweise jedoch bei der Siedetemperatur des Reaktionsgemisches, durchgeführt.
   Bedeutet in einer Verbindung der allgemeinen Formel VII Z₂ eine austauschbare Gruppe, so wird die Umsetzung vorzugsweise in einem Lösungsmittel oder Lösungsmittelgemisch wie Methylenchlorid, Dimethylformamid, Dimethylsulfoxid, Sulfolan, Benzol, Toluol, Chlorbenzol, Tetrahydrofuran, Benzol/Tetrahydrofuran oder Dioxan zweckmäßigerweise in Gegenwart einer tertiären organischen Base wie Triethylamin oder N-Ethyl-diisopropylamin (Hünig-Base), wobei diese organischen Basen gleichzeitig auch als Lösungsmittel dienen können, oder in Gegenwart einer anorganischen Base wie Natriumkarbonat, Kaliumcarbonat oder Natronlauge zweckmäßigerweise bei Temperaturen zwischen -20 und 200°C, vorzugsweise bei Temperaturen zwischen 0 und 150°C, oder
   bedeutet in einer Verbindung der allgemeinen Formel VII Z₂ zusammen mit einem benachbarten Wasserstoffatom eine weitere Kohlenstoff-Kohlenstoffbindung, die mit einer Carbonylgruppe verbunden ist, so wird die Umsetzung vorzugsweise in einem Lösungsmittel wie Methanol, Ethanol, Isopropanol oder Acetonitril bei Temperaturen zwischen 0 und 100°C, vorzugsweise jedoch bei der Temperaturen zwischen 20°C und der Siedetemperatur des Reaktionsgemisches, durchgeführt.
d. Zur Herstellung einer Verbindung der allgemeinen Formel I, in der C eine der für C eingangs erwähnten Gruppen darstellt, die eine durch eine R₅CO-, R₅-C₁₋₄-alkylen-CO-, (R₄NR₆)-C₁₋₄-alkylen-CO-, R₆O-C₁₋₄-alkylen-CO-, R₆S-C₁-₄₋alkylen-CO-, R₆SO-C₁₋₄-alkylen-CO-, R₆SO₂-C₁₋₄-alkylen-CO- oder 2-Oxo-morpholino-C₁₋₄-alkylen-CO-Gruppe substituierte Imino- oder HNR₄-Gruppe enthält, wobei R₄ bis R₆ wie eingangs erwähnt definiert sind und der 2-Oxo-morpholinoteil durch eine oder zwei C₁₋₂-Alkylgruppen substituiert sein kann:
   Umsetzung einer Verbindung der allgemeinen Formel 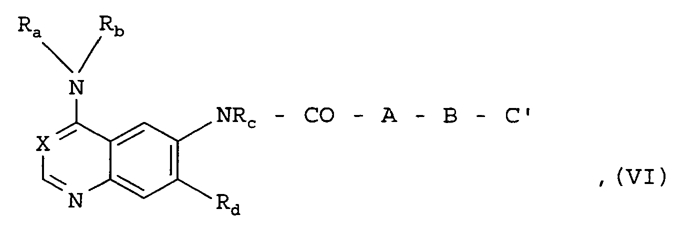 in der
   Rₐ bis R_{d}, A und B wie eingangs erwähnt definiert sind und
   C' eine der für C eingangs erwähnten Gruppen darstellt, die eine entsprechende unsubstituierte Imino- oder HNR₄-Gruppe enthält, wobei R₄ wie eingangs erwähnt definiert ist, darstellt,
   mit einer Verbindung der allgemeinen Formel

   HO-CO-W, (VIII)

   in der
   W den Rest R₅ oder eine R₅-C₁₋₄-alkyl-, (R₄NR₆)-C₁₋₄-alkyl-, R₆O-C₁₋₄-alkyl-, R₆S-C₁₋₄-alkyl-, R₆SO-C₁₋₄-alkyl-, R₆SO₂-C₁₋₄-alkyl-oder 2-Oxo-morpholino-C₁₋₄-alkyl-Gruppe darstellt, in denen R₄ bis R₆ wie eingangs erwähnt definiert sind und der 2-Oxo-morpholinoteil durch eine oder zwei C₁₋₂-Alkylgruppen substituiert sein kann.

Die Umsetzung wird gegebenenfalls in einem Lösungsmittel oder Lösungsmittelgemisch wie Methylenchlorid, Dimethylformamid, Benzol, Toluol, Chlorbenzol, Tetrahydrofuran oder Dioxan gegebenenfalls in Gegenwart in Gegenwart eines wasserentziehenden Mittels, z.B. in Gegenwart von Chlorameisensäureisobutylester, Thionylchlorid, Trimethylchlorsilan, Phosphortrichlorid, Phosphorpentoxid, N,N'-Dicyclohexylcarbodiimid, N,N'-Dicyclohexylcarbodiimid/N-Hydroxysuccinimid, N,N'-Carbonyldiimidazol, Triphenyl-phosphin/Tetrachlorkohlenstoff oder O-(Benzotriazol-1-yl)-N,N,N',N'-tetramethyluronium-tetrafluorborat oder mit einem entsprechenden reaktionsfähigen Derivat wie einem entsprechenden Ester, Säurehalogenid oder -anydrid gegebenenfalls unter Zusatz einer anorganischen oder organischen Base, vorzugsweise unter Zusatz einer organischen Base wie Triethylamin, N-Ethyl-diisopropylamin oder 4-Dimethylamino-pyridin, zweckmäßigerweise bei Temperaturen zwischen 0 und 150°C, vorzugsweise bei Temperaturen zwischen 0 und 80°C, durchgeführt.

Bei den vorstehend beschriebenen Umsetzungen können gegebenenfalls vorhandene reaktive Gruppen wie Hydroxy-, Carboxy-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, Methyl-, Ethyl-, tert.Butyl-, Trityl-, Benzyl- oder Tetrahydropyranylgruppe,
als Schutzreste für eine Carboxygruppe die Trimethylsilyl-, Methyl-, Ethyl-, tert.Butyl-, Benzyl- oder Tetrahydropyranylgruppe und
als Schutzreste für eine Amino-, Alkylamino- oder Iminogruppe die Formyl-, Acetyl-, Trifluoracetyl-, Ethoxycarbonyl-, tert.Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von Jodtrimethylsilan, bei Temperaturen zwischen 0 und 120°C, vorzugsweise bei Temperaturen zwischen 10 und 100°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.Butyl- oder tert.Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Diethylether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran bei Temperaturen zwischen 0 und 50°C.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971)) in ihre optischen Antipoden und Verbindungen der allgemeinen Formel I mit mindestens 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Die als Ausgangsstoffe verwendeten Verbindungen der allgemeinen Formeln II bis VIII sind teilweise literaturbekannt oder man erhält diese nach an sich literaturbekannten Verfahren (siehe Beispiele I bis XV).

Wie bereits eingangs erwähnt, weisen die erfindungsgemäßen Verbindungen der allgemeinen Formel I und ihre physiologisch verträglichen Salze wertvolle pharmakologische Eigenschaften auf, insbesondere eine Hemmwirkung auf die durch den Epidermal Growth Factor-Rezeptor (EGF-R) vermittelte Signaltransduktion, wobei diese beispielsweise durch eine Inhibition der Ligandenbindung, der Rezeptordimerisierung oder der Tyrosinkinase selbst bewirkt werden kann. Außerdem ist es möglich, daß die Signalübertragung an weiter abwärtsliegenden Komponenten blockiert wird.

Die biologischen Eigenschaften der neuen Verbindungen wurden wie folgt geprüft:

Die Hemmung der EGF-R vermittelten Signalübertragung kann z.B. mit Zellen nachgewiesen werden, die humanen EGF-R exprimieren und deren Überleben und Proliferation von Stimulierung durch EGF bzw. TGF-alpha abhängt. Hier wurde eine Interleukin-3-(IL-3) abhängige Zellinie murinen Ursprungs verwendet, die derart genetisch verändert wurde, daß sie funktionellen humanen EGF-R exprimiert. Die Proliferation dieser F/L-HERc genannten Zellen kann daher entweder durch murines IL-3 oder durch EGF stimuliert werden (siehe von Rüden, T. et al. in EMBO J. 7, 2749-2756 (1988) und Pierce, J. H. et al. in Science 239, 628-631 (1988)).

Als Ausgangsmaterial für die F/L-HERc Zellen diente die Zelllinie FDC-P₁, deren Herstellung von Dexter, T. M. et al. in J. Exp. Med. 152, 1036-1047 (1980) beschrieben wurde. Alternativ können aber auch andere Wachstumsfaktor-abhängige Zellen verwendet werden (siehe beispielsweise Pierce, J. H. et al. in Science 239, 628-631 (1988), Shibuya, H. et al. in Cell 70, 57-67 (1992) und Alexander, W. S. et al. in EMBO J. 10, 3683-3691 (1991)). Zur Expression der humanen EGF-R cDNA (siehe Ullrich, A. et al. in Nature 309, 418-425 (1984)) wurden rekombinante Retroviren verwendet, wie in von Rüden, T. et al., EMBO J. 7, 2749-2756 (1988) beschrieben, mit dem Unterschied, daß zur Expression der EGF-R cDNA der retrovirale Vektor LXSN (siehe Miller, A. D. et al. in BioTechniques 7, 980-990 (1989)) eingesetzt wurde und als Verpackungszelle die Linie GP+E86 (siehe Markowitz, D. et al. in J. Virol. 62, 1120-1124 (1988)) diente.

Der Test wurde wie folgt durchgeführt:

F/L-HERc Zellen wurden in RPMI/1640 Medium (BioWhittaker), supplementiert mit 10 % foetalem Rinderserum (FCS, Boehringer Mannheim), 2 mM Glutamin (BioWhittaker), Standardantibiotika und 20 ng/ml humanem EGF (Promega), bei 37°C und 5% CO₂ kultiviert. Zur Untersuchung der inhibitorischen Aktivität der erfindungsgemäßen Verbindungen wurden 1,5 x 10⁴ Zellen pro Vertiefung in Triplikaten in 96-Loch-Platten in obigem Medium (200 µl) kultiviert, wobei die Proliferation der Zellen entweder mit EGF (20 ng/ml) oder murinem IL-3 stimuliert wurde. Als Quelle für IL-3 dienten Kulturüberstände der Zellinie X63/0 mIL-3 (siehe Karasuyama, H. et al.in Eur. J. Immunol. 18, 97-104 (1988)). Die erfindungsgemäßen Verbindungen wurden in 100% Dimethylsulfoxid (DMSO) gelöst und in verschiedenen Verdünnungen den Kulturen zugefügt, wobei die maximale DMSO Konzentration 1 % betrug. Die Kulturen wurden für 48 Stunden bei 37°C inkubiert.

Zur Bestimmung der inhibitorischen Aktivität der erfindungsgemäßen Verbindungen wurde die relative Zellzahl mit dem Cell Titer 96^{™} AQᵤₑₒᵤₛ Non-Radioactive Cell Proliferation Assay (Promega) in O.D. Einheiten gemessen. Die relative Zellzahl wurde in Prozent der Kontrolle (F/LHERc Zellen ohne Inhibitor) berechnet und die Wirkstoffkonzentration, die die Proliferation der Zellen zu 50% hemmt (IC₅₀), abgeleitet. Hierbei wurden folgende Ergebnisse erhalten:

| Verbindung (Beispiel Nr.) | Hemmung der EGF-abhängigen Proliferation IC₅₀ [nM] |
|---|---|
| 1 | 0.05 |
| 2 | 0.60 |
| 4 | 3 |
| 4(3) | 10 |

Die erfindungsgemäßen Verbindungen der allgemeinen Formel I hemmen somit die Signaltransduktion durch Tyrosinkinasen, wie am Beispiel des humanen EGF-Rezeptors gezeigt wurde, und sind daher nützlich zur Behandlung pathophysiologischer Prozesse, die durch Überfunktion von Tyrosinkinasen hervorgerufen werden. Das sind z.B. benigne oder maligne Tumoren, insbesondere Tumoren epithelialen und neuroepithelialen Ursprungs, Metastasierung sowie die abnorme Proliferation vaskulärer Endothelzellen (Neoangiogenese).

Die erfindungsgemäßen Verbindungen sind auch nützlich zur Vorbeugung und Behandlung von Erkrankungen der Atemwege und der Lunge, die mit einer vermehrten oder veränderten Schleimproduktion einhergehen, die durch Stimulation von Tyrosinkinasen hervorgerufen wird, wie z.B. bei entzündlichen Erkrankungen der Atemwege wie chronische Bronchitis, chronisch obstruktive Bronchitis, Asthma, Bronchiektasien, allergische oder nicht-allergische Rhinitis oder Sinusitis, zystische Fibrose, α1-Antitrypsin-Mangel, oder bei Husten, Lungenemphysem, Lungenfibrose und hyperreaktiven Atemwegen.

Die Verbindungen sind auch geeignet für die Behandlung von Erkrankungen des Magen-Darm-Traktes und der Gallengänge und -blase, die mit einer gestörten Aktivität der Tyrosinkinasen einhergehen, wie sie z.B. bei chronisch entzündlichen Veränderungen zu finden sind, wie Cholezystitis, M. Crohn, Colitis ulcerosa, und Geschwüren im Magen-Darm-Trakt oder wie sie bei Erkrankungen des Magen-Darm-Traktes, die mit einer vermehrten Sekretion einhergehen, vorkommen, wie M. Ménétrier, sezernierende Adenome und Proteinverlustsyndrome,
desweiteren zur Behandlung von Nasenpolypen sowie von Polypen des Gastrointestinaltraktes unterschiedlicher Genese wie z.B. villöse oder adenomatöse Polypen des Dickdarms, aber auch von Polypen bei familiärer Polyposis coli, bei Darmpolypen im Rahmen des Gardner-Syndroms, bei Polypen im gesamten Magen-Darm-Trakt bei Peutz-Jeghers-Syndrom, bei entzündlichen Pseudopolypen, bei juvenilen Polypen, bei Colitis cystica profunda und bei Pneumatosis cystoides intestinales.

Außerdem können die Verbindungen der allgemeinen Formel I und deren physiologisch verträglichen Salze zur Behandlung von Nierenerkrankungen, insbesondere bei zystischen Veränderungen wie bei Zystennieren, zur Behandlung von Nierenzysten, die idiopathischer Genese sein können oder im Rahmen von Syndromen auftreten wie z.B. bei der tuberöser Sklerose, bei dem von-Hippel-Lindau-Syndrom, bei der Nephronophthisis und Markschwammniere sowie anderer Krankheiten verwendet werden, die durch aberrante Funktion von Tyrosinkinasen verursacht werden, wie z.B. epidermaler Hyperproliferation (Psoriasis), inflammatorischer Prozesse, Erkrankungen des Immunsystems, Hyperproliferation hämatopoetischer Zellen etc..

Auf Grund ihrer biologischen Eigenschaften können die erfindungsgemäßen Verbindungen allein oder in Kombination mit anderen pharmakologisch wirksamen Verbindungen angewendet werden, beispielsweise in der Tumortherapie in Monotherapie oder in Kombination mit anderen Anti-Tumor Therapeutika, beispielsweise in Kombination mit Topoisomerase-Inhibitoren (z.B. Etoposide), Mitoseinhibitoren (z.B. Vinblastin), mit Nukleinsäuren interagierenden Verbindungen (z.B. cis-Platin, Cyclophosphamid, Adriamycin), Hormon-Antagonisten (z.B. Tamoxifen), Inhibitoren metabolischer Prozesse (z.B. 5-FU etc.), Zytokinen (z.B. Interferonen), Antikörpern etc. Für die Behandlung von Atemwegserkrankungen können diese Verbindungen allein oder in Kombination mit anderen Atemwegstherapeutika, wie z.B. sekretolytisch, broncholytisch und/oder entzündungshemmend wirksamen Substanzen angewendet werden. Für die Behandlung von Erkrankungen im Bereich des Magen-Darm-Traktes können diese Verbindungen ebenfalls alleine oder in Kombination mit Motilitäts- oder Sekretions-beeinflussenden oder entzündungshemmenden Substanzen gegeben werden. Diese Kombinationen können entweder simultan oder sequentiell verabreicht werden.

Die Anwendung dieser Verbindungen entweder alleine oder in Kombination mit anderen Wirkstoffen kann intravenös, subkutan, intramuskulär, intrarektal, intraperitoneal, intranasal, durch Inhalation oder transdermal oder oral erfolgen, wobei zur Inhalation insbesondere Aerosolformulierungen geeignet sind.

Bei der pharmazeutischen Anwendung werden die erfindungsgemäßen Verbindungen in der Regel bei warmblütigen Wirbeltieren, insbesondere beim Menschen, in Dosierungen von 0,01-100 mg/kg Körpergewicht, vorzugsweise bei 0,1-15 mg/kg verwendet. Zur Verabreichung werden diese mit einem oder mehreren üblichen inerten Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Stearylalkohol, Carboxymethylcellulose oder fetthaltigen Substanzen wie Hartfett oder deren geeigneten Gemischen in übliche galenische Zubereitungen wie Tabletten, Dragees, Kapseln, Pulver, Suspensionen, Lösungen, Sprays oder Zäpfchen eingearbeitet.

Die nachfolgenden Beispiele sollen die vorliegende Erfindung näher erläutern ohne diese zu beschränken:

### Herstellung der Ausgangsverbindungen:

### Beispiel I

### 4-[(3-Chlor-4-fluor-phenyl)amino]-6-{[4-(piperazin-1-yl)-1-oxo-2-buten-1-yl]amino}-7-cyclopropylmethoxy-chinazolin

Zu 1.80 g 4-[(3-Chlor-4-fluor-phenyl)amino]-6-({4-[4-(tert.-butyloxycarbonyl)-piperazin-1-yl]-1-oxo-2-buten-1-yl}amino)-7-cyclopropylmethoxy-chinazolin in 10 ml Methylenchlorid werden unter Eisbad-Kühlung 5 ml Trifluoressigsäure getropft. Nach einer Stunde wird das Eisbad entfernt und das Reaktionsgemisch über Nacht bei Raumtemperatur gerührt. Anschließend wird das Gemisch zur Trockne eingeengt, der Kolbenrückstand zwischen 150 ml Methylenchlorid/Methanol (9:1) und 100 ml 1 N Natronlauge verteilt. Die wäßrige Phase wird noch zweimal mit dem Lösungsmittelgemisch extrahiert, die vereinigten organischen Phasen werden über Magnesiumsulfat getrocknet und eingeengt. Das bräunliche Rohprodukt wird ohne weitere Reinigung weiter umgesetzt.
Ausbeute: 1.32 g (88 % der Theorie),
R_{f}-Wert: 0.60 (Reversed Phase DC-Fertigplatte (E. Merck), Acetonitril/Wasser/Trifluoressigsäure = 50:50:1)
Massenspektrum (ESI⁺): m/z = 511, 513 [M+H]⁺

Analog Beispiel I werden folgende Verbindungen erhalten:
(1) N-{2-[(2-Oxo-tetrahydrofuran-3-yl)sulfanyl]-ethyl}-piperazin x 2 Trifluoressigsäure (Das Reaktionsgemisch wird ohne wäßrige Aufarbeitung eingeengt.)
   R_{f}-Wert: 0.68 (Reversed Phase DC-Fertigplatte (E. Merck), Acetonitril/Wasser/Trifluoressigsäure = 50:50:1)
(2) 4-[(3-Chlor-4-fluor-phenyl)amino]-6-{[4-(4-methylamino-piperidin-1-yl)-1-oxo-2-buten-1-yl]amino}-7-cyclopropylmethoxy-chinazolin
   R_{f}-Wert: 0.50 (Reversed Phase DC-Fertigplatte (E. Merck), Acetonitril/Wasser/Trifluoressigsäure = 50:50:1)
   Massenspektrum (ESI⁻): m/z = 537, 539 [M-H]⁻
(3) 4-[(3-Chlor-4-fluor-phenyl)amino]-6-{[3-(piperidin-4-yl)-1-oxo-2-propen-1-yl]amino}-7-cyclopropylmethoxy-chinazolin
   R_{f}-Wert: 0.50 (Reversed Phase DC-Fertigplatte (E. Merck), Acetonitril/Wasser/Trifluoressigsäure = 50:50:1)
   Massenspektrum (ESI⁻): m/z = 494, 496 [M-H]⁻
(4) Perhydro-pyrazino[2,1-c][1,4]oxazin-3-on x 2 Trifluoressigsäure
   (Das Reaktionsgemisch wird ohne wäßrige Aufarbeitung eingeengt.)
   Massenspektrum (ESI⁺): m/z = 157 [M+H]⁺
(5) 4-[(3-Chlor-4-fluor-phenyl)amino]-6-({4-[N-(piperidin-4-yl)-N-methyl-amino]-1-oxo-2-buten-1-yl}amino)-7-cyclopropylmethoxy-chinazolin
   Massenspektrum (ESI⁻): m/z = 537, 539 [M-H]⁻
(6) Perhydro-pyrazino[2,1-*c*][1,4]oxazin-1-on x 2 Trifluoressigsäure
   (Das Reaktionsgemisch wird ohne wäßrige Aufarbeitung eingeengt.)
   Massenspektrum (ESI⁺): m/z = 157 [M+H]⁺
(7) 4-[(2-Oxo-tetrahydrofuran-3-yl)sulfanyl]-piperidin x 1 Trifluoressigsäure
   (Das Reaktionsgemisch wird ohne wäßrige Aufarbeitung eingeengt.)
   R_{f}-Wert: 0.57 (Reversed Phase DC-Fertigplatte (E. Merck), Acetonitril/Wasser/Trifluoressigsäure = 50:50:1)
   Massenspektrum (ESI⁺): m/z = 202 [M+H]⁺
(8) 4-[(2-Oxo-6,6-dimethyl-morpholin-4-yl)methyl]-piperidin x Trifluoressigsäure
   (Das Ausgangsmaterial, 1-(tert.Butyloxycarbonyl)-4-[N-(ethoxycarbonylmethyl)-N-((2-hydroxy-2-methyl-propyl)-aminomethyl]-piperidin, wird durch Umsetzung von 1-(tert.Butyloxycarbonyl)-4-(methansulfonyloxymethyl)-piperidin mit
   N-(Ethoxycarbonylmethyl)-2-hydroxy-2-methyl-propylamin erhalten)
   Massenspektrum (ESI⁺): m/z = 227 [M+H]⁺

### Beispiel II

### 4-[(3-Chlor-4-fluor-phenyl)amino]-6-({4-[4-(tert.butyloxycarbonyl)-piperazin-1-yl]-1-oxo-2-buten-1-yl}amino)-7-cyclopropylmethoxy-chinazolin

4.7 ml Oxalylchlorid werden bei Raumtemperatur zu einer Lösung aus 4.51 g 4-Bromcrotonsäure in 100 ml Methylenchlorid getropft. Nach Zugabe von einem Tropfen N,N-Dimethylformamid wird das Reaktionsgemisch noch etwa 45 Minuten bei Raumtemperatur gerührt, bis die Gasentwicklung beendet ist. Anschließend wird das Lösungsmittel vom entstandenen Säurechlorid im Vakuum abdestilliert. Zwischenzeitlich werden 7.00 g 6-Amino-4-[(3-chlor-4-fluor-phenyl)amino]-7-cyclopropylmethoxy-chinazolin und 10.2 ml Diisopropylethylamin in 250 ml Tetrahydrofuran in einem Eisbad auf 0°C abgekühlt. Das rohe Säurechlorid wird in 20 ml Methylenchlorid aufgenommen und unter Eisbad-Kühlung innerhalb von 5 Minuten zugetropft. Das Reaktionsgemisch wird 45 Minuten bei 0°C und eine Stunde bei Raumtemperatur gerührt, dann wird eine Suspension aus 18.17 g Piperazin-1-carbonsäure-tert.butylester in 5 ml N,N-Dimethylformamid zugegeben. Nach weiteren 48 Stunden Rühren bei Raumtemperatur wird das Lösungsmittel im Vakuum abdestilliert und der Rückstand zwischen Wasser und Essigester verteilt. Die wäßrige Phase wird mit Essigester extrahiert, die vereinten organischen Extrakte werden mit gesättigter Natriumchlorid-Lösung gewaschen, über Magnesiumsulfat getrocknet und eingeengt. Das Rohprodukt wird chromatographisch über eine Kieselgelsäule mit Essigester/Methanol (15:1 bis 9:1) gereinigt.
Ausbeute: 5.2 g (44 % der Theorie),
R_{f}-Wert: 0.42 (Kieselgel, Methylenchlorid/Methanol = 9:1)
Massenspektrum (ESI⁻): m/z = 609, 611 [M-H]⁻
Analog Beispiel II werden folgende Verbindungen erhalten:
(1) 4-[(3-Chlor-4-fluor-phenyl)amino]-6-[(4-{4-[N-(tert.-butyloxycarbonyl)-N-methylamino]-piperidin-1-yl}-1-oxo-2-buten-1-yl)amino]-7-cyclopropylmethoxy-chinazolin
   R_{f}-Wert: 0.35 (Kieselgel, Essigester/Methanol = 9:1)
   Massenspektrum (ESI⁻): m/z = 637, 639 [M-H]⁻
(2) 4-[(3-Chlor-4-fluor-phenyl)amino]-6-[(4-{N-[1-(tert.butyloxycarbonyl)-piperidin-4-yl]-N-methyl-amino}-1-oxo-2-buten-1-yl)amino]-7-cyclopropylmethoxy-chinazolin
   R_{f}-Wert: 0.39 (Kieselgel, Essigester/Methanol = 9:1)
   Massenspektrum (ESI⁻): m/z = 637, 639 [M-H]⁻

### Beispiel III

### 6-Amino-4-[(3-chlor-4-fluor-phenyl)amino]-7-cyclopropylmethoxy-chinazolin

30.00 g 4-[(3-Chlor-4-fluor-phenyl)amino]-7-cyclopropylmethoxy-6-nitro-chinazolin werden in einem Gemisch aus 900 ml Ethanol, 120 ml Eisessig und 300 ml Wasser suspendiert. Die Suspension wird unter Rückfluß erhitzt, wobei eine klare Lösung entsteht. Dann werden vorsichtig 17.24 g Eisenpulver portionsweise zugegeben, wobei das Reaktionsgemisch jeweils aufschäumt. Etwa 15 Minuten nach beendeter Zugabe fällt ein Niederschlag aus. Das Reaktionsgemisch wird noch weitere 15 Minuten gerührt und anschließend im Vakuum zu Trockne eingeengt. Der Kolbenrückstand wird in 1000 ml Methylenchlorid/Methanol (9:1) aufgenommen und mit 30 ml 33%iger wäßriger Ammoniaklösung versetzt. Der Eisenschlamm wird abfiltiert und mit Methylenchlorid:Methanol (9:1) nachgewaschen. Das braune Filtrat wird über eine Kieselgelpackung filtriert und zur Trockne eingeengt. Der Kolbenrückstand wird mit 60 ml Diethylether verrührt, abgesaugt und an der Luft getrocknet.
Ausbeute: 22.60 g (82 % der Theorie),
Schmelzpunkt: 208°C
Massenspektrum (ESI⁺): m/z = 359, 361 [M+H]⁺

### Beispiel IV

### 4-[(3-Chlor-4-fluor-phenyl)amino]-7-cyclopropylmethoxy-6-nitro-chinazolin

Zu 47.07 ml Cyclopropylmethanol in 500 ml N,N-Dimethylformamid werden unter Eisbad-Kühlung portionsweise 66.66 g Kalium-tert.butylat gegeben, wobei die Temperatur 12°C nicht übersteigt. Das Reaktionsgemisch wird noch 30 Minuten im Kühlbad gerührt, dann werden portionsweise 50.00 g 4-[(3-Chlor-4-fluor-phenyl)-amino]-7-fluor-6-nitro-chinazolin zugegeben, wobei sich das Reaktionsgemisch tief rot färbt und die Temperatur auf maximal 15°C ansteigt. Anschließend wird das Kühlbad entfernt und das Reaktionsgemisch wird noch eine Stunde bei Raumtemperatur gerührt. Zur Aufarbeitung wird das Reaktionsgemisch auf 4000 ml Wasser gegossen und mit ca. 210 ml 2N Salzsäure neutralisiert. Der entstandene Niederschlag wird abgesaugt, mit Wasser nachgewaschen und bei 40°C getrocknet. Ausbeute: 60.47 g Rohprodukt,
R_{f}-Wert: 0.60 (Kieselgel, Cyclohexan/Essigester = 1:1)
Massenspektrum (ESI⁻): m/z = 387, 389 [M-H]⁻

### Beispiel V

### 1-(tert.Butyloxycarbonyl)-4-(2-[(2-oxo-tetrahydrofuran-3-yl)sulfanyl]-ethyl}-piperazin

Zu einer methanolischen Lösung aus 1.40 g Natrium-2-oxo-tetrahydrofuran-3-thiolat (hergestellt durch Behandeln von 3-[(Methylcarbonyl)sulfanyl]-tetrahydrofuran-2-on mit Natriummethylat in Methanol) werden 4.02 g 1-(tert.Butyloxycarbonyl)-4-[2-(methansulfonyloxy)-ethyl]-piperazin gelöst in Methylenchlorid gegeben. Anschließend werden 10 ml N,N-Dimethylformamid zugesetzt und das Reaktionsgemisch wird 2.5 Stunden bei 50°C gerührt. Zur Aufarbeitung werden 100 ml Essigester zugegeben. Die organische Phase wird abgetrennt, mit Wasser und Natriumchloridlösung gewaschen, über Magnesiumsulfat getrocknet und eingeengt. Das rote, ölige Rohprodukt wird ohne weitere Reinigung umgesetzt.
Ausbeute: 3.50 g (96 % der Theorie)
R_{f}-Wert: 0.48 (Kieselgel, Methylenchlorid/Methanol = 9:1)
Massenspektrum (ESI⁻): m/z = 329 [M-H]⁻

### Beispiel VI

### 3-(Piperidin-4-yl)-dihydro-furan-2-on

3.60 g 3-(1-Benzyl-piperidin-4-yliden)-dihydro-furan-2-on werden in 40 ml Methanol gelöst, mit 360 mg Palladium auf Aktivkohle (10%ig) versetzt und bei Raumtemperatur hydriert. Nachdem die berechnete Menge Wasserstoff aufgenommen ist, wird der Katalysator abfiltriert und das Filtrat im Vakuum eingeengt. Es bleibt ein farbloses Öl zurück, welches ohne weitere Reinigung umgesetzt wird.
R_{f}-Wert: 0.16 (Kieselgel, Essigester/Methanol = 9:1)
Massenspektrum (EI): m/z = 169 [M]⁺

Analog Beispiel VI werden folgende Verbindungen erhalten:
(1) 4-[N-(tert.-Butyloxycarbonylmethyl)-N-(2-hydroxyethyl)-amino]-piperidin
   Ausgangsmaterial: Verbindung des Beispiels XIV (1)
   R_{f}-Wert: 0,34 (Kieselgel, Methylenchlorid/Methanol/konz. wässriges Ammoniak = 4:1:0,1)
(2) (*R*)-4-[N-(tert.-Butyloxycarbonylmethyl)-N-(2-hydroxypropyl)-amino]-piperidin Ausgangsmaterial: Verbindung des Beispiels XIV (2)
   Massenspektrum (ESI⁺): m/z = 273 [M+H]⁺
(3) 4-Hydroxy-4-[N-methyl-N-(ethoxycarbonylmethyl)-aminomethyl]-piperidin
   Ausgangsmaterial: Verbindung des Beispiels XVI
   R_{f}-Wert: 0,38 (Kieselgel, Methylenchlorid/Methanol/konz. wässriges Ammoniak = 4:1:0,1)

### Beispiel VII

### 3-(1-Benzyl-piperidin-4-yliden)-dihydro-furan-2-on

Zu 4.40 g Natriumhydrid in 25 ml Toluol werden langsam 24.00 g (2-Oxo-tetrahydrofuran-3-yl)-phosphonsäurediethylester getropft. Dann werden 1-Benzyl-9-piperidin-4-on zugegeben und das Reaktionsgemisch wird 3 Stunden unter Rückfluß erhitzt. Nach Abkühlung auf Raumtemperatur wird die überstehende Lösung abdekantiert, mit Toluol verdünnt und mit Wasser und gesättigter Natriumchloridlösung gewaschen. Die organische Phase wird über Magnesiumsulfat getrocknet, eingeengt und über eine Kieselgelsäule mit Essigester/Methanol (95:5) als Laufmittel chromatographiert.
Ausbeute: 14.43 g (52 % der Theorie),
R_{f}-Wert: 0.64 (Kieselgel, Essigester/Methanol = 9:1)
Massenspektrum (ESI⁻): m/z = 256 [M-H]⁻

### Beispiel VIII

### 4-[(3-Chlor-4-fluor-phenyl)amino]-6-({3-[1-(tert.butyloxycarbonyl)-piperidin-4-yl]-1-oxo-2-propen-1-yl}amino)-7-cyclopropylmethoxy-chinazolin

Zu 127 mg wasserfreiem Lithiumchlorid in 20 ml absolutem Tetrahydrofuran unter Argon werden 1.61 g 4-[(3-Chlor-4-fluor-phenyl)amino]-6-({[(diethoxyphosphoryl)methyl]carbonyl}amino)-7-cyclopropylmethoxy-chinazolin gegeben. Das Gemisch wird 15 Minuten bei Raumtemperatur gerührt, anschließend wird es im Eisbad auf 0°C abgekühlt und mit 0.45 ml 1,8-Diazabicyclo [5.4.0]undec-7-en versetzt. Nach weiteren 30 Minuten bei 0°C werden 690 mg 4-Formyl-1-(tert.butoxycarbonyl)-piperidin zugegeben. Man läßt das Reaktionsgemisch über Nacht auf Raumtemperatur erwärmen. Zur Aufarbeitung wird das Lösungsmittel im Vakuum entfernt und der Kolbenrückstand mit Essigester/Methanol (9:1) aufgenommen. Die Lösung wird mit Wasser und gesättigter Natriumchloridlösung gewaschen, über Magnesiumsulfat getrocknet und eingeengt. Das ölige, braune Rohprodukt wird chromatographisch über eine Kieselgelsäule mit Essigester als Laufmittel gereinigt.
Ausbeute: 1.30 g (73 % der Theorie),
R_{f}-Wert: 0.80 (Kieselgel, Essigester)
Massenspektrum (EI): m/z = 595, 597 [M]⁺

### Beispiel IX

### 4-[(3-Chlor-4-fluor-phenyl)amino]-6-({[(diethoxyphosphoryl)methyl]carbonyl}amino)-7-cyclopropylmethoxy-chinazolin

Zu 5.00 g 6-Amino-4-[(3-chlor-4-fluor-phenyl)amino]-7-cyclopropylmethoxy-chinazolin in 25 ml N,N-Dimethylformamid werden nacheinander 2.77 ml Triethylamin, 3.43 g Diethoxyphosphoryl-essigsäure und 5.62 g Benzotriazol-1-yl-N-tetramethyl-uronium-tetrafluoroborat gegeben. Das Reaktionsgemisch wird eine Stunde bei Raumtemperatur gerührt. Anschließend wird es mit 250 ml Wasser versetzt und mit 250 ml Essigester/Methanol (10:1) extrahiert. Die organische Phase wird mit Wasser und gesättigter Natriumchloridlösung gewaschen, über Magnesiumsulfat getrocknet und eingeengt. Der Kolbenrückstand wird durch Verrühren mit Diethylether zur Kristallisation gebracht.
Ausbeute: 7.00 g (94 % der Theorie),
Schmelzpunkt: 186°C
Massenspektrum (ESI⁻): m/z = 535, 537 [M-H]⁻

### Beispiel X

### 8-(tert.Butyloxycarbonyl)-perhydro-pyrazino[2,1-c][1,4]oxazin-3-on

2.00 g 1-(tert.Butyloxycarbonyl)-4-[(ethoxycarbonyl)methyl]-3-hydroxymethyl-piperazin in 2.5 ml Acetonitril werden mit 500 mg p-Toluolsulfonsäuremonohydrat versetzt. Das Reaktionsgemisch wird 3 Stunden unter Rückfluß erhitzt, bis die Umsetzung vollständig ist. Anschließend wird das Lösungsmittel im Vakuum abdestilliert. Das Rohprodukt wird ohne weitere Reinigung direkt weiter umgesetzt.
R_{f}-Wert: 0.80 (Kieselgel, Essigester/Methanol = 9:1)

Analog Beispiel X wird folgende Verbindung erhalten:
(1) 8-(tert.Butyloxycarbonyl)-perhydro-pyrazino[2,1-c][1,4]oxazin-1-on
   R_{f}-Wert: 0.60 (Kieselgel, Methylenchlorid/Methanol = 95:5)
   Massenspektrum (ESI⁺): m/z = 257 [M+H]⁺

### Beispiel XI

### 1-(tert.Butyloxycarbonyl)-4-[(ethoxycarbonyl)methyl]-3-hydroxymethyl-piperazin und 8-(tert-Butyloxycarbonyl)-perhydro-pyrazino[2,1-c][1,4]oxazin-3-on

Zu 5.80 g 1-(tert.Butyloxycarbonyl)-3-hydroxymethyl-piperazin und 4.50 ml Triethylamin in 60 ml Acetonitril werden 3.90 ml Bromessigsäureethylester gegeben. Das Reaktionsgemisch wird über Nacht unter Rückfluss erhitzt, wobei laut Dünnschichtchromatographie zwei Produkte entstehen. Zur Aufarbeitung wird das Reaktionsgemisch im Vakuum eingeengt und der Rückstand zwischen Essigester und Wasser verteilt. Die organische Phase wird über Magnesiumsulfat getrocknet, eingeengt und über eine Kieselgelsäule mit Essigester/Methanol (97:3) chromatographiert. Man erhält die beiden folgenden Produkte als gelbliche Öle:
8-(tert.Butyloxycarbonyl)-perhydro-pyrazino[2,1-c][1,4]oxazin-3-on
   Ausbeute: 3.43 g (50 % der Theorie),
   R_{f}-Wert: 0.80 (Kieselgel, Essigester/Methanol = 9:1)
1-(tert.Butyloxycarbonyl)-4-[(ethoxycarbonyl)methyl]-3-hydroxymethyl-piperazin
   Aubeute: 2.08 g (26 % der Theorie),
   R_{f}-Wert: 0.58 (Kieselgel, Essigester/Methanol = 9:1)
   Massenspektrum (ESI⁺): m/z = 303 [M+H]⁺

Analog Beispiel XI werden folgende Verbindungen erhalten:
(1) 8-(tert.Butyloxycarbonyl)-perhydro-pyrazino[2,1-c][1,4]oxazin-1-on
   R_{f}-Wert: 0.60 (Kieselgel, Methylenchlorid/Methanol = 95:5)
   Massenspektrum (ESI⁺): m/z = 257 [M+H]⁺
(2) 1-(tert.Butyloxycarbonyl)-3-(ethoxycarbonyl)-4-(2-hydroxy-ethyl)piperazin
   R_{f}-Wert: 0.50 (Kieselgel, Methylenchlorid/Methanol = 95:5)
   Massenspektrum (EI): m/z = 302 [M]⁺

### Beispiel XII

### 1-(tert.Butyloxycarbonyl)-3-hydroxymethyl-piperazin

Eine Suspension aus 900 mg Lithiumborhydrid in 20 ml Tetrahydrofuran wird tropfenweise mit einer Lösung aus 8.00 g 1-(tert.Butyloxycarbonyl)-3-ethoxycarbonyl-piperazin in 10 ml Tetrahydrofuran versetzt und anschließend 3 Stunden unter Rückfluß erhitzt. Zur Aufarbeitung wird das Reaktionsgemisch eingeengt, mit 10%iger wäßriger Zitronensäurelösung auf pH 4 eingestellt und etwa 40 Minuten unter Eisbad-Kühlung gerührt. Anschließend wird das Gemisch mit konzentrierter Natronlauge alkalisch gestellt und über Nacht stehengelassen. Am nächsten Morgen wird es mit *tert*-Butylmethylether extrahiert. Die organische Phase wird über Magnesiumsulfat getrocknet und eingeengt. Es bleibt ein klares Öl zurück, welches langsam kristallisiert.
Ausbeute: 5.80 g (87 % der Theorie),
R_{f}-Wert: 0.28 (Kieselgel, Essigester/Methanol = 4:1)
Massenspektrum (ESI⁺): m/z = 217 [M+H]⁺

### Beispiel XIII

### 1-(tert.Butyloxycarbonyl)-3-ethoxycarbonyl-piperazin

Zu 15.80 g 2-Ethoxycarbonyl-piperazin in 400 ml Ethanol werden unter Eisbad-Kühlung 21.80 g Pyrokohlensäure-di-tert.butylester gegeben. Das Reaktionsgemisch wird noch 3 Stunden bei 0°C gerührt. Anschließend wird es eingeengt und der Rückstand zwischen Essigester und Wasser verteilt. Die organische Phase wird über Magnesiumsulfat getrocknet, eingeengt und chromatographisch über eine Kieselgelsäule mit Essigester/Methanol (95:5) als Laufmittel gereinigt.
Ausbeute: 24.30 g (94 % der Theorie),
R_{f}-Wert: 0.40 (Kieselgel, Essigester/Methanol = 9:1)
Massenspektrum (ESI⁺): m/z = 281 [M+Na]⁺

### Beispiel XIV

### 1-tert.Butyloxycarbonyl-4-methylamino-piperidin

Zu 15.00 g 1-(tert.Butyloxycarbonyl)-4-piperidinon und 31.20 g Natriumacetat in 300 ml Tetrahydrofuran werden 25.50 g Methylaminhydrochlorid gegeben. Anschließend werden portionsweise 19.00 g Natriumtriacetoxyborhydrid zugesetzt. Das Reaktionsgemisch wird über Nacht bei Raumtemperatur gerührt und am nächsten Morgen eingeengt. Der Rückstand wird zwischen 5%iger Natriumhydrogencarbonatlösung und Methylenchlorid verteilt. Die wäßrige Phase wird mit 4 N Salzsäure etwa auf pH 11 eingestellt und mit Essigester extrahiert. Die organische Phase wird über Magnesiumsulfat getrocknet und eingeengt, wobei ein farbloses Öl zurückbleibt.
Ausbeute: 12.74 g (79 % der Theorie),
R_{f}-Wert: 0.22 (Kieselgel, Essigester/Methanol/konzentrierte wäßrige
Ammoniaklösung = 90:10:1)
Massenspektrum (ESI⁺): m/z = 215 [M+H]⁺

Analog Beispiel XIV werden folgende Verbindungen erhalten:
(1) 1-Benzyloxycarbonyl-4-[N-(tert.-butyloxycarbonylmethyl)-N-(2-hydroxyethyl)-amino]-piperidin
   Massenspektrum (ESI⁺): m/z = 393 [M+H]⁺
   R_{f}-Wert: 0.50 (Kieselgel, Cyclohexan/Essigester = 9:1)
(2) (*R*)-1-Benzyloxycarbonyl-4-[N-(tert.-butyloxycarbonylmethyl)-N-(2-hydroxypropyl)-amino]-piperidin
   Massenspektrum (ESI⁺): m/z = 407 [M+H]⁺
   R_{f}-Wert: 0.56 (Kieselgel, Cyclohexan/Essigester = 9:1)

### Beispiel XV

### 1-(tert.Butyloxycarbonyl)-4-[(2-oxo-tetrahydrofuran-3-yl)sulfanyl)-piperidin

Zu 5.28 g 1-(tert.Butyloxycarbonyl)-4-mercapto-piperidin in 20 ml N,N-Dimethylformamid werden unter Eisbad-Kühlung langsam 2.73 g Kalium-tert.butylat gegeben. Das Gemisch wird noch 30 Minuten unter Eisbad-Kühlung gerührt, dann wird eine Lösung aus 2.02 ml 3-Brom-dihydro-furan-2-on in 20 ml N,N-Dimethylformamid zugetropft. Nach beendeter Zugabe wird das Reaktionsgemisch zwei Stunden bei Raumtemperatur gerührt. Anschließend wird es mit Eisessig neutralisiert und eingeengt. Der Rückstand wird zwischen Essigester und Wasser verteilt. Die organische Phase wird über Magnesiumsulfat getrocknet und eingeengt. Es bleiben 7.60 g eines orangebraunen Öls zurück, welches ohne weitere Reinigung umgesetzt wird.
R_{f}-Wert: 0.32 (Kieselgel, Essigester/Cyclohexan = 2:3)
Massenspektrum (ESI⁻): m/z = 300 [M-H]⁻

### Beispiel XVI

### 1-Benzyloxycarbonyl-4-hydroxy-4-[N-methyl-N-(ethoxycarbonylmethyl)-aminomethyl]-piperidin

Eine Mischung aus 2,7 g Sarcosinethylester und 3,09 g 6-Benzyloxycarbonyl-1-oxa-6-aza-spiro[2.5]octan in 20 ml Ethanol werden in einem Bombenrohr 6 Stunden auf 90°C erhitzt. Nach dem Abkühlen über Nacht wird eingeengt und der Rückstand durch Chromatographie über eine Kieselgelsäule mit Cyclohexan/Essigester (1:1) gereinigt.
Ausbeute: 1,8 g (25% d. Theorie)
Massenspektrum (ESI⁺): m/z = 365 [M+H]⁺
R_{f}-Wert: 0,35 (Kieselgel, Cyclohexan/Essigester= 1:1)

### Herstellung der Endverbindungen:

### Beispiel 1

### 4-[(3-Chlor-4-fluor-phenyl)amino]-6-({4-[4-(2-oxo-tetrahydrofuran-3-yl)-piperazin-1-yl]-1-oxo-2-buten-1-yl}amino)-7-cyclopropylmethoxy-chinazolin

Zu 608 mg 4-[(3-Chlor-4-fluor-phenyl)amino]-6-{[4-(piperazin-1-yl)-1-oxo-2-buten-1-yl]amino}-7-cyclopropylmethoxy-chinazolin in 3.0 ml Tetrahydrofuran werden 0.42 ml Triethylamin gegeben. Das Gemisch wird im Eisbad abgekühlt und tropfenweise mit einer Lösung aus 215 mg 3-Brom-dihydro-furan-2-on in 1.0 ml Tetrahydrofuran versetzt und eine Stunde unter Kühlung gerührt. Anschließend wird das Eisbad entfernt und ca. 48 Stunden bei Raumtemperatur gerührt. Das Reaktionsgemisch wird ohne weitere Aufarbeitung direkt über eine Kieselgelsäule mit Methylenchlorid/Methanol (95:5 bis 90:10) als Laufmittel chromatographiert. Das schaumige Rohprodukt wird durch Verreiben mit wenig Diethylether zu Kristallisation gebracht.
Ausbeute: 393 mg (56 % der Theorie),
Schmelzpunkt: 130-131 °C
Massenspektrum (ESI⁻): m/z = 593, 595 [M-H]⁻

Analog Beispiel 1 werden folgende Verbindungen erhalten:
(1) (R)-4-[(3-Chlor-4-fluor-phenyl)amino]-6-[(4-{4-[(5-oxo-tetrahydrofuran-2-yl)methyl]-piperazin-1-yl}-1-oxo-2-buten-1-yl)amino]-7-cyclopropylmethoxy-chinazolin
   (Die Reaktion wird ohne Lösungsmittel mit (R)-5-Mesyloxymethyl-2-oxo-tetrahydrofuran durchgeführt)
   Schmelzpunkt: 145-150°C
   Massenspektrum (ESI⁻): m/z = 607, 609 [M-H]⁻
(2) 4-[(3-Chlor-4-fluor-phenyl)amino]-6-[(4-{4-[N-(2-oxo-tetrahydrofuran-3-yl)-N-methylamino]-piperidin-1-yl}-1-oxo-2-buten-1-yl)amino]-7-cyclopropylmethoxy-chinazolin
   R_{f}-Wert: 0.22 (Kieselgel, Essigester/Methanol = 7:3)
   Massenspektrum (ESI⁻): m/z = 621, 623 [M-H]⁻
(3) 4-[(3-Chlor-4-fluor-phenyl)amino]-6-[(4-{N-[1-(2-oxo-tetrahydrofuran-3-yl)-piperidin-4-yl]-N-methyl-amino}-1-oxo-2-buten-1-yl)amino]-7-cyclopropylmethoxy-chinazolin
   R_{f}-Wert: 0.73 (Aluminiumoxid, Essigester/Methanol = 9:1)
   Massenspektrum (ESI⁻): m/z = 621, 623 [M-H]⁻

### Beispiel 2

### 4-[(3-Chlor-4-fluor-phenyl)amino]-6-({4-[4-(2-oxo-tetrahydrofuran-4-yl)-piperazin-1-yl]-1-oxo-2-buten-1-yl}amino)-7-cyclopropylmethoxy-chinazolin

Zu 500 mg 4-[(3-Chlor-4-fluor-phenyl)amino]-6-{[4-(piperazin-1-yl)-1-oxo-2-buten-1-yl]amino}-7-cyclopropylmethoxy-chinazolin in 2 ml Methanol werden 78 µl 2(5H)-Furanon gegeben. Das Reaktionsgemisch wird etwa 48 Stunden bei Raumtemperatur gerührt und anschließend im Vakuum zur Trockene eingeengt. Der Kolbenrückstand wird chromatographisch über eine Kieselgelsäule mit Methylenchlorid/Methanol (95:5 bis 90:10) als Laufmittel gereinigt. Das erhaltene Produkt wird aus Essigester umkristallisiert.
Ausbeute: 170 mg (29 % der Theorie),
R_{f}-Wert: 0.43 (Kieselgel, Methylenchlorid/Methanol = 9:1)
Massenspektrum (ESI⁻): m/z = 593, 595 [M-H]⁻

Analog Beispiel 2 wird folgende Verbindung erhalten:
(1) 4-[(3-Chlor-4-fluor-phenyl)amino]-6-({3-[1-(2-oxo-tetrahydrofuran-4-yl)-piperidin-4-yl]-1-oxo-2-propen-1-yl}amino)-7-cyclopropylmethoxy-chinazolin
   Schmelzpunkt: 138°C
   Massenspektrum (ESI⁻): m/z = 578, 580 [M-H]⁻

### Beispiel 3

### (S)-4-[(3-Chlor-4-fluor-phenyl)amino]-6-[(4-{4-[(5-oxo-tetrahydrofuran-2-yl)carbonyl]-piperazin-1-yl}-1-oxo-2-buten-1-yl)amino]-7-cyclopropylmethoxy-chinazolin

Zu 500 mg 4-[(3-Chlor-4-fluor-phenyl)amino]-6-{[4-(piperazin-1-yl)-1-oxo-2-buten-1-yl]amino}-7-cyclopropylmethoxy-chinazolin in 3 ml N,N-Dimethylformamid werden 130 mg (S)-5-Oxo-tetrahydrofuran-2-carbonsäure, 0.21 ml Triethylamin und 321 mg Benzotriazol-1-yl-N-tetramethyl-uronium-tetrafluoroborat gegeben. Das Reaktionsgemisch wird etwa 48 Stunden bei Raumtemperatur stehengelassen. Anschließend werden 20 ml Wasser zugegeben, wobei sich ein schmieriger Niederschlag bildet. Dieser wird abgesaugt, mit Wasser nachgewaschen und chromatographisch über eine Kieselgelsäule mit Methylenchlorid/Methanol (95:5 bis 90:10) als Laufmittel gereinigt.
Ausbeute: 330 mg (54 % der Theorie),
Schmelzpunkt: 155-157°C
Massenspektrum (ESI⁻): m/z = 621, 623 [M-H]⁻

Analog Beispiel 3 wird folgende Verbindung erhalten:
(1) 4-[(3-Chlor-4-fluor-phenyl)amino]-6-{[4-(N-{1-[((5)-5-oxo-tetrahydrofuran-2-yl)carbonyl]-piperidin-4-yl}-N-methyl-amino)-1-oxo-2-buten-1-yl]amino}-7-cyclopropylmethoxy-chinazolin
   R_{f}-Wert: 0.36 (Kieselgel, Essigester/Methanol/konzentrierte, wäßrige
   Ammoniaklösung = 9:1:0.2)
   Massenspektrum (EI): m/z = 650, 652 [M]⁺

### Beispiel 4

### 4-[(3-Chlor-4-fluor-phenyl)amino]-6-{[4-(4-{2-[(2-oxo-tetrahydrofuran-3-yl)sulfanyl]-ethyl}-piperazin-1-yl)-1-oxo-2-buten-1-yl]amino}-7-cyclopropylmethoxy-chinazolin

0.67 ml Oxalylchlorid werden bei Raumtemperatur zu einer Lösung aus 644 mg 4-Bromcrotonsäure in 15 ml Methylenchlorid getropft. Nach Zugabe von einem Tropfen N,N-Dimethylformamid wird das Reaktionsgemisch noch etwa eine Stunde bei Raumtemperatur gerührt, bis die Gasentwicklung beendet ist. Anschließend wird das Lösungsmittel vom entstandenen Säurechlorid im Vakuum abdestilliert. Zwischenzeitlich werden 1.00 g 6-Amino-4-[(3-chlor-4-fluor-phenyl)amino]-7-cyclopropylmethoxy-chinazolin und 8.0 ml Diisopropylethylamin in 30 ml Tetrahydrofuran in einem Eisbad auf 0°C abgekühlt. Das rohe Säurechlorid wird in 10 ml Methylenchlorid aufgenommen und unter Eisbad-Kühlung innerhalb von 5 Minuten zugetropft. Das Reaktionsgemisch wird noch eine Stunde bei 0°C und zwei Stunden bei Raumtemperatur gerührt. Anschließend wird eine Suspension aus 4.35 N-{2-[(2-Oxo-tetrahydrofuran-3-yl)sulfanyl]-ethyl}-piperazin in 5 ml Methylenchlorid zugegeben und das Ganze weitere 48 Stunden bei Raumtemperatur gerührt. Das Reaktionsgemisch wird im Vakuum eingeengt und der Kolbenrückstand über eine Kieselgelsäule mit Essigester/Methanol (95:5 bis 90:10) als Laufmittel chromatographiert.
Ausbeute: 20 mg (1 % der Theorie),
R_{f}-Wert: 0.32 (Kieselgel, Methylenchlorid/Methanol = 9:1)
Massenspektrum (ESI⁻): m/z = 653, 655 [M-H]⁻

Analog Beispiel 4 werden folgende Verbindungen erhalten:
(1) 4-[(3-Chlor-4-fluor-phenyl)amino]-6-({4-[4-(2-oxo-tetrahydrofuran-3-yl)-piperidin-1-yl]-1-oxo-2-buten-1-yl}amino)-7-cydopropylmethoxy-chinazolin
   R_{f}-Wert: 0.17 (Kieselgel, Essigester/Methanot = 9:1)
   Massenspektrum (ESI⁻): m/z = 592, 594 [M-H]⁻
(2) 4-[(3-Chlor-4-fluor-phenyl)amino]-6-{[4-(3-oxo-2-oxa-8-aza-spiro[4.5]dec-8-yl)-1-oxo-2-buten-1-yl]amino}-7-cyclopropylmethoxy-chinazolin
   (Das eingesetzte 2-Oxa-8-aza-spiro[4.5]decan-3-on wird aus 8-Benzyl-2-oxa-8-aza-spiro[4.5]decan-3-on durch hydrogenolytische Abspaltung der Benzylgruppe erhalten)
   R_{f}-Wert: 0.37 (Kieselgel, Methylenchlorid/Methanol = 9:1)
   Massenspektrum (El): m/z = 579, 581 [M]⁺
(3) 4-[(3-Chlor-4-fluor-phenyl)amino]-6-{[4-(3-oxo-perhydro-pyrazino-[2,1-*c*][1,4]oxazin-8-yl)-1-oxo-2-buten-1-yl]amino}-7-cyclopropylmethoxy-chinazolin
   R_{f}-Wert: 0.19 (Kieselgel, Essigester/Methanol =9:1)
   Massenspektrum (ESI⁻): m/z = 579, 581 [M-H]⁻
(4) 4-[(3-Chlor-4-fluor-phenyl)amino]-6-{[4-(1-oxo-2-oxa-8-aza-spiro[4.5]dec-8-yl)-1-oxo-2-buten-1-yl]amino}-7-cyclopropylmethoxy-chinazolin
   R_{f}-Wert: 0.48 (Kieselgel, Methylenchlorid/Methanol = 9:1)
   Massenspektrum (EI): m/z = 579, 581 [M]⁺
(5) 4-[(3-Chlor-4-fluor-phenyl)amino]-6-{[4-(1-oxo-perhydro-pyrazino-[2,1-c][1,4]oxazin-8-yl)-1-oxo-2-buten-1-yl]amino}-7-cyclopropylmethoxy-chinazolin
   R_{f}-Wert: 0.21 (Kieselgel, Essigester/Methanol = 9:1)
   Massenspektrum (ESI⁻): m/z = 579, 581 [M-H]⁻
(6) 4-[(3-Chlor-4-fluor-phenyl)amino]-6-[(4-{4-[(2-oxo-tetrahydrofuran-3-yl)sulfanyl]-piperidin-1-yl}-1-oxo-2-buten-1-yl)amino]-7-cyclopropylmethoxy-chinazolin
   R_{f}-Wert: 0.23 (Kieselgel, Essigester/Methanol = 9:1)
   Massenspektrum (ESI⁻): m/z = 624, 626 [M-H]⁻
(7) 4-[(3-Chlor-4-fluor-phenyl)amino]-6-[(4-{4-[(2-oxo-6,6-dimethyl-morpholin-4-yl)methyl]-piperidin-1-yl}-1-oxo-2-buten-1-yl)amino]-7-cyclopropylmethoxy-chinazolin
   R_{f}-Wert: 0.48 (Kieselgel, Essigester/Methanol/konz. wäßriger Ammoniak = 9:1:0.2)
   Massenspektrum (ESI⁻): m/z = 649, 651 [M-H]⁻
(8) 4-[(3-Chlor-4-fluor-phenyl)amino]-6-({4-[4-(2-oxo-morpholin-4-yl)-piperidin-1-yl]-1-oxo-2-buten-1-yl}amino)-7-cyclopropylmethoxy-chinazolin hergestellt durch Lactonisierung des Zwischenproduktes (4-[(3-Chlor-4-fluor-phenyl)-amino]-6-({4-[4-[N-(tert.-butyloxycarbonylmethyl)-N-(2-hydroxyethyl)-amino]-piperidin-1-yl]-1-oxo-2-buten-1-yl}amino)-7-cyclopropylmethoxy-chinazolin, Schmelzpunkt 114-117°C (unter Aufschäumen), R_{f}-Wert: 0,46 (Kieselgel, Methylenchlorid/Methanol = 9:1)) in Gegenwart von 5 Equivalenten Trifluoressigsäure in Acetonitril unter Rückfluß
   Schmelzpunkt: ab 140°C (unter Aufschäumen)
   Massenspektrum (ESI⁺): m/z = 609, 611 [M+H]⁺
   R_{f}-Wert: 0,26 (Kieselgel, Essigester/Methanol = 4:1)
(9) (*R*)-4-[(3-Chlor-4-fluor-phenyl)amino]-6-({4-[4-(6-methyl-2-oxo-morpholin-4-yl)-piperidin-1-yl]-1-oxo-2-buten-1-yl}amino)-7-cyclopropylmethoxy-chinazolin hergestellt durch Lactonisierung des Zwischenproduktes ((*R*)-4-[(3-Chlor-4-fluorphenyl)amino]-6-({4-[4-[N-(tert.-butyloxycarbonylmethyl)-N-(2-hydroxypropyl)-amino]-piperidin-1-yl]-1-oxo-2-buten-1-yl}amino)-7-cyclopropylmethoxy-chinazolin, Schmelzpunkt: ab 115°C (unter Aufschäumen); R_{f}-Wert: 0,53 (Kieselgel, Methylenchlorid/Methanol = 9:1)) in Gegenwart von 5 Equivalenten Trifluoressigsäure in Acetonitril unter Rückfluß
   Schmelzpunkt: ab 126°C (unter Aufschäumen)
   Massenspektrum (ESI⁺): m/z = 623, 625 [M+H]⁺
   R_{f}-Wert: 0,29 (Kieselgel, Essigester/Methanol = 4:1)
(10) 4-[(3-Chlor-4-fluor-phenyl)amino]-6-{[4-(4-methyl-2-oxo-1-oxa-4,9-diaza-spiro[5.5]undec-9-yl)-1-oxo-2-buten-1-yl]amino}-7-cyclopropylmethoxy-chinazolin
   Schmelzpunkt: 125-130°C (unter Aufschäumen)
   Massenspektrum (ESI⁺): m/z = 609, 611 [M+H]⁺
   R_{f}-Wert: 0,46 (Kieselgel, Methylenchlorid/Methanol = 9:1)
(11) 4-[(3-Chlor-4-fluor-phenyl)amino]-6-{[4-(2-oxo-3-oxa-9-aza-spiro[5.5]undec-9-yl)-1-oxo-2-buten-1-yl]amino}-7-cyclopropylmethoxy-chinazolin
   Massenspektrum (ESI⁻): m/z = 592, 594 [M+H]⁻
   R_{f}-Wert: 0,24 (Kieselgel, Methylenchlorid/Methanol = 9:1)
   Das Ausgangsmaterial 2-Oxo-3-oxa-9-aza-spiro[5.5]undecan wird wie folgt dargestellt:
   (a) 4,4-Bis-(2-hydroxyethyl)-piperidin
      hergestellt durch katalytische Hydrierung von 1-Benzyl-4,4-bis-(2-hydroxyethyl)-piperidin in Ethanol in Gegenwart von Palladium auf Aktivkohle (10% Pd) Massenspektrum (ESI⁺): m/z = 174 [M+H]⁺
   (b) 1-Benzyloxycarbonyl-4,4-bis-(2-hydroxyethyl)-piperidin
      hergestellt durch Umsetzung von 4,4-Bis-(2-hydroxyethyl)-piperidin mit Chlorameisensäure-benzylester in Tetrahydrofuran in Gegenwart von Triethylamin
      R_{f}-Wert: 0,32 (Kieselgel, Essigester/Methanol = 9:1)
   (c) 9-Benzyloxycarbonyl-2-oxo-3-oxa-9-aza-spiro[5.5]undecan
      hergestellt durch Umsetzung von 1-Benzyloxycarbonyl-4,4-bis-(2-hydroxyethyl)-piperidin mit 4-Methylmorpholin-N-oxid in Methylenchlorid/Acetonitril in Gegenwart von Tetrapropylammonium-perruthenat und pulverisiertem Molekularsieb
      Massenspektrum (ESI⁻): m/z = 302 [M+H]⁻
   (d) 2-Oxo-3-oxa-9-aza-spiro[5.5]undecan
      hergestellt durch katalytische Hydrierung von
      9-Benzyloxycarbonyl-2-oxo-3-oxa-9-aza-spiro[5.5]undecan in Ethanol in Gegenwart von Palladium auf Aktivkohle (10% Pd)
      (das Produkt muß gleich nach dem schonenden Einengen weiter umgesetzt werden)
      Massenspektrum (ESI⁺): m/z = 170 [M+H]⁺

Analog den vorstehenden Beispielen und anderen literaturbekannten Verfahren können auch die folgenden Verbindungen erhalten werden:
(1) 4-[(3-Chlor-4-fluor-phenyl)amino]-6-{[4-(7-oxo-6-oxa-2,9-diaza-spiro[4.5]dec-2-yl)-1-oxo-2-buten-1-yl]amino}-7-cyclopropylmethoxy-chinazolin
(2) 4-[(3-Chlor-4-fluor-phenyl)amino]-6-{[4-(7-oxo-6,9-dioxa-2-aza-spiro[4.5]dec-2-yl)-1-oxo-2-buten-1-yl]amino}-7-cyclopropylmethoxy-chinazolin
(3) 4-[(3-Chlor-4-fluor-phenyl)amino]-6-{[4-(3-oxo-2-oxa-7-aza-spiro[4.4]non-7-yl)-1-oxo-2-buten-1-yl]amino}-7-cyclopropylmethoxy-chinazolin
(4) 4-[(3-Chlor-4-fluor-phenyl)amino]-6-{[4-(2-oxo-1,4-dioxa-9-aza-spiro[5.5]undec-9-yl)-1-oxo-2-buten-1-yl]amino}-7-cyclopropylmethoxy-chinazolin
(5) 4-[(3-Chlor-4-fluor-phenyl)amino]-6-{[4-(2-oxo-4-methyl-1-oxa-4,9-diaza-spiro[5.5]undec-9-yl)-1-oxo-2-buten-1-yl]amino}-7-cyclopropylmethoxy-chinazolin
(6) 4-[(3-Chlor-4-fluor-phenyl)amino]-6-{[4-(2-oxo-1-oxa-9-aza-spiro[5.5]undec-9-yl)-1-oxo-2-buten-1-yl]amino}-7-cyclopropylmethoxy-chinazolin
(7) 4-[(3-Chlor-4-fluor-phenyl)amino]-6-{[4-(3-oxo-1,4-dioxa-9-aza-spiro[5.5]undec-9-yl)-1-oxo-2-buten-1-yl]amino}-7-cyclopropylmethoxy-chinazolin
(8) 4-[(3-Chlor-4-fluor-phenyl)amino]-6-{[4-(4-oxo-perhydro-furo[3.4-b]pyrrol-1-yl)-1-oxo-2-buten-1-yl]amino}-7-cyclopropylmethoxy-chinazolin
(9) 4-[(3-Chlor-4-fluor-phenyl)amino]-6-{[4-(6-oxo-perhydro-furo[3.4-b]pyrrol-1-yl)-1-oxo-2-buten-1-yl]aminol-7-cyclopropylmethoxy-chinazolin
(10) 4-[(3-Chlor-4-fluor-phenyl)amino]-6-{[4-(2-oxo-perhydro-furo[2,3-c]pyrrol-5-yl)-1-oxo-2-buten-1-yl]amino}-7-cyclopropylmethoxy-chinazolin
(11) 4-[(3-Chlor-4-fluor-phenyl)amino]-6-{[4-(2-oxo-4-methyl-perhydro-pyrrolo-[3,4-b][1.4]oxazin-6-yl)-1-oxo-2-buten-1-yl]amino}-7-cyclopropylmethoxy-chinazolin
(12) 4-[(3-Chlor-4-fluor-phenyl)amino]-6-{[4-(2-oxo-perhydro-[1.4]dioxino[2,3-c]pyrrol-6-yl)-1-oxo-2-buten-1-yl]amino}-7-cyclopropylmethoxy-chinazolin
(13) 4-[(3-Chlor-4-fluor-phenyl)amino]-6-({4-[4-(2-oxo-tetrahydrofuran-4-yl)-piperidin-1-yl]-1-oxo-2-buten-1-yl}amino)-7-cyclopropylmethoxy-chinazolin
(14) 4-[(3-Chlor-4-fluor-phenyl)amino]-6-({4-[4-(2-oxo-4-methyl-morpholin-3-yl)-piperidin-1-yl]-1-oxo-2-buten-1-yl}amino)-7-cyclopropylmethoxy-chinazolin
(15) 4-[(3-Chlor-4-fluor-phenyl)amino]-6-({4-[4-(2-oxo-4-methyl-morpholin-6-yl)-piperidin-1-yl]-1-oxo-2-buten-1-yl}amino)-7-cyclopropylmethoxy-chinazolin
(16) 4-[(3-Chlor-4-fluor-phenyl)amino]-6-({4-[3-(2-oxo-6,6-dimethyl-morpholin-4-yl)-pyrrolidin-1-yl]-1-oxo-2-buten-1-yl}amino)-7-cyclopropylmethoxy-chinazolin
(17) 4-[(3-Chlor-4-fluor-phenyl)amino]-6-({4-[4-(2-oxo-6,6-dimethyl-morpholin-4-yl)-piperidin-1-yl]-1-oxo-2-buten-1-yl}amino)-7-cyclopropylmethoxy-chinazolin
(18) 4-[(3-Chlor-4-fluor-phenyl)amino]-6-[(4-{4-[(2-oxo-tetrahydrofuran-3-yl)oxy]-piperidin-1-yl}-1-oxo-2-buten-1-yl)amino]-7-cyclopropylmethoxy-chinazolin
(19) 4-[(3-Chlor-4-fluor-phenyl)amino]-6-{[4-(4-{2-[(2-oxo-tetrahydrofuran-3-yl)oxy]ethyl}-piperazin-1-yl)-1-oxo-2-buten-1-yl]amino}-7-cyclopropylmethoxy-chinazolin
(20) 4-[(3-Chlor-4-fluor-phenyl)amino]-6-{[4-(4-{[(2-oxo-tetrahydrofuran-3-yl)oxy]acetyl}-piperazin-1-yl)-1-oxo-2-buten-1-yl]amino}-7-cyclopropylmethoxy-chinazolin
(21) 4-[(3-Chlor-4-fluor-phenyl)amino]-6-{[4-(4-{[(2-oxo-tetrahydrofuran-3-yl)sulfanyl]acetyl}-piperazin-1-yl)-1-oxo-2-buten-1-yl]amino}-7-cyclopropylmethoxy-chinazolin
(22) 4-[(3-Chlor-4-fluor-phenyl)amino]-6-[(4-{4-[2-(2-oxo-6,6-dimethyl-morpholin-4-yl)ethyl]-piperazin-1-yl}-1-oxo-2-buten-1-yl)amino]-7-cyclopropylmethoxy-chinazolin
(23) 4-[(3-Chlor-4-fluor-phenyl)amino]-6-[(4-{4-[(2-oxo-6,6-dimethyl-morpholin-4-yl)acetyl]-piperazin-1-yl}-1-oxo-2-buten-1-yl)amino]-7-cyclopropylmethoxy-chinazolin
(24) 4-[(3-Chlor-4-fluor-phenyl)amino]-6-[(4-{4-[(2-oxo-6,6-dimethyl-morpholin-4-yl)methyl]-piperidin-1-yl}-1-oxo-2-buten-1-yl)amino]-7-cyclopropylmethoxy-chinazolin
(25) 4-[(3-Chlor-4-fluor-phenyl)amino]-6-{[4-(N-{1-[(5-oxo-tetrahydrofuran-2-yl)carbonyl]-piperidin-4-yl}-N-methyl-amino)-1-oxo-2-buten-1-yl]amino}-7-cyclopropylmethoxy-chinazolin
(26) 4-[(3-Chlor-4-fluor-phenyl)amino]-6-[(4-{4-[(5-oxo-tetrahydrofuran-2-yl)carbonylamino]-piperidin-1-yl}-1-oxo-2-buten-1-yl)amino]-7-cyclopropylmethoxy-chinazolin
(27) 4-[(3-Chlor-4-fluor-phenyl)amino]-6-{[4-(4-{N-[(5-oxo-tetrahydrofuran-2-yl)carbonyl]-N-methyl-amino}-piperidin-1-yl)-1-oxo-2-buten-1-yl]amino}-7-cyclopropylmethoxy-chinazolin
(28) 4-[(3-Chlor-4-fluor-phenyl)amino]-6-{[4-(3-oxo-perhydro-pyrazino-[2,1-c][1,4]oxazin-8-yl)-1-oxo-2-buten-1-yl]amino}-7-cyclobutylmethoxy-chinazolin
(29) 4-[(3-Chlor-4-fluor-phenyl)amino]-6-{[4-(3-oxo-perhydro-pyrazino-[2,1-c][1,4]oxazin-8-yl)-1-oxo-2-buten-1-yl]amino}-7-cyclobutyloxy-chinazolin
(30) 4-[(3-Chlor-4-fluor-phenyl)amino]-6-{[4-(3-oxo-perhydro-pyrazino-[2,1-c][1,4]oxazin-8-yl)-1-oxo-2-buten-1-yl]amino}-7-cyclopentyloxy-chinazolin
(31) 4-[(3-Chlor-4-fluor-phenyl)amino]-6-{[4-(3-oxo-perhydro-pyrazino-[2,1-c][1,4]oxazin-8-yl)-1-oxo-2-buten-1-yl]amino}-7-[(tetrahydrofuran-3-yl)oxy]-chinazolin
(32) 4-[(3-Chlor-4-fluor-phenyl)amino]-6-{[4-(3-oxo-perhydro-pyrazino-[2,1-c][1,4]oxazin-8-yl)-1-oxo-2-buten-1-yl]amino}-7-cyclohexyloxy-chinazolin
(33) 4-[(3-Chlor-4-fluor-phenyl)amino]-6-{[4-(3-oxo-perhydro-pyrazino-[2,1-c][1,4]oxazin-8-yl)-1-oxo-2-buten-1-yl]amino}-7-[(tetrahydropyran-4-yl)oxy]-chinazolin
(34) 4-[(3-Chlor-4-fluor-phenyl)amino]-6-{[4-(3-oxo-perhydro-pyrazino-[2,1-c][1,4]oxazin-8-yl)-1-oxo-2-buten-1-yl]amino}-7-[2-(cyclobutyloxy)ethoxy]-chinazolin
(35) 4-[(3-Chlor-4-fluor-phenyl)amino]-6-{[4-(3-oxo-perhydro-pyrazino-[2,1-c][1,4]oxazin-8-yl)-1-oxo-2-buten-1-yl]amino}-7-[2-(cyclopropylmethoxy)ethoxy]-chinazolin
(36) (R)-4-[(1-Phenylethyl)amino]-6-{[4-(3-oxo-perhydro-pyrazino[2,1-c][1,4]oxazin-8-yl)-1-oxo-2-buten-1-yl]amino}-chinazolin
(37) (R)-4-[(1-Phenylethyl)amino]-6-{[4-(3-oxo-perhydro-pyrazino[2,1-c][1,4]oxazin-8-yl)-1-oxo-2-buten-1-yl]amino}-7-methoxy-chinazolin
(38) (R)-4-[(1-Phenyfethyl)amino]-6-{[4-(3-oxo-perhydro-pyrazino[2,1-c][1,4]oxazin-8-yl)-1-oxo-2-buten-1-yl]aminol-7-ethoxy-chinazolin
(39) (R)-4-[(1-Phenylethyl)amino]-6-{[4-(3-oxo-perhydro-pyrazino[2,1-c][1,4]oxazin-8-yl)-1-oxo-2-buten-1-yl]amino}-7-[2-(methoxy)ethoxy]-chinazolin
(40) (R)-4-[(1-Phenylethyl)amino]-6-{[4-(3-oxo-perhydro-pyrazino[2,1-c][1,4]oxazin-8-yl)-1-oxo-2-buten-1-yl]amino}-7-[3-(methoxy)propyloxy]-chinazolin
(41) 4-[(3-Chlor-4-fluor-phenyl)amino]-6-({4-[4-(2-oxo-morpholin-4-yl)-piperidin-1-yl]-1-oxo-2-buten-1-yl}amino)-7-methoxy-chinazolin
(42) 4-[(3-Chlor-4-fluor-phenyl)amino]-6-({4-[4-(2-oxo-morpholin-4-yl)-piperidin-1-yl]-1-oxo-2-buten-1-yl}amino)-7-cyclopropylmethoxy-chinazolin
(43) 4-[(*R*)-(1-Phenyl-ethyl)amino]-6-({4-[4-(2-oxo-morpholin-4-yl)-piperidin-1-yl]-1-oxo-2-buten-1-yl}amino)-chinazolin
(44) 4-[(3-Chlor-4-fluor-phenyl)amino]-6-({4-[4-(6-methyl-2-oxo-morpholin-4-yl)-piperidin-1-yl]-1-oxo-2-buten-1-yl}amino)-7-methoxy-chinazolin
(45) 4-[(3-Chlor-4-fluor-phenyl)amino]-6-({4-[4-(6-methyl-2-oxo-morpholin-4-yl)-piperidin-1-yl]-1-oxo-2-buten-1-yl}amino)-7-cyclopropylmethoxy-chinazolin
(46) 4-[(*R*)-(1-Phenyl-ethyl)amino]-6-({4-[4-(6-methyl-2-oxo-morpholin-4-yl)-piperidin-1-yl]-1-oxo-2-buten-1-yl}amino)-chinazolin
(47) 4-[(3-Chlor-4-fluor-phenyl)amino]-6-{[4-(2-oxo-3-oxa-9-aza-spiro[5.5]undec-9-yl)-1-oxo-2-buten-1-yl]amino}-7-methoxy-chinazolin
(48) 4-[(3-Chlor-4-fluor-phenyl)amino]-6-{[4-(2-oxo-3-oxa-9-aza-spiro[5.5]undec-9-yl)-1-oxo-2-buten-1-yl]amino}-7-cyclopropylmethoxy-chinazolin
(49) 4-[(*R*)-(1-Phenyl-ethyl)amino]-6-{[4-(2-oxo-3-oxa-9-aza-spiro[5.5]undec-9-yl)-1-oxo-2-buten-1-yl]amino}-chinazolin
(50) 4-[(3-Chlor-4-fluor-phenyl)amino]-6-{[4-(4-methyl-2-oxo-1-oxa-4,9-diaza-spiro[5.5]undec-9-yl)-1-oxo-2-buten-1-yl]amino}-7-methoxy-chinazolin
(51) 4-[(*R*)-(1-Phenyl-ethyl)amino]-6-{[4-(4-methyl-2-oxo-1-oxa-4,9-diaza-spiro[5.5]undec-9-yl)-1-oxo-2-buten-1-yl]amino}-chinazolin
(52) 4-[(3-Chlor-4-fluor-phenyl)amino]-6-[(4-{4-[(2-oxo-tetrahydrofuran-3-yl)sulfanyl]-pipeddin-1-yl}-1-oxo-2-buten-1-yl)amino]-7-[(tetrahydrofuran-2-yl)methoxy]-chinazolin
(53) 4-[(3-Chlor-4-fluor-phenyl)amino]-6-[(4-{4-[(2-oxo-tetrahydrofuran-3-yl)sulfanyl]-piperidin-1-yl}-1-oxo-2-buten-1-yl)amino]-7-[(tetrahydropyran-4-yl)methoxy]-chinazolin

### Beispiel 5

**Dragees mit 75 mg Wirksubstanz**

| 1 Dragéekern enthält: | |
|---|---|
| Wirksubstanz | 75,0 mg |
| Calciumphosphat | 93,0 mg |
| Maisstärke | 35,5 mg |
| Polyvinylpyrrolidon | 10,0 mg |
| Hydroxypropylmethylcellulose | 15,0 mg |
| Magnesiumstearat | 1,5 mg |
| | 230,0 mg |

### Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm-Maschenweite gerieben und mit der restlichen Menge Magnesiumstearat vermischt. Dieses Granulat wird auf einer Tablettiermaschine zu Tabletten mit der gewünschten Form gepreßt.

| | |
|---|---|
| Kerngewicht: | 230 mg |
| Stempel: | 9 mm, gewölbt |

Die so hergestellten Dragéekerne werden mit einem Film überzogen, der im wesentlichen aus Hydroxypropylmethylcellulose
besteht. Die fertigen Filmdragées werden mit Bienenwachs geglänzt.

| | |
|---|---|
| Dragéegewicht: | 245 mg. |

### Beispiel 6

**Tabletten mit 100 mg Wirksubstanz**

| Zusammensetzung: | |
|---|---|
| 1 Tablette enthält: | |
| Wirksubstanz | 100,0 mg |
| Milchzucker | 80,0 mg |
| Maisstärke | 34,0 mg |
| Polyvinylpyrrolidon | 4,0 mg |
| Magnesiumstearat | 2,0 mg |
| | 220,0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2,0 mm-Maschenweite) und Trocknen im Hordentrockenschrank bei 50°C wird erneut gesiebt (1,5 mm-Maschenweite) und das Schmiermittel zugemischt. Die preßfertige Mischung wird zu Tabletten verarbeitet.

| | |
|---|---|
| Tablettengewicht: | 220 mg |
| Durchmesser: | 10 mm, biplan mit beidseitiger Facette und einseitiger Teilkerbe. |

### Beispiel 7

**Tabletten mit 150 mg Wirksubstanz**

| | |
|---|---|
| Zusammensetzung: | |
| 1 Tablette enthält: | |
| Wirksubstanz | 150,0 mg |
| Milchzucker pulv. | 89,0 mg |
| Maisstärke | 40,0 mg |
| Kolloide Kieselgelsäure | 10,0 mg |
| Polyvinylpyrrolidon | 10,0 mg |
| Magnesiumstearat | 1,0 mg |
| | 300,0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1,5 mm-Maschenweite geschlagen.
Das bei 45°C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht: | 300 mg |
| Stempel: | 10 mm, flach |

### Beispiel 8

**Hartgelatine-Kapseln mit 150 mg Wirksubstanz**

| | |
|---|---|
| 1 Kapsel enthält: | |
| Wirkstoff | 150,0 mg |
| Maisstärke getr. | ca. 180,0 mg |
| Milchzucker pulv. | ca. 87,0 mg |
| Magnesiumstearat | 3,0 mg |
| | ca. 420,0 mg |

### Herstellung:

Der Wirkstoff wird mit den Hilfsstoffen vermengt, durch ein Sieb von 0,75 mm-Maschenweite gegeben und in einem geeigneten Gerät homogen gemischt. Die Endmischung wird in Hartgelatine-Kapseln der Größe 1 abgefüllt.

| | |
|---|---|
| Kapselfüllung: | ca. 320 mg |
| Kapselhülle: | Hartgelatine-Kapsel Größe 1. |

### Beispiel 9

**Suppositorien mit 150 mg Wirksubstanz**

| | |
|---|---|
| 1 Zäpfchen enthält: | |
| Wirkstoff | 150,0 mg |
| Polyäthylenglykol 1500 | 550,0 mg |
| Polyäthylenglykol 6000 | 460,0 mg |
| Polyoxyäthylensorbitanmonostearat | 840,0 mg |
| | 2 000,0 mg |

### Herstellung:

Nach dem Aufschmelzen der Suppositorienmasse wird der Wirkstoff darin homogen verteilt und die Schmelze in vorgekühlte Formen gegossen.

### Beispiel 10

**Suspension mit 50 mg Wirksubstanz**

| 100 ml Suspension enthalten: | |
|---|---|
| Wirkstoff | 1,00 g |
| Carboxymethylcellulose-Na-Salz | 0,10 g |
| p-Hydroxybenzoesäuremethylester | 0,05 g |
| p-Hydroxybenzoesäurepropylester | 0,01 g |
| Rohrzucker | 10,00 g |
| Glycerin | 5,00 g |
| Sorbitlösung 70%ig | 20,00 g |
| Aroma | 0,30 g |
| Wasser dest. | ad 100 ml |

### Herstellung:

Dest. Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.

5 ml Suspension enthalten 50 mg Wirkstoff.

### Beispiel 11

**Ampullen mit 10 mg Wirksubstanz**

| | |
|---|---|
| Zusammensetzung: | |
| Wirkstoff | 10,0 mg |
| 0,01 n Salzsäure s.q. | |
| Aqua bidest | ad 2,0 ml |

### Herstellung:

Die Wirksubstanz wird in der erforderlichen Menge 0,01 n HCl gelöst, mit Kochsalz isotonisch gestellt, sterilfiltriert und in 2 ml Ampullen abgefüllt.

### Beispiel 12

**Ampullen mit 50 mg Wirksubstanz**

| Zusammensetzung: | |
|---|---|
| Wirkstoff | 50,0 mg |
| 0,01 n Salzsäure s.q. | |
| Aqua bidest | ad 10,0 ml |

### Herstellung:

Die Wirksubstanz wird in der erforderlichen Menge 0,01 n HCl gelöst, mit Kochsalz isotonisch gestellt, sterilfiltriert und in 10 ml Ampullen abgefüllt.

### Beispiel 13

**Kapseln zur Pulverinhalation mit 5 mg Wirksubstanz**

| 1 Kapsel enthält: | |
|---|---|
| Wirksubstanz | 5,0 mg |
| Lactose für Inhalationszwecke | 15,0 mg |
| | 20,0 mg |

### Herstellung:

Die Wirksubstanz wird mit Lactose für Inhalationszwecke gemischt. Die Mischung wird auf einer Kapselmaschine in Kapseln (Gewicht der Leerkapsel ca. 50 mg) abgefüllt.

| | |
|---|---|
| Kapselgewicht: | 70,0 mg |
| Kapselgröße: | 3 |

### Beispiel 14

**Inhalationslösung für Handvernebler mit 2,5 mg Wirksubstanz**

| 1 Hub enthält: | |
|---|---|
| Wirksubstanz | 2,500 mg |
| Benzalkoniumchlorid | 0,001 mg |
| 1 N-Salzsäure q.s. Ethanol/Wasser (50/50) | ad 15,000 mg |

### Herstellung:

Die Wirksubstanz und Benzalkoniumchlorid werden in Ethanol/Wasser (50/50) gelöst. Der pH-Wert der Lösung wird mit 1 N-Salzsäure eingestellt. Die eingestellte Lösung wird filtriert und in für den Handvernebler geeignete Behälter (Kartuschen) abgefüllt.

| | |
|---|---|
| Füllmasse des Behälters: | 4,5 g |

## Patentansprüche

1. Bicyclische Heterocyclen der allgemeinen Formel 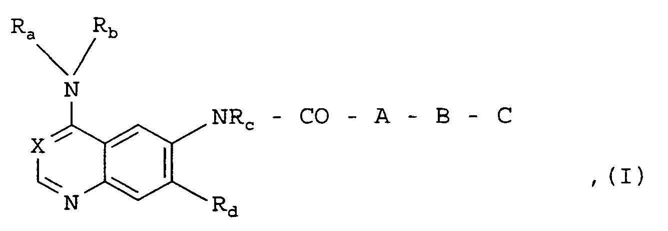 in der
X eine durch eine Cyanogruppe substituierte Methingruppe oder ein Stickstoffatom,
Rₐ ein Wasserstoffatom oder eine C₁₋₄-Alkylgruppe,
R_{b} eine Phenyl-, Benzyl- oder 1-Phenylethylgruppe, in denen der Phenylkern jeweils durch die Reste R₁ bis R₃ substituiert ist, wobei
R₁ und R₂, die gleich oder verschieden sein können, jeweils ein Wasserstoff-, Fluor-, Chlor-, Brom- oder Jodatom,
eine C₁₋₄-Alkyl-, Hydroxy-, C₁₋₄-Alkoxy-, C₃₋₆-Cycloalkyl-, C₄₋₆-Cycloalkoxy-, C₂₋₅-Alkenyl- oder C₂₋₅-Alkinylgruppe,
eine Aryl-, Aryloxy-, Arylmethyl- oder Arylmethoxygruppe,
eine C₃₋₅-Alkenyloxy- oder C₃₋₅-Alkinyloxygruppe, wobei der ungesättigte Teil nicht mit dem Sauerstoffatom verknüpft sein kann,
eine C₁₋₄-Alkylsulfenyl-, C₁₋₄-Alkylsulfinyl-, C₁₋₄-Alkylsulfonyl-, C₁₋₄-Alkylsulfonyloxy-, Trifluormethylsulfenyl-, Trifluormethylsulfinyl- oder Trifluormethylsulfonylgruppe,
eine durch 1 bis 3 Fluoratome substituierte Methyl- oder Methoxygruppe,
eine durch 1 bis 5 Fluoratome substituierte Ethyl- oder Ethoxygruppe,
eine Cyano- oder Nitrogruppe oder eine gegebenenfalls durch eine oder zwei C₁₋₄-Alkylgruppen substituierte Aminogruppe, wobei die Substituenten gleich oder verschieden sein können, oder
R₁ zusammen mit R₂, sofern diese an benachbarte Kohlenstoffatome gebunden sind, eine -CH=CH-CH=CH-, -CH=CH-NH- oder -CH=N-NH-Gruppe und
R₃ ein Wasserstoff-, Fluor-, Chlor- oder Bromatom,
eine C₁₋₄-Alkyl-, Trifluormethyl- oder C₁₋₄-Alkoxygruppe darstellen,
R_{c} ein Wasserstoffatom oder eine C₁₋₄-Alkylgruppe,
R_{d} ein Wasserstoffatom, eine C₁₋₆Alkoxy-, C₄₋₇Cycloalkoxy- oder C₃₋₇-Cycloalkyl-C₁₋₄-alkoxygruppe,
eine C₂₋₆-Alkoxygruppe, die ab Position 2 durch eine Hydroxy-, C₁₋₄-Alkoxy-, C₄₋₇-Cycloalkoxy-, C₃₋₇-Cycloalkyl-C₁₋₃-alkoxy-, Di-(C₁₋₄-Alkyl)-amino-, Pyrrolidino-, Piperidino-, Morpholino-, Piperazino- oder 4-(C₁₋₄-Alkyl)-piperazinogruppe substituiert ist, wobei die vorstehend erwähnten cyclischen Iminogruppen durch eine oder zwei C₁₋₂-Alkylgruppen substituiert sein können,
eine Tetrahydrofuran-3-yloxy-, Tetrahydropyran-3-yloxy-, Tetrahydropyran-4-yloxy-, Tetrahydrofuranylmethoxy- oder Tetrahydropyranylmethoxygruppe,
A eine gegebenenfalls durch eine Methyl- oder Trifluormethylgruppe oder durch zwei Methylgruppen substituierte 1,1- oder 1,2-Vinylengruppe,
eine gegebenenfalls durch eine Methyl- oder Trifluormethylgruppe oder durch zwei Methylgruppen substituierte 1,3-Butadien-1,4-ylengruppe oder eine Ethinylengruppe,
B eine C₁₋₆-Alkylengruppe, in der ein oder zwei Wasserstoffatome durch Fluoratome ersetzt sein können, oder, falls B an ein Kohlenstoffatom der Gruppe C gebunden ist, auch eine Bindung,
C eine Pyrrolidinogruppe, in der die beiden Wasserstoffatome in 2-Stellung durch eine Gruppe D ersetzt sind, in der
D eine gegebenenfalls durch eine oder zwei C₁₋₂-Alkylgruppen substituierte -CH₂-O-CO-CH₂-, -CH₂CH₂-O-CO-, -CH₂-O-CO-CH₂CH₂-, -CH₂CH₂-O-CO-CH₂- oder -CH₂CH₂CH₂-O-CO-Brücke darstellt,
eine Pyrrolidinogruppe, in der die beiden Wasserstoffatome in 3-Stellung durch eine Gruppe E ersetzt sind, in der
E eine gegebenenfalls durch eine oder zwei C₁₋₂-Alkylgruppen substituierte -O-CO-CH₂CH₂-, -CH₂-O-CO-CH₂-, -CH₂CH₂-O-CO-, -O-CO-CH₂CH₂CH₂-, -CH₂-O-CO-CH₂CH₂-, -CH₂CH₂-O-CO-CH₂-, -CH₂CH₂CH₂-O-CO-, -O-CO-CH₂-NR₄-CH₂-, -CH₂-O-CO-CH₂-NR₄-, -O-CO-CH₂-O-CH₂- oder -CH₂-O-CO-CH₂-O-Brücke darstellt,
wobei R₄ ein Wasserstoffatom oder eine C₁₋₄-Alkylgruppe bedeutet,
eine Piperidino- oder Hexahydroazepinogruppe, in denen die beiden Wasserstoffatome in 2-Stellung durch eine Gruppe D ersetzt sind, wobei D wie vorstehend erwähnt definiert ist,
eine Piperidino- oder Hexahydroazepinogruppe, in denen jeweils die beiden Wasserstoffatome in 3-Stellung oder in 4-Stellung durch eine Gruppe E ersetzt sind, wobei E wie vorstehend erwähnt definiert ist,
eine Piperazino- oder 4-(C₁₋₄-Alkyl)-piperazinogruppe, in denen die beiden Wasserstoffatome in 2-Stellung oder in 3-Stellung des Piperazinoringes durch eine Gruppe D ersetzt sind, wobei D wie vorstehend erwähnt definiert ist,
eine Pyrrolidino- oder Piperidinogruppe, in denen zwei vicinale Wasserstoffatome durch eine gegebenenfalls durch eine oder zwei C₁₋₂-Alkylgruppen substituierte -O-CO-CH₂-, -CH₂-O-CO-, -O-CO-CH₂CH₂-, -CH₂-O-CO-CH₂-, -CH₂CH₂-O-CO-, -O-CO-CH₂-NR₄- oder -O-CO-CH₂-O-Brücke ersetzt sind, wobei R₄ wie vorstehend erwähnt definiert ist und die Heteroatome der vorstehend erwähnten Brücken nicht an die 2- oder 5-Stellung des Pyrrolidinringes und nicht an die 2- oder 6-Stellung des Piperidinoringes gebunden sind,
eine Piperazino- oder 4-(C₁₋₄-Alkyl)-piperazinogruppe, in denen ein Wasserstoffatom in 2-Stellung zusammen mit einem Wasserstoffatom in 3-Stellung des Piperazinoringes durch eine gegebenenfalls durch eine oder zwei C₁₋₂-Alkylgruppen substituierte -CH₂-O-CO-CH₂- oder -CH₂CH₂-O-CO-Brücke ersetzt sind,
eine Piperazinogruppe, in der ein Wasserstoffatom in 3-Stellung zusammen mit dem Wasserstoffatom in 4-Stellung durch eine gegebenenfalls durch eine oder zwei C₁₋₂-Alkylgruppen substituierte -CO-O-CH₂CH₂- oder -CH₂-O-CO-CH₂-Brücke ersetzt sind, wobei jeweils das linke Ende der vorstehend erwähnten Brücken an die 3-Stellung des Piperazinoringes gebunden ist,
eine durch den Rest R₅ substituierte Pyrrolidino-, Piperidino- oder Hexahydroazepinogruppe, in denen
R₅ eine gegebenenfalls durch eine oder zwei C₁₋₂-Alkylgruppen substituierte 2-Oxo-tetrahydrofuranyl-, 2-Oxo-tetrahydropyranyl-, 2-Oxo-1,4-dioxanyl- oder 2-Oxo-4-(C₁₋₄-alkyl)-morpholinylgruppe darstellt,
eine in 3-Stellung durch eine 2-Oxo-morpholinogruppe substituierte Pyrrolidinogruppe, wobei die 2-Oxo-morpholinogruppe durch eine oder zwei C₁₋₂-Alkylgruppen substituiert sein kann,
eine in 3- oder 4-Stellung durch eine 2-Oxo-morpholinogruppe substituierte Piperidino- oder Hexahydroazepinogruppe, wobei die 2-Oxo-morpholinogruppe durch eine oder zwei C₁₋₂-Alkylgruppen substituiert sein kann,
eine an einem Ringkohlenstoffatom durch R₅ substituierte 4-(C₁₋₄-Alkyl)-piperazino- oder 4-(C₁₋₄-Alkyl)-homopiperazinogruppe, in denen R₅ wie vorstehend erwähnt definiert ist,
eine in 4-Stellung durch den Rest R₆ substituierte Piperazino- oder Homopiperazinogruppe, in denen
R₆ eine gegebenenfalls durch eine oder zwei C₁₋₂-Alkylgruppen substituierte 2-Oxo-tetrahydrofuran-3-yl-, 2-Oxo-tetrahydrofuran-4-yl-, 2-Oxo-tetrahydropyran-3-yl-, 2-Oxo-tetrahydropyran-4-yl- oder 2-Oxo-tetrahydropyran-5-yl-Gruppe darstellt,
eine in 3-Stellung durch eine (R₄NR₆)-, R₆O-, R₆S-, R₆SO- oder R₆SO₂-Gruppe substituierte Pyrrolidinogruppe, wobei R₄ und R₆ wie vorstehend erwähnt definiert sind,
eine in 3- oder 4-Stellung durch eine (R₄NR₆)-, R₆O-, R₆S-, R₆SO- oder R₆SO₂-Gruppe substituierte Piperidino- oder Hexahydroazepinogruppe, in denen R₄ und R₆ wie vorstehend erwähnt definiert sind,
eine durch eine R₅-C₁₋₄-alkyl-, (R₄NR₆)-C₁₋₄-alkyl-, R₆O-C₁₋₄-alkyl-, R₆S-C₁₋₄-alkyl-, R₆SO-C₁₋₄-alkyl-, R₆SO₂-C₁₋₄alkyl- oder R₄NR₆-CO-Gruppe substituierte Pyrrolidino-, Piperidino- oder Hexahydroazepinogruppe, in denen R₄ bis R₆ wie vorstehend erwähnt definiert sind,
eine in 3-Stellung durch eine R₅-CO-NR₄-, R₅-C₁₋₄-alkylen-CONR₄-, (R₄NR₆)-C₁₋₄-alkylen-CONR₄-, R₆O-C₁₋₄-alkylen-CONR₄-, R₆S-C₁₋₄-alkylen-CONR₄-, R₆SO-C₁₋₄-alkylen-CONR₄-, R₆SO₂-C₁₋₄-alkylen-CONR₄-, 2-Oxo-morpholino-C₁₋₄-alkylen-CONR₄-, R₅-C₁₋₄-alkylen-Y- oder C₂₋₄-Alkyl-Y-Gruppe substituierte Pyrrolidinogruppe, wobei der C₂₋₄-Alkylteil der C₂₋₄-Alkyl-Y-Gruppe jeweils ab Position 2 durch eine (R₄NR₆)-, R₆O-, R₆S-, R₆SO- oder R₆SO₂-Gruppe substituiert ist und der 2-Oxo-morpholinoteil durch eine oder zwei C₁₋₂-Alkylgruppen substituiert sein kann, in denen
R₄ bis R₆ wie vorstehend erwähnt definiert sind und
Y ein Sauerstoff- oder Schwefelatom, eine lmino-, N-(C₁₋₄-Alkyl)-imino-, Sulfinyl- oder Sulfonylgruppe darstellt,
eine in 3- oder 4-Stellung durch eine R₅-CO-NR₄-, R₅-C₁₋₄-alkylen-CONR₄-, (R₄NR₆)-C₁₋₄-alkylen-CONR₄-, R₆O-C₁₋₄-alkylen-CONR₄-, R₆S-C₁₋₄-alkylen-CONR₄-, R₆SO-C₁₋₄-alkylen-CONR₄-, R₆SO₂-C₁₋₄-alkylen-CONR₄-, 2-Oxo-morpholino-C₁₋₄-alkylen-CONR₄-, R₅-C₁₋₄-alkylen-Y- oder C₂₋₄-Alkyl-Y-Gruppe substituierte Piperidino- oder Hexahydroazepinogruppe, in denen Y wie vorstehend erwähnt definiert ist, der 2-Oxo-morpholinoteil durch eine oder zwei C₁₋₂-Alkylgruppen substituiert sein kann und der C₂₋₄-Alkylteil der C₂₋₄-Alkyl-Y-Gruppe jeweils ab Position 2 durch eine (R₄NR₆)-, R₆O-, R₆S-, R₆SO- oder R₆SO₂-Gruppe substituiert ist, wobei R₄ bis R₆ wie vorstehend erwähnt definiert sind,
eine an einem Ringkohlenstoffatom durch eine R₅-C₁₋₄-alkyl-, (R₄NR₆)-C₁₋₄-alkyl-, R₆O-C₁₋₄-alkyl-, R₆S-C₁₋₄-alkyl-, R₆SO-C₁₋₄-alkyl-, R₆SO₂-C₁₋₄-alkyl- oder R₄NR₆-CO-Gruppe substituierte 4-(C₁₋₄-Alkyl)-piperazino- oder 4-(C₁₋₄-Alkyl)-homopiperazinogruppe, in denen R₄ bis R₆ wie vorstehend erwähnt definiert sind,
eine in 4-Stellung durch eine R₅-C₁₋₄-alkyl-, R₅-CO-, R₅-C₁₋₄-alkylen-CO-, (R₄NR₆)-C₁₋₄-alkylen-CO-, R₆O-C₁₋₄-alkylen-CO-, R₆S-C₁₋₄-alkylen-CO-, R₆SO-C₁₋₄-alkylen-CO- oder R₆SO₂-C₁₋₄-alkylen-CO-Gruppe substituierte Piperazino- oder Homopiperazinogruppe, in denen R₄ bis R₆ wie vorstehend erwähnt definiert sind,
eine in 4-Stellung durch eine C₂₋₄-Alkylgruppe substituierte Piperazino- oder Homopiperazinogruppe, in denen die C₂₋₄-Alkylgruppe jeweils ab Position 2 durch eine (R₄NR₆)-, R₆O-, R₆S-, R₆SO- oder R₆SO₂-Gruppe substituiert ist, wobei R₄ und R₆ wie vorstehend erwähnt definiert sind,
eine durch eine 2-Oxo-morpholino-C₁₋₄-alkylgruppe substituierte Pyrrolidino-, Piperidino- oder Hexahydroazepinogruppe, in denen der 2-Oxo-morpholinoteil durch eine oder zwei C₁₋₂-Alkylgruppen substituiert sein kann,
eine in 3-Stellung durch eine C₂₋₄-Alkyl-Y-Gruppe substituierte Pyrrolidinogruppe, in denen Y wie vorstehend erwähnt definiert ist und der C₂₋₄-Alkylteil der C₂₋₄-Alkyl-Y-Gruppe jeweils ab Position 2 durch eine gegebenenfalls durch eine oder zwei C₁₋₂-Alkylgruppen substituierte 2-Oxo-morpholinogruppe substituiert ist,
eine in 3- oder 4-Stellung durch eine C₂₋₄-Alkyl-Y-Gruppe substituierte Piperidino- oder Hexahydroazepinogruppe, in denen Y wie vorstehend erwähnt definiert ist und der C₂₋₄-Alkylteil der C₂₋₄-Alkyl-Y-Gruppe jeweils ab Position 2 durch eine gegebenenfalls durch eine oder zwei C₁₋₂-Alkylgruppen substituierte 2-Oxo-morpholinogruppe substituiert ist,
eine an einem Ringkohlenstoffatom durch eine 2-Oxo-morpholino-C₁₋₄-alkyl-Gruppe substituierte 4-(C₁₋₄-Alkyl)-piperazino- oder 4-(C₁₋₄-Alkyl)-homopiperazinogruppe, in denen der 2-Oxo-morpholinoteil durch eine oder zwei C₁₋₂-Alkylgruppen substituiert sein kann,
eine in 4-Stellung durch eine 2-Oxo-morpholino-C₁₋₄-alkylen-CO-Gruppe substituierte Piperazino- oder Homopiperazinogruppe, in denen der 2-Oxo-morpholinoteil durch eine oder zwei C₁₋₂-Alkylgruppen substituiert sein kann,
eine in 4-Stellung durch eine C₂₋₄-Alkylgruppe substituierte Piperazino- oder Homopiperazinogruppe, in denen der C₂₋₄-Alkylteil jeweils ab Position 2 durch eine gegebenenfalls durch eine oder zwei C₁₋₂-Alkylgruppen substituierte 2-Oxo-morpholinogruppe substituiert ist,
eine in 1-Stellung durch den Rest R₆, durch eine R₅-C₁₋₄-alkyl-, R₅-CO-, R₅-C₁₋₄-alkylen-CO-, (R₄NR₆)-C₁₋₄-alkylen-CO-, R₆O-C₁₋₄-alkylen-CO-, R₆S-C₁₋₄-alkylen-CO-, R₆SO-C₁₋₄-alkylen-CO-, R₆SO₂-C₁₋₄-alkylen-CO- oder 2-Oxo-morpholino-C₁₋₄-alkylen-CO-Gruppe substituierte Pyrrolidinyl- oder Piperidinylgruppe, in denen R₄ bis R₆ wie vorstehend erwähnt definiert sind und der 2-Oxo-morpholinoteil durch eine oder zwei C₁₋₂-Alkylgruppen substituiert sein kann,
eine in 1-Stellung durch eine C₂₋₄-Alkylgruppe substituierte Pyrrolidinyl- oder Piperidinylgruppe, in denen der C₂₋₄-Alkylteil jeweils ab Position 2 durch eine (R₄NR₆)-, R₆O-, R₆S-, R₆SO-, R₆SO₂- oder 2-Oxo-morpholinogruppe substituiert ist, wobei R₄ und R₆ wie vorstehend erwähnt definiert sind und der 2-Oxo-morpholinoteil durch eine oder zwei C₁₋₂-Alkylgruppen substituiert sein kann,
eine jeweils am Ringstickstoffatom durch den Rest R₆, durch eine R₅-C₁₋₄-alkyl-, R₅-CO-, R₅-C₁₋₄-alkylen-CO-, (R₄NR₆)-C₁₋₄-alkylen-CO-, R₆O-C₁₋₄-alkylen-CO-, R₆S-C₁₋₄-alkylen-CO-, R₆SO-C₁₋₄-alkylen-CO-, R₆SO₂-C₁₋₄-alkylen-CO- oder 2-Oxo-morpholino-C₁₋₄-alkylen-CO-Gruppe substituierte Pyrrolidin-3-yl-NR₄-, Piperidin-3-yl-NR₄- oder Piperidin-4-yl-NR₄-Gruppe, in denen R₄ bis R₆ wie vorstehend erwähnt definiert sind und der 2-Oxo-morpholinoteil durch eine oder zwei C₁₋₂-Alkylgruppen substituiert sein kann,
eine jeweils am Ringstickstoffatom durch eine C₂₋₄-Alkylgruppe substituierte Pyrrolidin-3-yl-NR₄-, Piperidin-3-yl-NR₄- oder Piperidin-4-yl-NR₄-Gruppe, in denen der C₂₋₄-Alkylteil jeweils ab Position 2 durch eine (R₄NR₆)-, R₆O-, R₆S-, R₆SO-, R₆SO₂- oder 2-Oxo-morpholinogruppe substituiert ist, wobei R₄ und R₆ wie vorstehend erwähnt definiert sind und der 2-Oxo-morpholinoteil durch eine oder zwei C₁₋₂-Alkylgruppen substituiert sein kann,
eine R₅-C₁₋₄-alkylen-NR₄-Gruppe, in der R₄ und R₅ wie vorstehend erwähnt definiert sind, oder
eine C₂₋₄-Alkyl-NR₄-Gruppe, in denen der C₂₋₄-Alkylteil jeweils ab Position 2 durch eine (R₄NR₆)-, R₆O-, R₆S-, R₆SO-, R₆SO₂- oder 2-Oxo-morpholinogruppe substituiert ist, wobei R₄ und R₆ wie vorstehend erwähnt definiert sind und der 2-Oxo-morpholinoteil durch eine oder zwei C₁₋₂-Alkylgruppen substituiert sein kann, bedeuten,
wobei unter den vorstehend erwähnten Arylteilen eine Phenylgruppe zu verstehen ist, die jeweils durch R' mono- oder disubstituiert sein kann, wobei die Substituenten gleich oder verschieden sein können und
R' ein Fluor-, Chlor-, Brom- oder lodatom, eine C₁₋₂-Alkyl-, Trifluormethyl- oder C₁₋₂-Alkoxygruppe oder
zwei Reste R', sofern sie an benachbarte Kohlenstoffatome gebunden sind, zusammen eine C₃₋₄-Alkylen-, Methylendioxy- oder 1,3-Butadien-1,4-ylengruppe darstellen,
deren Tautomeren, deren Stereoisomere und deren Salze.

2. Bicyclische Heterocyclen der allgemeinen Formel I gemäß Anspruch 1, in der
X ein Stickstoffatom,
Rₐ ein Wasserstoffatom,
R_{b} eine Phenyl-, Benzyl- oder 1-Phenylethylgruppe, in denen der Phenylkern jeweils durch die Reste R₁ bis R₃ substituiert ist, wobei
R₁ und R₂, die gleich oder verschieden sein können, jeweils eine Methylgruppe oder ein Wasserstoff-, Fluor-, Chlor- oder Bromatom und
R₃ ein Wasserstoffatom darstellen,
R_{c} ein Wasserstoffatom,
R_{d} ein Wasserstoffatom, eine Methoxy-, Ethoxy-, C₄₋₆-Cycloalkoxy-, C₃₋₆-Cycloalkylmethoxy-, 2-Methoxy-ethoxy-, 2-(Cyclobutyloxy)-ethoxy-, 2-(Cyclopentyloxy)-ethoxy-, 2-(Cyclohexyloxy)-ethoxy-,2-(Cyclopropylmethoxy)-ethoxy-, Tetrahydrofuran-3-yloxy-, Tetrahydropyran-4-yloxy-, Tetrahydrofuran-2-ylmethoxy-, Tetrahydrofuran-3-ylmethoxy-, Tetrahydropyran-2-ylmethoxy- oder Tetrahydropyran-4-yl-methoxygruppe,
A eine 1,2-Vinylengruppe,
B eine Methylen- oder Ethylengruppe oder, falls B an ein Kohlenstoffatom der Gruppe C gebunden ist, auch eine Bindung,
C eine Pyrrolidinogruppe, in der die beiden Wasserstoffatome in 3-Stellung durch eine Gruppe E ersetzt sind, in der
E eine gegebenenfalls durch eine oder zwei Methylgruppen substituierte -O-CO-CH₂CH₂-, -CH₂-O-CO-CH₂-, -CH₂CH₂-O-CO-, -CH₂CH₂-O-CO-CH₂-, -O-CO-CH₂-NR₄-CH₂-, -CH₂-O-CO-CH₂-NR₄-, -O-CO-CH₂-O-CH₂- oder -CH₂-O-CO-CH₂-O-Brücke darstellt,
wobei R₄ eine Methyl- oder Ethylgruppe bedeutet,
eine Piperidinogruppe, in der die beiden Wasserstoffatome in 3-Stellung oder in 4-Stellung durch eine Gruppe E ersetzt sind, wobei E wie vorstehend erwähnt definiert ist,
eine Pyrrolidino- oder Piperidinogruppe, in denen zwei vicinale Wasserstoffatome durch eine gegebenenfalls durch eine oder zwei Methylgruppen substituierte -O-CO-CH₂-, -CH₂-O-CO-, -O-CO-CH₂-NR₄- oder -O-CO-CH₂-O-Brücke ersetzt sind, wobei R₄ wie vorstehend erwähnt definiert ist und die Heteroatome der vorstehend erwähnten Brücken nicht an die 2- oder 5-Stellung des Pyrrolidinringes und nicht an die 2- oder 6-Stellung des Piperidinoringes gebunden sind,
eine Piperazinogruppe, in der ein Wasserstoffatom in 3-Stellung zusammen mit dem Wasserstoffatom in 4-Stellung durch eine gegebenenfalls durch eine oder zwei Methylgruppen substituierte -CO-O-CH₂CH₂- oder -CH₂-O-CO-CH₂-Brücke ersetzt sind, wobei jeweils das linke Ende der vorstehend erwähnten Brücken an die 3-Stellung des Piperazinoringes gebunden ist,
eine durch den Rest R₅ substituierte Pyrrolidino- oder Piperidinogruppe, in denen
R₅ eine gegebenenfalls durch eine oder zwei Methylgruppen substituierte 2-Oxo-tetrahydrofuranyl-, 2-Oxo-1,4-dioxanyl- oder 2-Oxo-4-(C₁₋₄-alkyl)-morpholinylgruppe darstellt,
eine in 3-Stellung durch eine 2-Oxo-morpholinogruppe substituierte Pyrrolidinogruppe, wobei die 2-Oxo-morpholinogruppe durch eine oder zwei Methylgruppen substituiert sein kann,
eine in 3- oder 4-Stellung durch eine 2-Oxo-morpholinogruppe substituierte Piperidinogruppe, wobei die 2-Oxo-morpholinogruppe durch eine oder zwei Methylgruppen substituiert sein kann,
eine in 4-Stellung durch den Rest R₆ substituierte Piperazinogruppe, in der
R₆ eine gegebenenfalls durch eine oder zwei Methylgruppen substituierte 2-Oxo-tetrahydrofuran-3-yl- oder 2-Oxo-tetrahydrofuran-4-yl-Gruppe darstellt,
eine in 3-Stellung durch eine (R₄NR₆)-, R₆O-, R₆S-, R₆SO- oder R₆SO₂-Gruppe substituierte Pyrrolidinogruppe, wobei R₄ und R₆ wie vorstehend erwähnt definiert sind,
eine in 3- oder 4-Stellung durch eine (R₄NR₆)-, R₆O-, R₆S-, R₆SO- oder R₆SO₂-Gruppe substituierte Piperidinogruppe, wobei R₄ und R₆ wie vorstehend erwähnt definiert sind,
eine durch eine (R₄NR₆)-C₁₋₂-alkyl-, HNR₆-CO- oder R₄NR₆-CO-Gruppe substituierte Pyrrolidino- oder Piperidinogruppe, wobei R₄ und R₆ wie vorstehend erwähnt definiert sind,
eine in 3-Stellung durch eine R₅-CO-NH- oder R₅-CO-NR₄-Gruppe substituierte Pyrrolidinogruppe, wobei R₄ und R₅ wie vorstehend erwähnt definiert sind,
eine in 3- oder 4-Stellung durch eine R₅-CO-NH- oder R₅-CO-NR₄-Gruppe substituierte Piperidinogruppe, wobei R₄ und R₅ wie vorstehend erwähnt definiert sind,
eine in 4-Stellung durch eine R₅-C₁₋₂-alkyl-, R₅-CO-, R₅-C₁₋₂-alkylen-CO-, (R₄NR₆)-C₁₋₂-alkylen-CO-, R₆O-C₁₋₂-alkylen-CO-, R₆S-C₁₋₂-alkylen-CO-, R₆SO-C₁₋₂-alkylen-CO- oder R₆SO₂-C₁₋₂-alkylen-CO-Gruppe substituierte Piperazinogruppe, in der R₄ bis R₆ wie vorstehend erwähnt definiert sind,
eine in 4-Stellung durch eine C₂₋₃-Alkylgruppe substituierte Piperazinogruppe, in der die C₂₋₃-Alkylgruppe jeweils ab Position 2 durch eine (R₄NR₆)-, R₆O-, R₆S-, R₆SO- oder R₆SO₂-Gruppe substituiert ist, wobei R₄ und R₆ wie vorstehend erwähnt definiert sind,
eine durch eine 2-Oxo-morpholino-C₁₋₂-alkylgruppe substituierte Pyrrolidino- oder Piperidinogruppe, in denen der 2-Oxo-morpholinoteil durch eine oder zwei Methylgruppen substituiert sein kann,
eine in 4-Stellung durch eine 2-Oxo-morpholino-C₁₋₂-alkylen-CO-Gruppe substituierte Piperazinogruppe, in der der 2-Oxo-morpholinoteil durch eine oder zwei Methylgruppen substituiert sein kann,
eine in 4-Stellung durch eine C₂₋₃-Alkylgruppe substituierte Piperazinogruppe, in der der C₂₋₃-Alkylteil jeweils ab Position 2 durch eine gegebenenfalls durch eine oder zwei Methylgruppen substituierte 2-Oxo-morpholinogruppe substituiert ist,
eine in 1-Stellung durch den Rest R₆, durch eine R₅-C₁₋₂-alkyl-, R₅-CO-, R₅-C₁₋₂-alkylen-CO-, (R₄NR₆)-C₁₋₂-alkylen-CO-, R₆O-C₁₋₂-alkylen-CO-, R₆S-C₁₋₂-alkylen-CO-, R₆SO-C₁₋₂-alkylen-CO-, R₆SO₂-C₁₋₂-alkylen-CO- oder 2-Oxo-morpholino-C₁₋₂-alkylen-CO-Gruppe substituierte Piperidinylgruppe, wobei R₄ bis R₆ wie vorstehend erwähnt definiert sind und der 2-Oxo-morpholinoteil durch eine oder zwei Methylgruppen substituiert sein kann,
eine in 1-Stellung durch eine C₂₋₃-Alkylgruppe substituierte Piperidinylgruppe, in der der C₂₋₃-Alkylteil jeweils ab Position 2 durch eine (R₄NR₆)-, R₆O-, R₆S-, R₆SO-, R₆SO₂- oder 2-Oxo-morpholinogruppe substituiert ist, wobei R₄ und R₆ wie vorstehend erwähnt definiert sind und der 2-Oxo-morpholinoteil durch eine oder zwei Methylgruppen substituiert sein kann,
eine jeweils am Ringstickstoffatom durch den Rest R₆, durch eine R₅-C₁₋₂-alkyl-, R₅-CO-, R₅-C₁₋₂-alkylen-CO-, (R₄NR₆)-C₁₋₂-alkylen-CO-, R₆O-C₁₋₂-alkylen-CO-, R₆S-C₁₋₂-alkylen-CO-, R₆SO-C₁₋₂-alkylen-CO-, R₆SO₂-C₁₋₂-alkylen-CO- oder 2-Oxo-morpholino-C₁₋₂-alkylen-CO-Gruppe substituierte Pyrrolidin-3-yl-NR₄-, Piperidin-3-yl-NR₄- oder Piperidin-4-yl-NR₄-Gruppe, in denen R₄ bis R₆ wie vorstehend erwähnt definiert sind und der 2-Oxo-morpholinoteil durch eine oder zwei Methylgruppen substituiert sein kann, oder
eine jeweils am Ringstickstoffatom durch eine C₂₋₃-Alkylgruppe substituierte Pyrrolidin-3-yl-NR₄-, Piperidin-3-yl-NR₄- oder Piperidin-4-yl-NR₄-Gruppe, in denen der C₂₋₃-Alkylteil jeweils ab Position 2 durch eine (R₄NR₆)-, R₆O-, R₆S-, R₆SO-, R₆SO₂- oder 2-Oxo-morpholinogruppe substituiert ist, wobei R₄ und R₆ wie vorstehend erwähnt definiert sind und der 2-Oxo-morpholinoteil durch eine oder zwei Methylgruppen substituiert sein kann,
bedeuten, deren Tautomeren, deren Stereoisomere und deren Salze.

3. Bicyclische Heterocyclen der allgemeinen Formel I gemäß Anspruch 1, in der
X ein Stickstoffatom,
Rₐ ein Wasserstoffatom,
R_{b} eine Phenyl-, Benzyl- oder 1-Phenylethylgruppe, in denen der Phenylkern jeweils durch die Reste R₁ bis R₃ substituiert ist, wobei
R₁ und R₂, die gleich oder verschieden sein können, jeweils eine Methylgruppe oder ein Wasserstoff-, Fluor-, Chlor- oder Bromatom und
R₃ ein Wasserstoffatom darstellen,
R_{c} ein Wasserstoffatom,
R_{d} ein Wasserstoffatom, eine Methoxy-, Ethoxy-, C₄₋₆-Cycloalkoxy-, C₃₋₆-Cycloalkylmethoxy-, 2-Methoxy-ethoxy-, 2-(Cyclobutyloxy)-ethoxy-, 2-(Cyclopentyloxy)-ethoxy-, 2-(Cyclohexyloxy)-ethoxy-, 2-(Cyclopropylmethoxy)-ethoxy-, Tetrahydrofuran-3-yloxy-, Tetrahydropyran-4-yloxy-, Tetrahydrofuran-2-ylmethoxy-, Tetrahydrofuran-3-ylmethoxy-, Tetrahydropyran-2-ylmethoxy- oder Tetrahydropyran-4-ylmethoxygruppe,
A eine 1,2-Vinylengruppe,
B eine Methylen- oder Ethylengruppe oder, falls B an ein Kohlenstoffatom der Gruppe C gebunden ist, auch eine Bindung,
C eine Piperidinogruppe, in der die beiden Wasserstoffatome in 4-Stellung durch eine -CH₂-O-CO-CH₂-, -CH₂CH₂-O-CO-, -CH₂CH₂-O-CO-CH₂-, -O-CO-CH₂-NCH₃-CH₂- oder -O-CO-CH₂-O-CH₂-Brücke ersetzt sind,
eine Piperazinogruppe, in der ein Wasserstoffatom in 3-Stellung zusammen mit dem Wasserstoffatom in 4-Stellung durch eine -CO-O-CH₂-CH₂- oder -CH₂-O-CO-CH₂-Brücke ersetzt sind, wobei jeweils das linke Ende der vorstehenden Brücken an die 3-Stellung des Piperazinoringes gebunden ist,
eine durch eine 2-Oxo-tetrahydrofuranylgruppe substituierte Piperidinogruppe,
eine Piperidinogruppe, die in 4-Stellung durch eine 2-Oxo-morpholino- oder 2-Oxomorpholinomethylgruppe substituiert ist, wobei der 2-Oxo-morpholinoteil jeweils durch eine oder zwei Methylgruppen substituiert sein kann,
eine Piperazinogruppe, die in 4-Stellung durch eine 2-Oxo-tetrahydrofuran-3-yl- oder 2-Oxo-tetrahydrofuran-4-yl-Gruppe substituiert ist,
eine Piperidinogruppe, die in 4-Stellung durch eine CH₃NR₆- oder R₆S-Gruppe substituiert ist, wobei
R₆ eine 2-Oxo-tetrahydrofuran-3-yl- oder 2-Oxo-tetrahydrofuran-4-yl-Gruppe darstellt,
eine Piperazinogruppe, die in 4-Stellung durch eine 2-Oxo-tetrahydrofuranylmethyl- oder 2-Oxo-tetrahydrofuranylcarbonylgruppe substituiert ist,
eine Piperazinogruppe, die in 4-Stellung durch eine geradkettige C₂₋₃-Alkylgruppe substituiert ist, wobei der C₂₋₃-Alkylteil jeweils endständig durch eine 2-Oxo-tetrahydrofuran-3-ylsulfenylgruppe substituiert ist,
eine Piperidin-4-yl-Gruppe, die in 1-Stellung durch eine 2-Oxo-tetrahydrofuran-3-yl-oder 2-Oxo-tetrahydrofuran-4-yl-Gruppe substituiert ist, oder
eine Piperidin-4-yl-NCH₃-Gruppe, die am Ringstickstoffatom durch eine 2-Oxo-tetrahydrofuran-3-yl-, 2-Oxo-tetrahydrofuran-4-yl- oder 2-Oxo-tetrahydrofuranylcarbonyl-Gruppe substituiert ist, bedeuten,
deren Tautomeren, deren Stereoisomere und deren Salze.

4. Bicyclische Heterocyclen der allgemeinen Formel I gemäß Anspruch 1, in der
X ein Stickstoffatom,
Rₐ ein Wasserstoffatom,
R_{b} eine 1-Phenylethylgruppe oder eine durch die Reste R₁ bis R₃ substituierte Phenylgruppe, wobei
R₁ und R₂, die gleich oder verschieden sein können, jeweils eine Methylgruppe oder ein Wasserstoff-, Fluor-, Chlor- oder Bromatom und
R₃ ein Wasserstoffatom darstellen,
R_{c} ein Wasserstoffatom,
R_{d} ein Wasserstoffatom, eine Methoxy-, Ethoxy-, 2-Methoxy-ethoxy-, Cyclobutyloxy-, Cyclopentyloxy-, Cyclopropylmethoxy-, Cyclobutylmethoxy-, Tetrahydrofuran-3-yloxy-, Tetrahydropyran-4-yloxy- oder Tetrahydrofuran-2-ylmethoxygruppe,
A eine 1,2-Vinylengruppe,
B eine Methylengruppe oder, falls B an ein Kohlenstoffatom der Gruppe C gebunden ist, auch eine Bindung,
C eine Piperidinogruppe, in der die beiden Wasserstoffatome in 4-Stellung durch eine -CH₂-O-CO-CH₂-, -CH₂CH₂-O-CO-, -CH₂CH₂-O-CO-CH₂-, -O-CO-CH₂-NCH₃-CH₂- oder -O-CO-CH₂-O-CH₂-Brücke ersetzt sind,
eine Piperazinogruppe, in der ein Wasserstoffatom in 3-Stellung zusammen mit dem Wasserstoffatom in 4-Stellung durch eine -CO-O-CH₂-CH₂- oder -CH₂-O-CO-CH₂-Brücke ersetzt sind, wobei jeweils das linke Ende der vorstehenden Brücken an die 3-Stellung des Piperazinoringes gebunden ist,
eine durch eine 2-Oxo-tetrahydrofuranylgruppe substituierte Piperidinogruppe,
eine Piperidinogruppe, die in 4-Stellung durch eine 2-Oxo-morpholino- oder 2-Oxomorpholinomethylgruppe substituiert ist, wobei der 2-Oxo-morpholinoteil jeweils durch eine oder zwei Methylgruppen substituiert sein kann,
eine Piperazinogruppe, die in 4-Stellung durch eine 2-Oxo-tetrahydrofuran-3-yl- oder 2-Oxo-tetrahydrofuran-4-yl-Gruppe substituiert ist,
eine Piperidinogruppe, die in 4-Stellung durch eine CH₃NR₆- oder R₆S-Gruppe substituiert ist, wobei
R₆ eine 2-Oxo-tetrahydrofuran-3-yl- oder 2-Oxo-tetrahydrofuran-4-yl-Gruppe darstellt,
eine Piperazinogruppe, die in 4-Stellung durch eine 2-Oxo-tetrahydrofuranylmethyl- oder 2-Oxo-tetrahydrofuranylcarbonylgruppe substituiert ist,
eine Piperazinogruppe, die in 4-Stellung durch eine [2-(2-Oxo-tetrahydrofuran-3-ylsulfenyl)ethyl]gruppe substituiert ist,
eine Piperidin-4-yl-Gruppe, die in 1-Stellung durch eine 2-Oxo-tetrahydrofuran-3-yl- oder 2-Oxo-tetrahydrofuran-4-yl-Gruppe substituiert ist, oder
eine Piperidin-4-yl-NCH₃-Gruppe, die am Ringstickstoffatom durch eine 2-Oxo-tetrahydrofuran-3-yl-, 2-Oxo-tetrahydrofuran-4-yl- oder 2-Oxo-tetrahydrofuranylcarbonyl-Gruppe substituiert ist, bedeuten,
deren Tautomeren, deren Stereoisomere und deren Salze.

5. Folgende Verbindungen der allgemeinen Formel I gemäß Anspruch 1:
(a) 4-[(3-Chlor-4-fluor-phenyl)amino]-6-({4-[4-(2-oxo-tetrahydrofuran-3-yl)-piperazin-1-yl]-1-oxo-2-buten-1-yl)amino)-7-cydopropylmethoxy-chinazolin,
(b) 4-[(3-Chlor-4-fluor-phenyl)amino]-6-({4-[4-(2-oxo-tetrahydrofuran-4-yl)-piperazin-1-yl]-1-oxo-2-buten-1-yl}amino)-7-cyclopropylmethoxy-chinazolin,
(c) 4-[(3-Chlor-4-fluor-phenyl)amino]-6-{[4-(4-{2-[(2-oxo-tetrahydrofuran-3-yl)sulfanyl]-ethyl}-piperazin-1-yl)-1-oxo-2-buten-1-yl]aminol-7-cyclopropylmethoxy-chinazolin,
(d) 4-[(3-Chlor-4-fluor-phenyl)amino]-6-{[4-(3-oxo-perhydro-pyrazino-[2,1-c][1,4]oxazin-8-yl)-1-oxo-2-buten-1-yl]amino}-7-cyclopropylmethoxy-chinazolin,
(e) (S)-4-[(3-Chlor-4-fluor-phenyl)amino]-6-[(4-{4-[(5-oxo-tetrahydrofuran-2-yl)carbonyl]-piperazin-1-yl}-1-oxo-2-buten-1-yl)amino]-7-cyclopropylmethoxy-chinazolin,
(f) 4-[(3-Chlor-4-fluor-phenyl)amino]-6-{[4-(1-oxo-perhydro-pyrazino[2,1-c][1,4]oxazin-8-yl)-1-oxo-2-buten-1-yl]amino}-7-cydopropylmethoxy-chinazolin und
(g) 4-[(3-Chlor-4-fluor-phenyl)amino]-6-[(4-{4-[(2-oxo-tetrahydrofuran-3-yl)sulfanyl]-piperidin-1-yl}-1-oxo-2-buten-1-yl)amino]-7-cyclopropylmethoxy-chinazolin
sowie deren Salze.

6. Physiologisch verträgliche Salze der Verbindungen gemäß den Ansprüchen 1 bis 5 mit anorganischen oder organischen Säuren oder Basen.

7. Arzneimittel, enthaltend eine Verbindung gemäß den Ansprüchen 1 bis 5 oder ein physiologisch verträgliches Salz gemäß Anspruch 6 neben gegebenenfalls einem oder mehreren inerten Trägerstoffen und/oder Verdünnungsmitteln.

8. Verwendung einer Verbindung gemäß den Ansprüchen 1 bis 6 zur Herstellung eines Arzneimittels, das zur Behandlung von benignen oder malignen Tumoren, zur Vorbeugung und Behandlung von Erkrankungen der Atemwege und der Lunge, zur Behandlung von Polypen, von Erkrankungen des Magen-Darm-Traktes, der Gallengänge und -blase sowie der Niere und Haut geeignet ist.

9. Verfahren zur Herstellung eines Arzneimittels gemäß Anspruch 7, **dadurch gekennzeichnet, daß** auf nichtchemischem Wege eine Verbindung gemäß den Ansprüchen 1 bis 6 in einen oder mehrere inerte Trägerstoffe und/oder Verdünnungsmittel eingearbeitet wird.

10. Verfahren zur Herstellung der Verbindungen der allgemeinen Formel I gemäß den Ansprüchen 1 bis 6, **dadurch gekennzeichnet, daß**
a. eine Verbindung der allgemeinen Formel 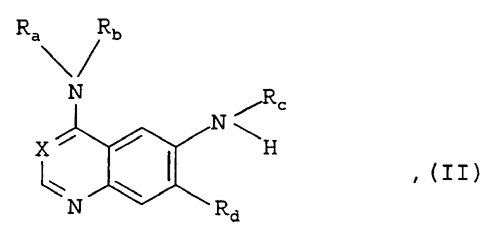 in der
Rₐ bis R_{d} und X wie in den Ansprüchen 1 bis 5 erwähnt definiert sind,
mit einer Verbindung der allgemeinen Formel
HO-CO - A - B - C, (III)
in der
A bis C wie in den Ansprüchen 1 bis 5 erwähnt definiert sind, oder mit deren reaktionsfähigen Derivaten umgesetzt wird
b. eine gegebenenfalls im Reaktionsgemisch gebildete Verbindung der allgemeinen Formel 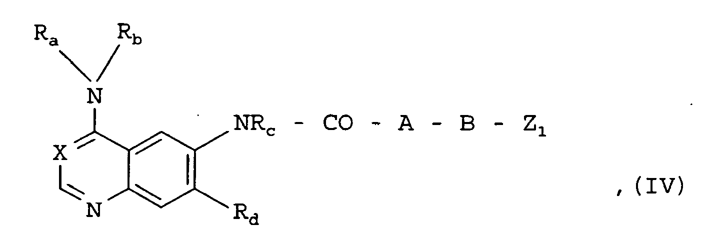 in der
Rₐ bis R_{d}, A, B und X wie in den Ansprüchen 1 bis 5 erwähnt definiert sind und
Z₁ eine austauschbare Gruppe bedeutet,
mit einer Verbindung der allgemeinen Formel
H - G, (V)
in der
G einen der für C in den Ansprüchen 1 bis 5 erwähnten Reste darstellt, der über ein Stickstoffatom mit dem Rest B verknüpft ist, umgesetzt wird oder
c. zur Herstellung einer Verbindung der allgemeinen Formel I, in der C eine der für C in den Ansprüchen 1 bis 5 erwähnten Gruppen darstellt, die eine durch R₆ oder durch eine R₅-C₁₋₄-alkyl-Gruppe substituierte lmino- oder HNR₄-Gruppe enthält, wobei R₄ bis R₆ wie in den Ansprüchen 1 bis 5 erwähnt definiert sind, eine Verbindung der allgemeinen Formel 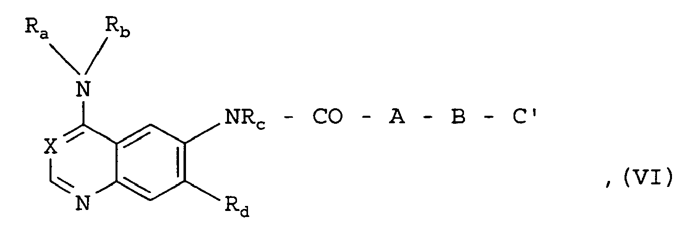 in der
Rₐ bis R_{d}, A und B wie in den Ansprüchen 1 bis 5 erwähnt definiert sind und
C' eine der für C in den Ansprüchen 1 bis 5 erwähnten Gruppen darstellt, die eine entsprechende unsubstituierte Imino- oder HNR₄-Gruppe enthält, wobei R₄ wie in den Ansprüchen 1 bis 5 erwähnt definiert ist, darstellt,
mit einer Verbindung der allgemeinen Formel
Z₂ - U, (VII)
in der
U den Rest R₆ oder eine R₅-C₁₋₄-alkyl-Gruppe bedeutet, wobei R₅ und R₆ wie in den Ansprüchen 1 bis 5 erwähnt definiert sind, und
Z₂ eine austauschbare Gruppe oder
Z₂ zusammen mit einem benachbarten Wasserstoffatom eine weitere Kohlenstoff-Kohlenstoffbindung, die mit einer Carbonylgruppe verbunden ist, bedeutet, umgesetzt wird oder
d. zur Herstellung einer Verbindung der allgemeinen Formel I, in der C eine der für C in den Ansprüchen 1 bis 5 erwähnten Gruppen darstellt, die eine durch eine R₅CO-, R₅-C₁₋₄-alkylen-CO-, (R₄NR₆)-C₁₋₄-alkylen-CO-, R₆O-C₁₋₄-alkylen-CO-, R₆S-C₁₋₄-alkylen-CO-, R₆SO-C₁₋₄-alkylen-CO-, R₆SO₂-C₁₋₄-alkylen-CO- oder 2-Oxo-morpholino-C₁₋₄-alkylen-CO-Gruppe substituierte Imino- oder HNR₁-Gruppe enthält, wobei R₄ bis R₆ wie in den Ansprüchen 1 bis 5 erwähnt definiert sind und der 2-Oxo-morpholinoteil durch eine oder zwei C₁₋₂-Alkylgruppen substituiert sein kann,
eine Verbindung der allgemeinen Formel 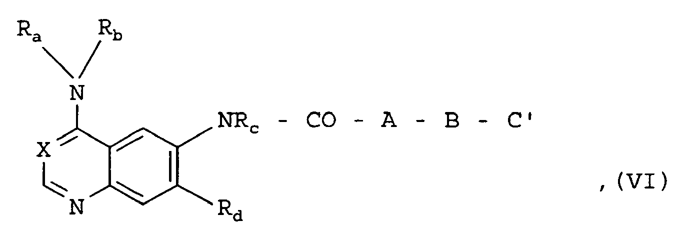 in der
Rₐ bis R_{d}, A und B wie in den Ansprüchen 1 bis 5 erwähnt definiert sind und C' eine der für C in den Ansprüchen 1 bis 5 erwähnten Gruppen darstellt, die eine entsprechende unsubstituierte Imino- oder HNR₄-Gruppe enthält, wobei R₄ wie in den Ansprüchen 1 bis 5 erwähnt definiert ist, darstellt,
mit einer Verbindung der allgemeinen Formel
HO-CO - W, (VIII)
in der
W den Rest R₅ oder eine R₅-C₁₋₄-alkyl-, (R₄NR₆)-C₁₋₄-alkyl-, R₆O-C₁₋₄-alkyl-, R₆S-C₁₋₄-alkyl-, R₆SO-C₁₋₄-alkyl-, R₆SO₂-C₁₋₄-alkyl- oder 2-Oxo-morpholino-C₁₋₄-alkyl-Gruppe darstellt, in denen R₄ bis R₆ wie in den Ansprüchen 1 bis 5 erwähnt definiert sind und der 2-Oxo-morpholinoteil durch eine oder zwei C₁₋₂-Alkylgruppen substituiert sein kann, umgesetzt wird und
erforderlichenfalls ein bei den vorstehend beschriebenen Umsetzungen verwendeter Schutzrest wieder abgespalten wird und/oder
gewünschtenfalls eine so erhaltene Verbindung der allgemeinen Formel I in ihre Stereoisomere aufgetrennt wird und/oder
eine so erhaltene Verbindung der allgemeinen Formel I in ihre Salze, insbesondere für die pharmazeutische Anwendung in ihre physiologisch verträglichen Salze übergeführt wird.

## Claims

1. Bicyclic heterocycles of general formula 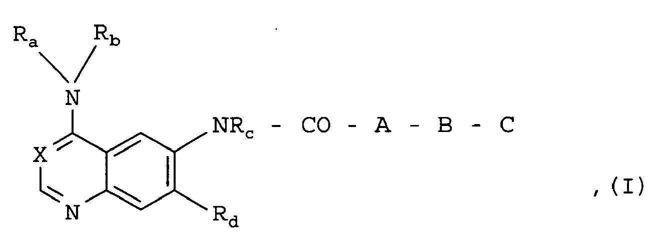 wherein
X denotes a methyne group substituted by a cyano group or a nitrogen atom,
Rₐ denotes a hydrogen atom or a C₁₋₄-alkyl group,
R_{b} denotes a phenyl, benzyl or 1-phenylethyl group wherein the phenyl nucleus in each case is substituted by the groups R₁ to R₃, whilst
R₁ and R₂, which may be identical or different, in each case denote a hydrogen, fluorine, chlorine, bromine or iodine atom,
a C₁₋₄-alkyl, hydroxy, C₁₋₄-alkoxy, C₃₋₆-cycloalkyl, C₄-₆-cycloalkoxy, C₂₋₅-alkenyl or C₂₋₅-alkynyl group,
an aryl, aryloxy, arylmethyl or arylmethoxy group,
a C₃₋₅-alkenyloxy or C₃₋₅-alkynyloxy group, whilst the unsaturated moiety may not be linked to the oxygen atom,
a C₁₋₄-alkylsulphenyl, C₁₋₄-alkylsulphinyl, C₁₋₄-alkylsulphonyl, C₁₋₄-alkylsulphonyloxy, trifluoromethylsulphenyl, trifluoromethylsulphinyl or trifluoromethylsulphonyl group,
a methyl or methoxy group substituted by 1 to 3 fluorine atoms,
an ethyl or ethoxy group substituted by 1 to 5 fluorine atoms,
a cyano or nitro group or an amino group optionally substituted by one or two C₁₋₄-alkyl groups, whilst the substituents may be identical or different, or
R₁ together with R₂, if they are bound to adjacent carbon atoms, denote a -CH=CH-CH=CH-, -CH=CH-NH- or -CH=N-NH-group and
R₃ denotes a hydrogen, fluorine, chlorine or bromine atom,
a C₁₋₄-alkyl, trifluoromethyl or C₁₋₄-alkoxy group,
R_{c} denotes a hydrogen atom or a C₁₋₄-alkyl group,
R_{d} denotes a hydrogen atom, a C₁₋₆-alkoxy, C₄₋₇-cycloalkoxy or C₃₋₇-cycloalkyl-C₁₋₄-alkoxy group,
a C₂₋₆-alkoxy group, which is substituted from position 2 by a hydroxy, C₁₋₄-alkoxy, C₄₋₇-cycloalkoxy, C₃₋₇-cycloalkyl-C₁₋₃-alkoxy, di-(C₁₋₄-alkyl)-amino, pyrrolidino, piperidino, morpholino, piperazino or 4-(C₁₋₄-alkyl)-piperazino group, whilst the abovementioned cyclic imino groups may be substituted by one or two C₁₋₂-alkyl groups,
a tetrahydrofuran-3-yloxy, tetrahydropyran-3-yloxy, tetrahydropyran-4-yloxy, tetrahydrofuranylmethoxy or tetrahydropyranylmethoxy group,
A denotes a 1,1- or 1,2-vinylene group optionally substituted by a methyl or trifluoromethyl group or by two methyl groups,
a 1,3-butadien-1,4-ylene group optionally substituted by a methyl or trifluoromethyl group or by two methyl groups, or an ethynylene group,
B denotes a C₁₋₆-alkylene group wherein one or two hydrogen atoms may be replaced by fluorine atoms, or, if B is bound to a carbon atom of group C, it may also denote a bond,
C denotes a pyrrolidino group wherein the two hydrogen atoms in the 2 position are replaced by a group D, wherein
D denotes a -CH₂-O-CO-CH₂-, -CH₂CH₂-O-CO-, -CH₂-O-CO-CH₂CH₂-, -CH₂CH₂-O-CO-CH₂- or -CH₂CH₂CH₂-O-CO- bridge optionally substituted by one or two C₁₋₂-alkyl groups,
a pyrrolidino group wherein the two hydrogen atoms in the 3 position are replaced by a group E, wherein
E denotes an -O-CO-CH₂CH₂-, -CH₂-O-CO-CH₂-, -CH₂CH₂-O-CO-, -O-CO-CH₂CH₂CH₂-, -CH₂-O-CO-CH₂CH₂-, -CH₂CH₂-O-CO-CH₂-, -CH₂CH₂CH₂-O-CO-, -O-CO-CH₂-NR₄-CH₂-, -CH₂-O-CO-CH₂-NR₄-, -O-CO-CH₂-O-CH₂- or -CH₂-O-CO-CH₂-O- bridge optionally substituted by one or two C₁₋₂-alkyl groups, whilst R₄ denotes a hydrogen atom or a C₁₋₄-alkyl group,
a piperidino or hexahydroazepino group wherein the two hydrogen atoms in the 2 position are replaced by a group D, whilst D is as hereinbefore defined,
a piperidino or hexahydroazepino group wherein the two hydrogen atoms in the 3 position or in the 4 position are replaced by a group E, whilst E is as hereinbefore defined,
a piperazino or 4-(C₁₋₄-alkyl)-piperazino group wherein the two hydrogen atoms in the 2 position or in the 3 position of the piperazino ring are replaced by a group D, where D is as hereinbefore defined,
a pyrrolidino or piperidino group wherein two vicinal hydrogen atoms are replaced by an -O-CO-CH₂-, -CH₂-O-CO-, -O-CO-CH₂CH₂-, -CH₂-O-CO-CH₂-, -CH₂CH₂-O-CO-, -O-CO-CH₂-NR₄- or -O-CO-CH₂-O-bridge optionally substituted by one or two C₁₋₂-alkyl groups, whilst R₄ is as hereinbefore defined and the heteroatoms of the abovementioned bridges are not bound at the 2 or 5 position of the pyrrolidine ring and are not bound at the 2 or 6 position of the piperidino ring,
a piperazino or 4-(C₁₋₄-alkyl)-piperazino group wherein a hydrogen atom in the 2 position together with a hydrogen atom in the 3 position of the piperazino ring are replaced by a -CH₂-O-CO-CH₂- or -CH₂CH₂-O-CO- bridge optionally substituted by one or two C₁₋₂-alkyl groups,
a piperazino group wherein a hydrogen atom in the 3 position together with the hydrogen atom in the 4 position are replaced by a -CO-O-CH₂CH₂- or -CH₂-O-CO-CH₂- bridge optionally substituted by one or two C₁₋₂-alkyl groups, whilst in each case the left-hand end of the abovementioned bridges is bound to the 3 position of the piperazino ring,
a pyrrolidino, piperidino or hexahydroazepino group substituted by the group R₅, wherein
R₅ denotes a 2-oxo-tetrahydrofuranyl, 2-oxo-tetrahydropyranyl, 2-oxo-1,4-dioxanyl or 2-oxo-4-(C₁₋₄-alkyl)-morpholinyl group optionally substituted by one or two C₁₋₂-alkyl groups,
a pyrrolidino group substituted in the 3 position by a 2-oxo-morpholino group, whilst the 2-oxo-morpholino group may be substituted by one or two C₁₋₂-alkyl groups,
a piperidino or hexahydroazepino group substituted in the 3 or 4 position by a 2-oxo-morpholino group, whilst the 2-oxo-morpholino group may be substituted by one or two C₁₋₂-alkyl groups,
a 4-(C₁₋₄-alkyl)-piperazino or 4-(C₁₋₄alkyl)-homopiperazino group substituted at a cyclic carbon atom by R₅ , wherein R₅ is as hereinbefore defined,
a piperazino or homopiperazino group substituted in the 4 position by the group R₆, wherein
R₆ denotes a 2-oxo-tetrahydrofuran-3-yl, 2-oxo-tetrahydrofuran-4-yl, 2-oxo-tetrahydropyran-3-yl, 2-oxo-tetrahydropyran-4-yl or 2-oxo-tetrahydropyran-5-yl group optionally substituted by one or two C₁₋₂-alkyl groups,
a pyrrolidino group substituted in the 3 position by a (R₄NR₆), R₆O, R₆S, R₆SO or R₆SO₂ group, whilst R₄ and R₆ are as hereinbefore defined,
a piperidino or hexahydroazepino group substituted in the 3 or 4 position by an (R₄NR₆), R₆O, R₆S, R₆SO or R₆SO₂ group wherein R₄ and R₆ are as hereinbefore defined,
a pyrrolidino, piperidino or hexahydroazepino group substituted by a R₅-C₁₋₄-alkyl, (R₄NR₆)-C₁₋₄-alkyl, R₆O-C₁₋₄-alkyl, R₆S-C₁₋₄-alkyl, R₆SO-C₁₋₄-alkyl, R₆SO₂-C₁₋₄-alkyl or R₄NR₆-CO group wherein R₄ to R₆ are as hereinbefore defined,
a pyrrolidino group substituted in the 3 position by a R₅-CO-NR₄, R₅-C₁₋₄-alkylene-CONR₄, (R₄NR₆) -C₁₋₄-alkylene-CONR₄, R₆O-C₁₋₄-alkylene-CONR₄, R₆S-C₁₋₄-alkylene-CONR₄, R₆SO-C₁₋₄-alkylene-CONR₄, R₆SO₂-C₁₋₄-alkylene-CONR₄, 2-oxo-morpholino-C₁₋₄-alkylene-CONR₄, R₅-C₁₋₄-alkylene-Y or C₂₋₄-alkyl-Y group, whilst the C₂₋₄-alkyl moiety of the C₂₋₄-alkyl-Y group is substituted in each case from position 2 by a (R₄NR₆), R₆O, R₆S, R₆SO or R₆SO₂ group and the 2-oxo-morpholino moiety may be substituted by one or two C₁₋₂-alkyl groups, wherein
R₄ to R₆ are as hereinbefore defined and
Y denotes an oxygen or sulphur atom, an imino, N-(C₁₋₄-alkyl)-imino, sulphinyl or sulphonyl group,
a piperidino or hexahydroazepino group substituted in the 3 or 4 position by a R₅-CO-NR₄, R₅-C₁₋₄-alkylene-CONR₄, (R₄NR₆) -C₁₋₄-alkylene-CONR₄, R₆O-C₁₋₄-alkylene-CONR₄, R₆S-C₁₋₄-alkylene-CONR₄, R₆SO-C₁₋₄-alkylene-CONR₄,
R₆SO₂-C₁₋₄-alkylene-CONR₄, 2-oxo-morpholino-C₁₋₄-alkylene-CONR₄, R₅-C₁₋₄-alkylene-Y or C₂₋₄-alkyl-Y group wherein Y is as hereinbefore defined, the 2-oxo-morpholino moiety may be substituted by one or two C₁₋₂-alkyl groups and the C₂₋₄-alkyl moiety of the C₂₋₄-alkyl-Y group is substituted in each case from position 2 by a (R₄NR₆), R₆O, R₆S, R₆SO or R₆SO₂ group, whilst R₄ to R₆ are as hereinbefore defined,
a 4-(C₁₋₄-alkyl)-piperazino or 4-(C₁₋₄-alkyl)-homopiperazino group substituted at a cyclic carbon atom by an R₅-C₁₋₄-alkyl, (R₄NR₆)-C₁₋₄-alkyl, R₆O-C₁₋₄-alkyl, R₆S-C₁₋₄-alkyl, R₆SO-C₁₋₄-alkyl, R₆SO₂-C₁₋₄-alkyl or R₄NR₆-CO group, wherein R₄ to R₆ are as hereinbefore defined,
a piperazino or homopiperazino group substituted in the 4 position by an R₅-C₁₋₄-alkyl, R₅-CO, R₅-C₁₋₄-alkylene-CO, (R₄NR₆)-C₁₋₄-alkylene-CO, R₆O-C₁₋₄-alkylene-CO, R₆S-C₁₋₄-alkylene-CO, R₆SO-C₁₋₄-alkylene-CO or R₆SO₂-C₁₋₄-alkylene-CO group wherein R₄ to R₆ are as hereinbefore defined,
a piperazino or homopiperazino group substituted in the 4 position by a C₂₋₄-alkyl group, wherein the C₂₋₄-alkyl group is substituted in each case from position 2 by an (R₄NR₆), R₆O, R₆S, R₆SO or R₆SO₂ group, whilst R₄ and R₆ are as hereinbefore defined,
a pyrrolidino, piperidino or hexahydroazepino group substituted by a 2-oxo-morpholino-C₁₋₄-alkyl group, wherein the 2-oxo-morpholino moiety may be substituted by one or two C₁₋₂-alkyl groups,
a pyrrolidino group substituted in the 3 position by a C₂₋₄-alkyl-Y group, wherein Y is as hereinbefore defined and the C₂₋₄-alkyl moiety of the C₂₋₄-alkyl-Y group is substituted in each case from position 2 by a 2-oxo-morpholino group optionally substituted by one or two C₁₋₂-alkyl groups,
a piperidino or hexahydroazepino group substituted in the 3 or 4 position by a C₂₋₄-alkyl-Y group wherein Y is as hereinbefore defined and the C₂₋₄-alkyl moiety of the C₂₋₄-alkyl-Y group is substituted in each case from position 2 by a 2-oxo-morpholino group optionally substituted by one or two C₁₋₂-alkyl groups,
a 4-(C₁₋₄-alkyl)-piperazino or 4-(C₁₋₄-alkyl)-homopiperazino group substituted at a cyclic carbon atom by a 2-oxo-morpholino-C₁₋₄-alkyl group, wherein the 2-oxo-morpholino moiety may be substituted by one or two C₁₋₂-alkyl groups,
a piperazino or homopiperazino group substituted in the 4 position by a 2-oxo-morpholino-C₁₋₄-alkylene-CO group, wherein the 2-oxo-morpholino moiety may be substituted by one or two C₁₋₂-alkyl groups,
a piperazino or homopiperazino group substituted in the 4 position by a C₂₋₄-alkyl group, wherein the C₂₋₄-alkyl moiety is substituted in each case from position 2 by a 2-oxo-morpholino group optionally substituted by one or two C₁₋₂-alkyl groups,
a pyrrolidinyl or piperidinyl group substituted in the 1 position by the group R₆, by an R₅-C₁₋₄-alkyl, R₅-CO, R₅-C₁₋₄-alkylene-CO, (R₄NR₆)-C₁₋₄-alkylene-CO, R₆O-C₁₋₄-alkylene-CO, R₆S-C₁₋₄-alkylene-CO, R₆SO-C₁₋₄-alkylene-CO, R₆SO₂-C₁₋₄-alkylene-CO or 2-oxo-morpholino-C₁₋₄-alkylene-CO group wherein R₄ to R₆ are as hereinbefore defined and the 2-oxo-morpholino moiety may be substituted by one or two C₁₋₂-alkyl groups,
a pyrrolidinyl or piperidinyl group substituted in the 1 position by a C₂₋₄-alkyl group wherein the C₂₋₄-alkyl moiety is substituted in each case from position 2 by an (R₄NR₆), R₆O, R₆S, R₆SO, R₆SO₂ or 2-oxo-morpholino group, whilst R₄ and R₆ are as hereinbefore defined and the 2-oxo-morpholino moiety may be substituted by one or two C₁₋₂-alkyl groups,
a pyrrolidin-3-yl-NR₄, piperidin-3-yl-NR₄ or piperidin-4-yl-NR₄ group substituted in each case at the cyclic nitrogen atom by the group R₆, by an R₅-C₁₋₄-alkyl, R₅-CO, R₅-C₁₋₄-alkylene-CO, (R₄NR₆)-C₁₋₄-alkylene-CO, R₆O-C₁₋₄-alkylene-CO, R₆S-C₁₋₄-alkylene-CO, R₆SO-C₁₋₄-alkylene-CO, R₆SO₂-C₁₋₄-alkylene-CO or 2-oxo-morpholino-C₁₋₄-alkylene-CO group, wherein R₄ to R₆ are as hereinbefore defined and the 2-oxo-morpholino moiety may be substituted by one or two C₁₋₂-alkyl groups,
a pyrrolidin-3-yl-NR₄, piperidin-3-yl-NR₄ or piperidin-4-yl-NR₄ group substituted in each case at the cyclic nitrogen atom by a C₂₋₄-alkyl group, wherein the C₂₋₄-alkyl moiety is substituted in each case from position 2 by an (R₄NR₆), R₆O, R₆S, R₆SO, R₆SO₂ or 2-oxo-morpholino group, whilst R₄ and R₆ are as hereinbefore defined and the 2-oxo-morpholino moiety may be substituted by one or two C₁₋₂-alkyl groups,
a R₅-C₁₋₄-alkylene-NR₄ group wherein R₄ and R₅ are as hereinbefore defined, or
a C₂₋₄-alkyl-NR₄-group, wherein the C₂₋₄-alkyl moiety is substituted in each case from position 2 by an (R₄NR₆), R₆O, R₆S, R₆SO, R₆SO₂ or 2-oxo-morpholino group, whilst R₄ and R₆ are as hereinbefore defined and the 2-oxo-morpholino moiety may be substituted by one or two C₁₋₂-alkyl groups, while by the abovementioned aryl moieties is meant a phenyl group which may in each case be mono- or disubstituted by R', while the substituents may be identical or different and
R' denotes a fluorine, chlorine, bromine or iodine atom, a C₁₋₂-alkyl, trifluoromethyl or C₁₋₂-alkoxy group or
two groups R', if they are bound to adjacent carbon atoms, together denote a C₃₋₄-alkylene, methylenedioxy or 1,3-butadien-1,4-ylene group.
the tautomers, the stereoisomers and the salts thereof.

2. Bicyclic heterocycles of general formula I according to claim 1, wherein
X denotes a nitrogen atom,
Rₐ denotes a hydrogen atom,
R_{b} denotes a phenyl, benzyl or 1-phenylethyl group wherein the phenyl nucleus is substituted in each case by the groups R₁ to R₃, whilst
R₁ and R₂, which may be identical or different, in each case denote a methyl group or a hydrogen, fluorine, chlorine or bromine atom and
R₃ denotes a hydrogen atom,
R_{c} denotes a hydrogen atom,
R_{d} denotes a hydrogen atom, a methoxy, ethoxy, C₄₋₆-cycloalkoxy, C₃₋₆-cycloalkylmethoxy, 2-methoxy-ethoxy, 2-(cyclobutyloxy)-ethoxy, 2-(cyclopentyloxy)-ethoxy, 2-(cyclohexyloxy)-ethoxy, 2-(cyclopropylmethoxy)-ethoxy, tetrahydrofuran-3-yloxy, tetrahydropyran-4-yloxy, tetrahydrofuran-2-ylmethoxy, tetrahydrofuran-3-ylmethoxy, tetrahydropyran-2-ylmethoxy or tetrahydropyran-4-ylmethoxy group,
A denotes a 1,2-vinylene group,
B denotes a methylene or ethylene group or, if B is bound to a carbon atom of group C, it may also denote a bond,
C denotes a pyrrolidino group wherein the two hydrogen atoms in the 3 position are replaced by a group E, wherein
E denotes a -O-CO-CH₂CH₂-, -CH₂-O-CO-CH₂-, -CH₂CH₂-O-CO-, -CH₂CH₂-O-CO-CH₂-, -O-CO-CH₂-NR₄-CH₂-, -CH₂-O-CO-CH₂-NR₄-, -O-CO-CH₂-O-CH₂- or -CH₂-O-CO-CH₂-O- bridge optionally substituted by one or two methyl groups,
where R₄ denotes a methyl or ethyl group,
a piperidino group wherein the two hydrogen atoms in the 3 position or in the 4 position are replaced by a group E, where E is as hereinbefore defined,
a pyrrolidino or piperidino group wherein two vicinal hydrogen atoms are replaced by an -O-CO-CH₂-, -CH₂-O-CO-, -O-CO-CH₂-NR₄- or -O-CO-CH₂-O- bridge optionally substituted by one or two methyl groups, whilst R₄ is as hereinbefore defined and the heteroatoms of the abovementioned bridges are not bound at the 2 or 5 position of the pyrrolidine ring and are not bound at the 2 or 6 position of the piperidino ring,
a piperazino group wherein a hydrogen atom in the 3 position together with the hydrogen atom in the 4 position are replaced by a -CO-O-CH₂CH₂- or -CH₂-O-CO-CH₂- bridge optionally substituted by one or two methyl groups, whilst in each case the left-hand end of the abovementioned bridges is bound to the 3 position of the piperazino ring,
a pyrrolidino or piperidino group substituted by the group R₅, wherein
R₅ denotes a 2-oxo-tetrahydrofuranyl, 2-oxo-1,4-dioxanyl or 2-oxo-4-(C₁₋₄-alkyl)-morpholinyl group optionally substituted by one or two methyl groups,
a pyrrolidino group substituted in the 3 position by a 2-oxo-morpholino group, whilst the 2-oxo-morpholino group may be substituted by one or two methyl groups,
a piperidino group substituted in the 3 or 4 position by a 2-oxo-morpholino group, whilst the 2-oxo-morpholino group may be substituted by one or two methyl groups,
a piperazino group substituted in the 4 position by the group R₆, wherein
R₆ denotes a 2-oxo-tetrahydrofuran-3-yl or 2-oxo-tetrahydro-furan-4-yl group optionally substituted by one or two methyl groups,
a pyrrolidino group substituted in the 3 position by an (R₄NR₆), R₆O, R₆S, R₆SO or R₆SO₂ group, whilst R₄ and R₆ are as hereinbefore defined,
a piperidino group substituted in the 3 or 4 position by an (R₄NR₆), R₆O, R₆S, R₆SO or R₆SO₂ group, whilst R₄ and R₆ are as hereinbefore defined,
a pyrrolidino or piperidino group substituted by an (R₄NR₆)-C₁₋₂-alkyl, HNR₆-CO or R₄NR₆-CO group, whilst R₄ and R₆ are as hereinbefore defined,
a pyrrolidino group substituted in the 3 position by an R₅-CO-NH or R₅-CO-NR₄ group, whilst R₄ and R₅ are as hereinbefore defined,
a piperidino group substituted in the 3 or 4 position by an R₅-CO-NH or R₅-CO-NR₄ group, whilst R₄ and R₅ are as hereinbefore defined,
a piperazino group substituted in the 4 position by an R₅-C₁₋₂-alkyl, R₅-CO, R₅-C₁₋₂-alkylene-CO, (R₄NR₆)-C₁₋₂-alkylene-CO, R₆O-C₁₋₂-alkylene-CO, R₆S-C₁₋₂-alkylene-CO, R₆SO-C₁₋₂-alkylene-CO or R₆SO₂-C₁₋₂-alkylene-CO group wherein R₄ to R₆ are as hereinbefore defined,
a piperazino group substituted in the 4 position by a C₂₋₃-alkyl group wherein the C₂₋₃-alkyl group is substituted in each case from position 2 by an (R₄NR₆), R₆O, R₆S, R₆SO or R₆SO₂ group, where R₄ and R₆ are as hereinbefore defined,
a pyrrolidino or piperidino group substituted by a 2-oxo-morpholino-C₁₋₂-alkyl group wherein the 2-oxo-morpholino moiety may be substituted by one or two methyl groups,
a piperazino group substituted in the 4 position by a 2-oxo-morpholino-C₁₋₂-alkylene-CO group wherein the 2-oxo-morpholino moiety may be substituted by one or two methyl groups,
a piperazino group substituted in the 4 position by a C₂₋₃-alkyl group wherein the C₂₋₃-alkyl moiety is substituted in each case from position 2 by a 2-oxo-morpholino group optionally substituted by one or two methyl groups,
a piperidinyl group substituted in the 1 position by the group R₆, by an R₅-C₁₋₂-alkyl, R₅-CO, R₅-C₁₋₂-alkylene-CO, (R₄NR₆) -C₁₋₂-alkylene-CO, R₆O-C₁₋₂-alkylene-CO, R₆S-C₁₋₂-alkylene-CO, R₆SO-C₁₋₂-alkylene-CO, R₆SO₂-C₁₋₂-alkylene-CO or 2-oxo-morpholino-C₁₋₂-alkylene-CO group, whilst R₄ to R₆ are as hereinbefore defined and the 2-oxo-morpholino moiety may be substituted by one or two methyl groups,
a piperidinyl group substituted in the 1 position by a C₂₋₃-alkyl group wherein the C₂₋₃-alkyl moiety is substituted in each case from position 2 by an (R₄NR₆), R₆O, R₆S, R₆SO, R₆SO₂ or 2-oxo-morpholino group, whilst R₄ and R₆ are as hereinbefore defined and the 2-oxo-morpholino moiety may be substituted by one or two methyl groups,
a pyrrolidin-3-yl-NR₄, piperidin-3-yl-NR₄ or piperidin-4-yl-NR₄ group substituted in each case at the cyclic nitrogen atom by the group R₆, by an R₅-C₁₋₂-alkyl, R₅-CO, R₅-C₁₋₂-alkylene-CO, (R₄NR₆)-C₁₋₂-alkylene-CO, R₆O-C₁₋₂-alkylene-CO, R₆S-C₁₋₂-alkylene-CO, R₆SO-C₁₋₂-alkylene-CO, R₆SO₂-C₁₋₂-alkylene-CO or 2-oxo-morpholino-C₁₋₂-alkylene-CO group, wherein R₄ to R₆ are as hereinbefore defined and the 2-oxo-morpholino moiety may be substituted by one or two methyl groups, or
a pyrrolidin-3-yl-NR₄, piperidin-3-yl-NR₄ or piperidin-4-yl-NR₄ group substituted in each case at the cyclic nitrogen atom by a C₂₋₃-alkyl group, wherein the C₂₋₃-alkyl moiety is substituted in each case from position 2 by an (R₄NR₆), R₆O, R₆S, R₆SO, R₆SO₂ or 2-oxo-morpholino group, whilst R₄ and R₆ are as hereinbefore defined and the 2-oxo-morpholino moiety may be substituted by one or two methyl groups,
the tautomers, the stereoisomers and the salts thereof.

3. Bicyclic heterocycles of general formula I according to claim 1, wherein
X denotes a nitrogen atom,
Rₐ denotes a hydrogen atom,
R_{b} denotes a phenyl, benzyl or 1-phenylethyl group wherein the phenyl nucleus is substituted in each case by the groups R₁ to R₃, whilst
R₁ and R₂, which may be identical or different, each denote a methyl group or a hydrogen, fluorine, chlorine or bromine atom and
R₃ denotes a hydrogen atom,
R_{c} denotes a hydrogen atom,
R_{d} denotes a hydrogen atom, a methoxy, ethoxy, C₄₋₆-cycloalkoxy, C₃₋₆-cycloalkylmethoxy, 2-methoxy-ethoxy, 2-(cyclobutyloxy)-ethoxy, 2-(cyclopentyloxy)-ethoxy, 2-(cyclohexyloxy)-ethoxy, 2-(cyclopropylmethoxy)-ethoxy, tetrahydrofuran-3-yloxy, tetrahydropyran-4-yloxy, tetrahydrofuran-2-ylmethoxy, tetrahydrofuran-3-ylmethoxy, tetrahydropyran-2-ylmethoxy or tetrahydropyran-4-ylmethoxy group,
A denotes a 1,2-vinylene group,
B denotes a methylene or ethylene group or, if B is bound to a carbon atom of group C, it may also denote a bond,
C denotes a piperidino group wherein the two hydrogen atoms in the 4 position are replaced by a -CH₂-O-CO-CH₂-, -CH₂CH₂-O-CO-, -CH₂CH₂-O-CO-CH₂-, -O-CO-CH₂-NCH₃-CH₂- or -O-CO-CH₂-O-CH₂- bridge,
a piperazino group wherein a hydrogen atom in the 3 position together with the hydrogen atom in the 4 position are replaced by a -CO-O-CH₂-CH₂- or -CH₂-O-CO-CH₂- bridge, whilst in each case the left-hand end of the above bridges is bound to the 3 position of the piperazino ring,
a piperidino group substituted by a 2-oxo-tetrahydrofuranyl group,
a piperidino group which is substituted in the 4 position by a 2-oxo-morpholino or 2-oxo-morpholinomethyl group, whilst the 2-oxo-morpholino moiety may in each case be substituted by one or two methyl groups,
a piperazino group which is substituted in the 4 position by a 2-oxo-tetrahydrofuran-3-yl or 2-oxo-tetrahydrofuran-4-yl group,
a piperidino group which is substituted in the 4 position by a CH₃NR₆ or R₆S group, whilst
R₆ denotes a 2-oxo-tetrahydrofuran-3-yl or 2-oxo-tetrahydro-furan-4-yl group,
a piperazino group which is substituted in the 4 position by a 2-oxo-tetrahydrofuranylmethyl or 2-oxo-tetrahydrofuranylcarbonyl group,
a piperazino group which is substituted in the 4 position by a straight-chained C₂₋₃-alkyl group, whilst the C₂₋₃-alkyl moiety is terminally substituted in each case by a 2-oxo-tetrahydrofuran-3-ylsulphenyl group,
a piperidin-4-yl group which is substituted in the 1 position by a 2-oxo-tetrahydrofuran-3-yl or 2-oxo-tetrahydrofuran-4-yl group, or
a piperidin-4-yl-NCH₃ group which is substituted at the cyclic nitrogen atom by a 2-oxo-tetrahydrofuran-3-yl, 2-oxo-tetrahydrofuran-4-yl or 2-oxo-tetrahydrofuranylcarbonyl group,
the tautomers, the stereoisomers and the salts thereof.

4. Bicyclic heterocycles of general formula I according to claim 1, wherein
X denotes a nitrogen atom,
Rₐ denotes a hydrogen atom,
R_{b} denotes a 1-phenylethyl group or a phenyl group substituted by the groups R₁ to R₃, wherein
R₁ and R₂, which may be identical or different, each denote a methyl group or a hydrogen, fluorine, chlorine or bromine atom and
R₃ denotes a hydrogen atom,
R_{c} denotes a hydrogen atom,
R_{d} denotes a hydrogen atom, a methoxy, ethoxy, 2-methoxy-ethoxy, cyclobutyloxy, cyclopentyloxy, cyclopropylmethoxy, cyclobutylmethoxy, tetrahydrofuran-3-yloxy, tetrahydropyran-4-yloxy or tetrahydrofuran-2-ylmethoxy group,
A denotes a 1,2-vinylene group,
B denotes a methylene group or, if B is bound to a carbon atom of group C, it may also denote a bond,
C denotes a piperidino group in which the two hydrogen atoms are replaced in the 4 position by a -CH₂-O-CO-CH₂-, -CH₂CH₂-O-CO-, -CH₂CH₂-O-CO-CH₂-, -O-CO-CH₂-NCH₃-CH₂- or -O-CO-CH₂-O-CH₂- bridge,
a piperazino group wherein a hydrogen atom in the 3 position together with the hydrogen atom in the 4 position are replaced by a -CO-O-CH₂-CH₂- or -CH₂-O-CO-CH₂- bridge, while in each case the left-hand end of the abovementioned bridges is bound to the 3 position of the piperazino ring,
a piperidino group substituted by a 2-oxo-tetrahydrofuranyl group,
a piperidino group which is substituted in the 4 position by a 2-oxo-morpholino or 2-oxo-morpholinomethyl group, while the 2-oxo-morpholino moiety may in each case be substituted by one or two methyl groups,
a piperazino group which is substituted in the 4 position by a 2-oxo-tetrahydrofuran-3-yl or 2-oxo-tetrahydrofuran-4-yl group,
a piperidino group which is substituted in the 4 position by a CH₃NR₆ or R₆S group, where
R₆ denotes a 2-oxo-tetrahydrofuran-3-yl or 2-oxo-tetrahydrofuran-4-yl group,
a piperazino group which is substituted in the 4 position by a 2-oxo-tetrahydrofuranylmethyl or 2-oxo-tetrahydro-furanylcarbonyl group,
a piperazino group which is substituted in the 4 position by a [2-(2-oxo-tetrahydrofuran-3-ylsulphenyl)ethyl] group,
a piperidin-4-yl group which is substituted in the 1 position by a 2-oxo-tetrahydrofuran-3-yl or 2-oxo-tetrahydrofuran-4-yl group, or
a piperidin-4-yl-NCH₃ group which is substituted at the cyclic nitrogen atom by a 2-oxo-tetrahydrofuran-3-yl, 2-oxo-tetrahydrofuran-4-yl or 2-oxo-tetrahydrofuranylcarbonyl group,
the tautomers, the stereoisomers and the salts thereof.

5. The following compounds of general formula I according to claim 1:
(a) 4-[(3-chloro-4-fluoro-phenyl)amino]-6-({4-[4-(2-oxo-tetrahydrofuran-3-yl)-piperazin-1-yl]-1-oxo-2-buten-1-yl}amino)-7-cyclopropylmethoxy-quinazoline,
(b) 4-[(3-chloro-4-fluoro-phenyl)amino]-6-({4-[4-(2-oxo-tetrahydrofuran-4-yl)-piperazin-1-yl]-1-oxo-2-buten-1-yl}amino)-7-cyclopropylmethoxy-quinazoline,
(c) 4-[(3-chloro-4-fluoro-phenyl)amino]-6-{[4-(4-{2-[(2-oxo-tetrahydrofuran-3-yl)sulphanyl]-ethyl}-piperazin-1-yl)-1-oxo-2-buten-1-yl]amino}-7-cyclopropylmethoxy-quinazoline,
(d) 4-[(3-chloro-4-fluoro-phenyl)amino]-6-{[4-(3-oxo-perhydropyrazino[2,1-c][1,4]oxazin-8-yl)-1-oxo-2-buten-1-yl]amino}-7-cyclopropylmethoxy-quinazoline,
(e) (S)-4-[(3-chloro-4-fluoro-phenyl)amino]-6-[(4-{4-[(5-oxo-tetrahydrofuran-2-yl)carbonyl]-piperazin-1-yl}-1-oxo-2-buten-1-yl)amino]-7-cyclopropylmethoxy-quinazoline,
(f) 4-[(3-chloro-4-fluoro-phenyl)amino]-6-{[4-(1-oxo-perhydropyrazino[2,1-c][1,4]oxazin-8-yl)-1-oxo-2-buten-1-yl]amino}-7-cyclopropylmethoxy-quinazoline and
(g) 4-[(3-chloro-4-fluoro-phenyl)amino]-6-[(4-{4-[(2-oxo-tetrahydrofuran-3-yl)sulphanyl]-piperidin-1-yl}-1-oxo-2-buten-1-yl)amino]-7-cyclopropylmethoxy-quinazoline
and the salts thereof.

6. Physiologically acceptable salts of the compounds according to claims 1 to 5 with inorganic or organic acids or bases.

7. Pharmaceutical compositions containing a compound according to claims 1 to 5 or a physiologically acceptable salt according to claim 6 optionally together with one or more inert carriers and/or diluents.

8. Use of a compound according to claims 1 to 6 for preparing a pharmaceutical composition which is suitable for the treatment of benign or malignant tumours, for preventing and treating diseases of the respiratory tract and lungs, for treating polyps, diseases of the gastro-intestinal tract, bile duct and gall bladder as well as the kidneys and skin.

9. Process for preparing a pharmaceutical composition according to claim 7, **characterised in that** a compound according to claims 1 to 6 is incorporated in one or more inert carriers and/or diluents by a non-chemical method.

10. Process for preparing the compounds of general formula I according to claims 1 to 6, **characterised in that**
a. a compound of general formula 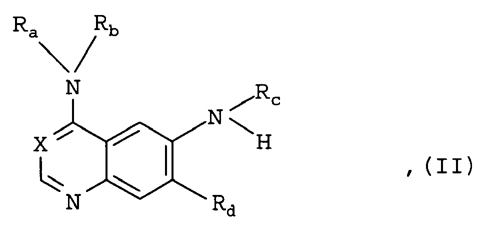 wherein
Rₐ to R_{d} and X are defined as in claims 1 to 5,
is reacted with a compound of general formula
HO-CO - A - B - C, (III)
wherein
A to C are defined as in claims 1 to 5, or with the reactive derivatives thereof
b. a compound of general formula 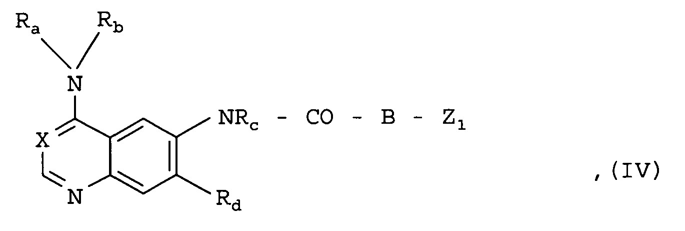 optionally formed in the reaction mixture
wherein
Rₐ to R_{d}, A, B and X are defined as in claims 1 to 5 and Z₁ denotes an exchangeable group,
is reacted with a compound of general formula
H - G, (V)
wherein
G denotes one of the groups mentioned for C in claims 1 to 5, which is attached to the group B via a nitrogen atom, or
c. in order to prepare a compound of general formula I wherein C denotes one of the groups mentioned for C in claims 1 to 5 which contains an imino or HNR₄ group substituted by R₆ or by an R₅-C₁₋₄-alkyl group, where R₄ to R₆ are defined as in claims 1 to 5, a compound of general formula 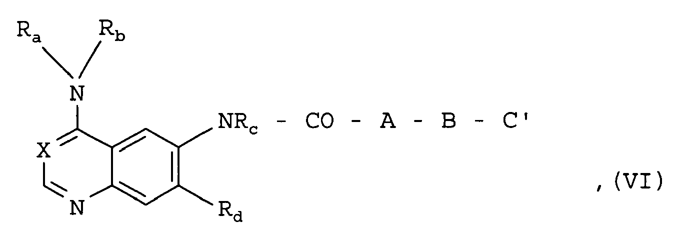 wherein
Rₐ to R_{d}, A and B are defined as in claims 1 to 5 and
C' denotes one of the groups mentioned for C in claims 1 to 5 which contains a corresponding unsubstituted imino or HNR₄ group, where R₄ is defined as in claims 1 to 5,
is reacted with a compound of general formula
Z₂ - U, (VII)
wherein
U denotes the group R₆ or a R₅-C₁₋₄-alkyl group, where R₅ and R₆ are defined as in claims 1 to 5, and
Z₂ denotes an exchangeable group or
Z₂ together with an adjacent hydrogen atom denotes another carbon-carbon bond attached to a carbonyl group, or
d. in order to prepare a compound of general formula I wherein C denotes one of the groups mentioned for C in claims 1 to 5 which contains an imino or HNR₁ group substituted by an R₅CO, R₅-C₁₋₄-alkylene-CO, (R₄NR₆) -C₁₋₄-alkylene-CO, R₆O-C₁₋₄-alkylene-CO, R₆S-C₁₋₄-alkylene-CO, R₆SO-C₁₋₄-alkylene-CO, R₆SO₂-C₁₋₄-alkylene-CO or 2-oxo-morpholino-C₁₋₄-alkylene-CO group, where R₄ to R₆ are defined as in claims 1 to 5 and the 2-oxo-morpholino moiety may be substituted by one or two C₁₋₂-alkyl groups,
a compound of general formula 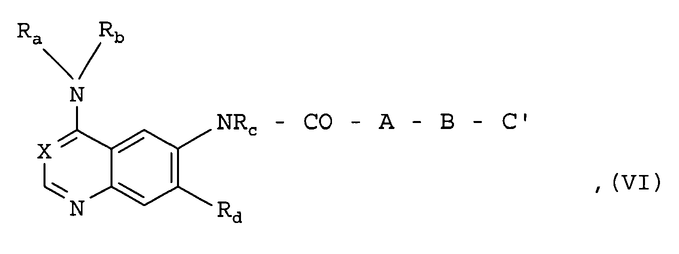 wherein
Rₐ to R_{d}, A and B are defined as in claims 1 to 5 and C' denotes one of the groups mentioned for C in claims 1 to 5 which contains a corresponding unsubstituted imino or HNR₄ group, where R₄ is defined as in claims 1 to 5,
is reacted with a compound of general formula
HO-CO - W, (VIII)
wherein
W denotes the group R₅ or an R₅-C₁₋₄-alkyl, (R₄NR₆)-C₁₋₄-alkyl, R₆O-C₁₋₄-alkyl, R₆S-C₁₋₄-alkyl, R₆SO-C₁₋₄-alkyl, R₆SO₂-C₁₋₄-alkyl or 2-oxo-morpholino-C₁₋₄-alkyl group, wherein R₄ to R₆ are defined as in claims 1 to 5 and the 2-oxo-morpholino moiety may be substituted by one or two C₁₋₂-alkyl groups, and
if necessary any protecting group used in the reactions described above is cleaved again and/or
if desired a compound of general formula I thus obtained is resolved into its stereoisomers and/or
a compound of general formula I thus obtained is converted into the salts thereof, particularly, for pharmaceutical use, into the physiologically acceptable salts thereof.

## Revendications

1. Hétérocycles bicycliques de formule générale : 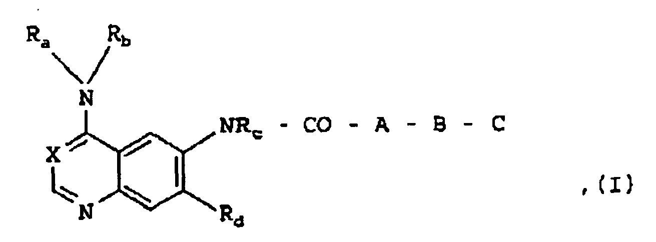 dans laquelle
X représente un groupe méthine substitué par un groupe cyano, ou un atome d'azote,
Rₐ représente un atome d'hydrogène ou un groupe alkyle en C₁ à C₄,
R_{b} représente un groupe phényle, benzyle ou 1-phényléthyle, dans lesquels le noyau phényle est substitué respectivement par les radicaux R₁ à R₃, sachant que
R₁ et R₂, qui peuvent être identiques ou différents, représentent respectivement un atome d'hydrogène, de fluor, de chlore, de brome ou d'iode,
un groupe alkyle en C₁ à C₄, hydroxy, alcoxy en C₁ à C₄, cycloalkyle en C₃ à C₆, cycloalcoxy en C₄ à C₆, alcényle en C₂ à C₅ ou alcynyle en C₂ à C₅,
un groupe aryle, aryloxy, arylméthyle ou arylméthoxy,
un groupe alcényloxy en C₃ à C₅ ou alcynyloxy en C₃ à C₅, sachant que la partie insaturée peut ne pas être reliée à l'atome d'oxygène,
un groupe alkylsulfényle en C₁ à C₄, alkylsulfinyle en C₁ à C₄, alkylsulfonyle en C₁ à C₄, alkylsulfonyloxy en C₁ à C₄, trifluorométhylsulfényle, trifluorométhylsulfinyle ou trifluorométhylsulfonyle,
un groupe méthyle ou méthoxy substitué par 1 à 3 atomes de fluor,
un groupe éthyle ou éthoxy substitué par 1 à 5 atomes de fluor,
un groupe cyano ou nitro, ou un groupe amino éventuellement substitué par un ou deux groupes alkyle en C₁ à C₄, sachant que les substituants peuvent être identiques ou différents, ou
R₁ et R₂, si ceux-ci sont reliés à des atomes de carbone voisins, représentent conjointement un groupe -CH=CH-CH=CH-, -CH=CH-NH- ou -CH=N-NH-, et
R₃ représente un atome d'hydrogène, de fluor, de chlore ou de brome,
un groupe alkyle en C₁ à C₄, trifluorométhyle ou alcoxy en C₁ à C₄,
R_{c} représente un atome d'hydrogène ou un groupe alkyle en C₁ à C₄,
R_{d} représente un atome d'hydrogène, un groupe alcoxy en C₁ à C₆, cycloalcoxy en C₄ à C₇ ou (cycloalkyle en C₃ à C₇) - (alcoxy en C₁ à C₄),
un groupe alcoxy en C₂ à C₆, qui est substitué à partir de la position 2 par un groupe hydroxy, alcoxy en C₁ à C₄, cycloalcoxy en C₄ à C₇, (cycloalkyle en C₃ à C₇) - (alcoxy en C₁ à C₃), di-(alkyle en C₁ à C₄)-amino, pyrrolidino, pipéridino, morpholino, pipérazino ou 4-(alkyle en C₁ à C₄)-pipérazino, sachant que les groupes imino cycliques mentionnés précédemment peuvent être substitués par un ou deux groupes alkyle en C₁ à C₂,
un groupe tétrahydrofuran-3-yloxy, tétrahydropyran-3-yloxy, tétrahydropyran-4-yloxy, tétrahydrofurannylméthoxy ou tétrahydropyrannylméthoxy,
A représente un groupe 1,1- ou 1,2-vinylène éventuellement substitué par un groupe méthyle ou trifluorométhyle ou par deux groupes méthyle,
un groupe 1,3-butadièn-1,4-ylène éventuellement substitué par un groupe méthyle ou trifluorométhyle ou par deux groupes méthyle, ou un groupe éthinylène,
B représente un groupe alkylène en C₁ à C₆, dans lequel un ou deux atomes d'hydrogène peuvent être remplacés par des atomes de fluor, ou bien, dans le cas où B est relié à un atome de carbone du groupe C, encore une liaison,
C représente un groupe pyrrolidino, dans lequel les deux atomes d'hydrogène en position 2 sont remplacés par un groupe D, dans lequel
D représente un pont -CH₂-O-CO-CH₂-, -CH₂CH₂-O-CO-, -CH₂-O-CO-CH₂CH₂-, -CH₂CH₂-O-CO-C H₂- ou -CH₂CH₂CH₂-O-CO- éventuellement substitué par un ou deux groupes alkyle en C₁ à C₂,
un groupe pyrrolidino, dans lequel les deux atomes d'hydrogène en position 3 sont remplacés par un groupe E, dans lequel
E représente un pont -O-CO-CH₂CH₂-, -CH₂-O-CO-CH₂-, -CH₂CH₂-O-CO-, -O-CO-CH₂CH₂CH₂-, -CH₂-O-CO-CH₂CH₂-, -CH₂CH₂-O-CO-CH₂-, -CH₂CH₂CH₂-O-CO-, -O-CO-CH₂-NR₄-CH₂-, -CH₂-O-CO-CH₂-NR₄-, -O-CO-CH₂-O-CH₂- ou -CH₂-O-CO-CH₂-O- éventuellement substitué par un ou deux groupes alkyle en C₁ à C₂,
sachant que R₄ représente un atome d'hydrogène ou un groupe alkyle en C₁ à C₄,
un groupe pipéridino ou hexahydroazépino, dans lesquels les deux atomes d'hydrogène en position 2 sont remplacés par un groupe D, sachant que D est défini comme mentionné précédemment,
un groupe pipéridino ou hexahydroazépino, dans lesquels les deux atomes d'hydrogène en position 3 ou en position 4 sont remplacés respectivement par un groupe E, sachant que E est défini comme mentionné précédemment,
un groupe pipérazino ou 4-(alkyle en C₁ à C₄)-pipérazino, dans lesquels les deux atomes d'hydrogène en position 2 ou en position 3 du cycle pipérazine sont remplacés par un groupe D, sachant que D est défini comme mentionné précédemment,
un groupe pyrrolidino ou pipéridino, dans lesquels deux atomes d'hydrogène vicinaux sont remplacés par un pont -O-CO-CH₂-, -CH₂-O-CO-, -O-CO-CH₂CH₂-, -CH₂-O-CO-CH₂-, -CH₂CH₂-O-CO-, -O-CO-CH₂-NR₄- ou -O-CO-CH₂-O- éventuellement substitué par un ou deux groupes alkyle en C₁ à C₂, sachant que R₄ est défini comme mentionné précédemment, et que les hétéroatomes des ponts mentionnés précédemment ne sont reliés ni à la position 2 ou 5 du cycle pyrrolidine, ni à la position 2 ou 6 du cycle pipéridine,
un groupe pipérazino ou 4-(alkyle en C₁ à C₄)-pipérazino, dans lesquels un atome d'hydrogène en position 2, conjointement avec un atome d'hydrogène en position 3 du cycle pipérazine, sont remplacés par un pont -CH₂-O-CO-CH₂- ou -CH₂CH₂-O-CO- éventuellement substitué par un ou deux groupes alkyle en C₁ à C₂,
un groupe pipérazino, dans lequel un atome d'hydrogène en position 3, conjointement avec l'atome d'hydrogène en position 4, sont remplacés par un pont -CO-O-CH₂CH₂- ou -CH₂-O-CO-CH₂- éventuellement substitué par un ou deux groupes alkyle en C₁ à C₂, sachant que l'extrémité gauche respective des ponts mentionnés précédemment est reliée à la position 3 du cycle pipérazine,
un groupe pyrrolidino, pipéridino ou hexahydroazépino substitué par le radical R₅, dans lesquels
R₅ représente un groupe 2-oxo-tétrahydrofurannyle, 2-oxo-tétrahydropyrannyle, 2-oxo-1,4-dioxannyle ou 2-oxo-4-(alkyle en C₁ à C₄)-morpholinyle éventuellement substitué par un ou deux groupes alkyle en C₁ à C₂,
un groupe pyrrolidino substitué en position 3 par un groupe 2-oxo-morpholino, sachant que le groupe 2-oxo-morpholino peut être substitué par un ou deux groupes alkyle en C₁ à C₂,
un groupe pipéridino ou hexahydroazépino substitué en position 3 ou 4 par un groupe 2-oxo-morpholino, sachant que le groupe 2-oxo-morpholino peut être substitué par un ou deux groupes alkyle en C₁ à C₂,
un groupe 4-(alkyle en C₁ à C₄)-pipérazino ou 4-(alkyle en C₁ à C₄)-homopipérazino substitué au niveau d'un atome de carbone du cycle par R₅, dans lesquels R₅ est défini comme mentionné précédemment,
un groupe pipérazino ou homopipérazino substitué en position 4 par le radical R₆, dans lesquels
R₆ représente un groupe 2-oxo-tétrahydrofuran-3-yle, 2-oxo-tétrahydrofuran-4-yle, 2-oxo-tétrahydropyran-3-yle, 2-oxo-tétrahydropyran-4-yle ou 2-oxo-tétrahydropyran-5-yle éventuellement substitué par un ou deux groupes alkyle en C₁ à C₂,
un groupe pyrrolidino substitué en position 3 par un groupe (R₄NR₆)-, R₆O-, R₆S-, R₆SO- ou R₆SO₂-, sachant que R₄ et R₆ sont définis comme mentionné précédemment,
un groupe pipéridino ou hexahydroazépino substitué en position 3 ou 4 par un groupe (R₄NR₆)-, R₆O-, R₆S-, R₆SO- ou R₆SO₂-, dans lesquels R₄ et R₆ sont définis comme mentionné précédemment,
un groupe pyrrolidino, pipéridino ou hexahydroazépino substitué par un groupe R₅-(alkyle en C₁ à C₄), (R₄NR₆)-(alkyle en C₁ à C₄), R₆O-(alkyle en C₁ à C₄), R₆S-(alkyle en C₁ à C₄), R₆SO-(alkyle en C₁ à C₄), R₆SO₂-(alkyle en C₁ à C₄) ou R₄NR₆-CO-, dans lesquels R₄ à R₆ sont définis comme mentionné précédemment,
un groupe pyrrolidino substitué en position 3 par un groupe R₅-CO-NR₄-, R₅-(alkylène en C₁ à C₄)-CONR₄-, (R₄NR₆)-(alkylène en C₁ à C₄)-CONR₄-, R₆O-(alkylène en C₁ à C₄)-CONR₄-, R₆S-(alkylène en C₁ à C₄)-CONR₄-, R₆SO-(alkylène en C₁ à C₄)-CONR₄-, R₆SO₂-(alkylène en C₁ à C₄)-CONR₄-, 2-oxo-morpholino-(alkylène en C₁ à C₄)-CONR₄-, R₅-(alkylène en C₁ à C₄)-Y- ou (alkyle en C₂ à C₄)-Y-, sachant que la partie alkyle en C₂ à C₄ du groupe (alkyle en C₂ à C₄)-Y- est substituée en position 2 respectivement par un groupe (R₄NR₆)-, R₆O-, R₆S-, R₆SO- ou R₆SO₂-, et que la partie 2-oxo-morpholino peut être substituée par un ou deux groupes alkyle en C₁ à C₂, dans lesquels
R₄ à R₆ sont définis comme mentionné précédemment et
Y représente un atome d'oxygène ou de soufre, un groupe imino, N-(alkyle en C₁ à C₄)-imino, sulfinyle ou sulfonyle,
un groupe pipéridino ou hexahydroazépino substitué en position 3 ou 4 par un groupe R₅-CO-NR₄-, R₅-(alkylène en C₁ à C₄)-CONR₄-, (R₄NR₆)-(alkylène en C₁ à C₄)-CONR₄-, R₆O-(alkylène en C₁ à C₄)-CONR₄-, R₆S-(alkylène en C₁ à C₄)-CONR₄-, R₆SO-(alkylène en C₁ à C₄)-CONR₄-, R₆SO₂-(alkylène en C₁ à C₄)-CONR₄-, 2-oxo-morpholino-(alkylène en C₁ à C₄)-CONR₄-, R₅-(alkylène en C₁ à C₄)-Y- ou (alkyle en C₂ à C₄)-Y-, dans lesquels Y est défini comme mentionné précédemment, la partie 2-oxo-morpholino peut être substituée par un ou deux groupes alkyle en C₁ à C₂, et la partie alkyle en C₂ à C₄ du groupe (alkyle en C₂ à C₄)-Y- est substituée à partir de la position 2 respectivement par un groupe (R₄NR₆)-, R₆O-, R₆S-, R₆SO- ou R₆SO₂-, sachant que R₄ à R₆ sont définis comme mentionné précédemment,
un groupe 4-(alkyle en C₁ à C₄)-pipérazino ou 4-(alkyle en C₁ à C₄)-homopipérazino substitué au niveau d'un atome de carbone du cycle par un groupe R₅-(alkyle en C₁ à C₄), (R₄NR₆)-(alkyle en C₁ à C₄), R₆O-(alkyle en C₁ à C₄), R₆S-(alkyle en C₁ à C₄), R₆SO-(alkyle en C₁ à C₄), R₆SO₂-(alkyle en C₁ à C₄) ou R₄NR₆-CO-, dans lesquels R₄ à R₆ sont définis comme mentionné précédemment,
un groupe pipérazino ou homopipérazino substitué en position 4 par un groupe R₅-(alkyle en C₁ à C₄), R₅-CO-, R₅-(alkylène en C₁ à C₄)-CO-, (R₄NR₆)-(alkylène en C₁ à C₄)-CO-, R₆O-(alkylène en C₁ à C₄)-CO-, R₆S-(alkylène en C₁ à C₄)-CO-, R₆SO-(alkylène en C₁ à C₄)-CO- ou R₆SO₂-(alkylène en C₁ à C₄)-CO-, dans lesquels R₄ à R₆ sont définis comme mentionné précédemment,
un groupe pipérazino ou homopipérazino substitué en position 4 par un groupe alkyle en C₂ à C₄, dans lesquels le groupe alkyle en C₂ à C₄ est substitué à partir de la position 2 respectivement par un groupe (R₄NR₆)-, R₆O-, R₆S-, R₆SO- ou R₆SO₂-, sachant que R₄ et R₆ sont définis comme mentionné précédemment,
un groupe pyrrolidino, pipéridino ou hexahydroazépino substitué par un groupe 2-oxo-morpholino-(alkyle en C₁ à C₄), dans lesquels la partie 2-oxo-morpholino peut être substituée par un ou deux groupes alkyle en C₁ à C₂,
un groupe pyrrolidino substitué en position 3 par un groupe (alkyle en C₂ à C₄)-Y-, dans lequel Y est défini comme mentionné précédemment, et la partie alkyle en C₂ à C₄ du groupe (alkyle en C₂ à C₄)-Y- est substituée à partir de la position 2 respectivement par un groupe 2-oxo-morpholino éventuellement substitué par un ou deux groupes alkyle en C₁ à C₂,
un groupe pipéridino ou hexahydroazépino substitué en position 3 ou 4 par un groupe (alkyle en C₂ à C₄)-Y-, dans lequel Y est défini comme mentionné précédemment, et la partie alkyle en C₂ à C₄ du groupe (alkyle en C₂ à C₄)-Y- est substituée à partir de la position 2 respectivement par un groupe 2-oxo-morpholino éventuellement substitué par un ou deux groupes alkyle en C₁ à C₂,
un groupe 4-(alkyle en C₁ à C₄)-pipérazino ou 4-(alkyle en C₁ à C₄)-homopipérazino substitué au niveau d'un atome de carbone du cycle par un groupe 2-oxo-morpholino-(alkyle en C₁ à C₄), dans lequel la partie 2-oxo-morpholino peut être substituée par un ou deux groupes alkyle en C₁ à C₂,
un groupe pipérazino ou homopipérazino substitué en position 4 par un groupe 2-oxo-morpholino-(alkylène en C₁ à C₄)-CO-, dans lequel la partie 2-oxo-morpholino peut être substituée par un ou deux groupes alkyle en C₁ à C₂,
un groupe pipérazino ou homopipérazino substitué en position 4 par un groupe alkyle en C₂ à C₄, dans lequel la partie alkyle en C₂ à C₄ est substituée à partir de la position 2 respectivement par un groupe 2-oxo-morpholino éventuellement substitué par un ou deux groupes alkyle en C₁ à C₂,
un groupe pyrrolidinyle ou pipéridinyle substitué en position 1 par le radical R₆, par un groupe R₅-(alkyle en C₁ à C₄), R₅-CO-, R₅-(alkylène en C₁ à C₄)-CO-, (R₄NR₆)-(alkylène en C₁ à C₄)-CO-, R₆O-(alkylène en C₁ à C₄)-CO-, R₆S-(alkylène en C₁ à C₄)-CO-, R₆SO-(alkylène en C₁ à C₄)-CO-, R₆SO₂-(alkylène en C₁ à C₄)-CO-, ou 2-oxo-morpholino-(alkylène en C₁ à C₄)-CO-, dans lesquels R₄ à R₆ sont définis comme mentionné précédemment, et la partie 2-oxo-morpholino peut être substituée par un ou deux groupes alkyle en C₁ à C₂,
un groupe pyrrolidinyle ou pipéridinyle substitué en position 1 par un groupe alkyle en C₂ à C₄, dans lequel la partie alkyle en C₂ à C₄ est substituée à partir de la position 2 respectivement par un groupe (R₄NR₆)-, R₆O-, R₆S-, R₆SO-, R₆SO₂- ou 2-oxo-morpholino, sachant que R₄ et R₆ sont définis comme mentionné précédemment, et que la partie 2-oxo-morpholino peut être substituée par un ou deux groupes alkyle en C₁ à C₂,
un groupe pyrrolidin-3-yl-NR₄-, pipéridin-3-yl-NR₄- ou pipéridin-4-yl-NR₄- substitué au niveau de l'atome d'azote du cycle respectivement par le radical R₆, par un groupe R₅-(alkyle en C₁ à C₄), R₅-CO-, R₅-(alkylène en C₁ à C₄)-CO-, (R₄NR₆)-(alkylène en C₁ à C₄)-CO-, R₆O-(alkylène en C₁ à C₄)-CO-, R₆S-(alkylène en C₁ à C₄)-CO-, R₆SO-(alkylène en C₁ à C₄)-CO-, R₆SO₂-(alkylène en C₁ à C₄)-CO-, ou 2-oxo-morpholino-(alkylène en C₁ à C₄)-CO-, dans lesquels R₄ à R₆ sont définis comme mentionné précédemment, et la partie 2-oxo-morpholino peut être substituée par un ou deux groupes alkyle en C₁ à C₂,
un groupe pyrrolidin-3-yl-NR₄-, pipéridin-3-yl-NR₄- ou pipéridin-4-yl-NR₄- substitué au niveau de l'atome d'azote du cycle respectivement par un groupe alkyle en C₂ à C₄, dans lequel la partie alkyle en C₂ à C₄ est substituée à partir de la position 2 respectivement par un groupe (R₄NR₆)-, R₆O-, R₆S-, R₆SO-, R₆SO₂- ou 2-oxo-morpholino, sachant que R₄ et R₆ sont définis comme mentionné précédemment, et que la partie 2-oxo-morpholino peut être substituée par un ou deux groupes alkyle en C₁ à C₂,
un groupe R₅-(alkylène en C₁ à C₄)-NR₄-, dans lequel R₄ et R₅ sont définis comme mentionné précédemment, ou bien
un groupe (alkyle en C₂ à C₄)-NR₄-, dans lequel la partie alkyle en C₂ à C₄ est substituée à partir de la position 2 respectivement par un groupe (R₄NR₆)-, R₆O-, R₆S-, R₆SO-, R₆SO₂- ou 2-oxo-morpholino, sachant que R₄ et R₆ sont définis comme mentionné précédemment, et que la partie 2-oxo-morpholino peut être substituée par un ou deux groupes alkyle en C₁ à C₂,
sachant qu'on doit comprendre les parties aryle mentionnées précédemment comme étant un groupe phényle, qui peut être mono- ou di-substitué respectivement par R', sachant que les substituants peuvent être identiques ou différents, et que
R' représente un atome de fluor, de chlore, de brome ou d'iode, un groupe alkyle en C₁ à C₂, trifluorométhyle ou alcoxy en C₁ à C₂, ou que
deux radicaux R', s'ils sont reliés à des atomes de carbone voisins, représentent conjointement un groupe alkylène en C₃ à C₄, méthylène dioxy ou 1,3-butadièn-1,4-ylène,
leurs tautomères, leurs stéréoisomères et leurs sels.

2. Hétérocycles bicycliques de formule générale I selon la revendication 1, dans lesquels
X représente un atome d'azote,
Rₐ représente un atome d'hydrogène,
R_{b} représente un groupe phényle, benzyle ou 1-phényléthyle, dans lesquels le noyau phényle est substitué respectivement par les radicaux R₁ à R₃, sachant que
R₁ et R₂, qui peuvent être identiques ou différents, représentent respectivement un groupe méthyle ou un atome d'hydrogène, de fluor, de chlore ou de brome, et que
R₃ représente un atome d'hydrogène,
R_{c} représente un atome d'hydrogène,
R_{d} représente un atome d'hydrogène, un groupe méthoxy, éthoxy, cycloalcoxy en C₄ à C₆, (cycloalkyle en C₃ à C₆)-méthoxy, 2-méthoxy-éthoxy, 2-(cyclobutyloxy)-éthoxy, 2-(cyclopentyloxy)-éthoxy, 2-(cyclohexyloxy)-éthoxy, 2-(cyclopropylméthoxy)-éthoxy, tétrahydrofuran-3-yloxy, tétrahydropyran-4-yloxy, tétrahydrofuran-2-ylméthoxy, tétrahydrofuran-3-ylméthoxy, tétrahydropyran-2-ylméthoxy ou tétrahydropyran-4-ylméthoxy,
A représente un groupe 1,2-vinylène,
B représente un groupe méthylène ou éthylène, ou, dans le cas où B est relié à un atome de carbone du groupe C, encore une liaison,
C représente un groupe pyrrolidino, dans lequel les deux atomes d'hydrogène en position 3 sont remplacés par un groupe E, dans lequel
E représente un pont -O-CO-CH₂CH₂-, -CH₂-O-CO-CH₂-, -CH₂CH₂-O-CO-, -CH₂CH₂-O-CO-CH₂-, -O-CO-CH₂-NR₄-CH₂-, -CH₂-O-CO-CH₂-NR₄-, -O-CO-CH₂-O-CH₂- ou -CH₂-O-CO-CH₂-O- éventuellement substitué par un ou deux groupes méthyle,
sachant que R₄ représente un groupe méthyle ou éthyle,
un groupe pipéridino, dans lequel les deux atomes d'hydrogène en position 3 ou en position 4 sont remplacés par un groupe E, sachant que E est défini comme mentionné précédemment,
un groupe pyrrolidino ou pipéridino, dans lesquels deux atomes d'hydrogène vicinaux sont remplacés par un pont -O-CO-CH₂-, -CH₂-O-CO-, -O-CO-CH₂-NR₄- ou -O-CO-CH₂-O- éventuellement substitué par un ou deux groupes méthyle, sachant que R₄ est défini comme mentionné précédemment, et que les hétéroatomes des ponts mentionnés précédemment ne sont reliés ni à la position 2 ou 5 du cycle pyrrolidine, ni à la position 2 ou 6 du cycle pipéridine,
un groupe pipérazino, dans lequel un atome d'hydrogène en position 3, conjointement avec l'atome d'hydrogène en position 4, sont remplacés par un pont -CO-O-CH₂CH₂- ou -CH₂-O-CO-CH₂- éventuellement substitué par un ou deux groupes méthyle, sachant que l'extrémité gauche respective des ponts mentionnés précédemment est reliée à la position 3 du cycle pipérazine,
un groupe pyrrolidino ou pipéridino substitué par le radical R₅, dans lequel
R₅ représente un groupe 2-oxo-tétrahydrofurannyle, 2-oxo-1,4-dioxannyle ou 2-oxo-4-(alkyle en C₁ à C₄)-morpholinyle éventuellement substitué par un ou deux groupes méthyle,
un groupe pyrrolidino substitué en position 3 par un groupe 2-oxo-morpholino, sachant que le groupe 2-oxo-morpholino peut être substitué par un ou deux groupes méthyle,
un groupe pipéridino substitué en position 3 ou 4 par un groupe 2-oxo-morpholino, sachant que le groupe 2-oxo-morpholino peut être substitué par un ou deux groupes méthyle,
un groupe pipérazino substitué en position 4 par le radical R₆, dans lequel
R₆ représente un groupe 2-oxo-tétrahydrofuran-3-yle ou 2-oxo-tétrahydrofuran-4-yle éventuellement substitué par un ou deux groupes méthyle,
un groupe pyrrolidino substitué en position 3 par un groupe (R₄NR₆)-, R₆O-, R₆S-, R₆SO- ou R₆SO₂-, sachant que R₄ et R₆ sont définis comme mentionné précédemment,
un groupe pipéridino substitué en position 3 ou 4 par un groupe (R₄NR₆)-, R₆O-, R₆S-, R₆SO- ou R₆SO₂-, sachant que R₄ et R₆ sont définis comme mentionné précédemment,
un groupe pyrrolidino ou pipéridino substitué par un groupe (R₄NR₆)-(alkyle en C₁ à C₂), HNR₆-CO- ou R₄NR₆-CO-, sachant que R₄ et R₆ sont définis comme mentionné précédemment,
un groupe pyrrolidino substitué en position 3 par un groupe R₅-CO-NH- ou R₅-CO-NR₄-, sachant que R₄ et R₅ sont définis comme mentionné précédemment,
un groupe pipéridino substitué en position 3 ou 4 par un groupe R₅-CO-NH- ou R₅-CO-NR₄-, sachant que R₄ et R₅ sont définis comme mentionné précédemment,
un groupe pipérazino substitué en position 4 par un groupe R₅-(alkyle en C₁ à C₂), R₅-CO-, R₅-(alkylène en C₁ à C₂)-CO-, (R₄NR₆)-(alkylène en C₁ à C₂)-CO-, R₆O-(alkylène en C₁ à C₂)-CO-, R₆S-(alkylène en C₁ à C₂)-CO-, R₆SO-(alkylène en C₁ à C₂)-CO- ou R₆SO₂-(alkylène en C₁ à C₂)-CO-, dans lesquels R₄ à R₆ sont définis comme mentionné précédemment,
un groupe pipérazino substitué en position 4 par un groupe alkyle en C₂ à C₃, dans lequel le groupe alkyle en C₂ à C₃ est substitué à partir de la position 2 respectivement par un groupe (R₄NR₆)-, R₆O-, R₆S-, R₆SO- ou R₆SO₂-, sachant que R₄ et R₆ sont définis comme mentionné précédemment,
un groupe pyrrolidino ou pipéridino substitué par un groupe 2-oxo-morpholino-(alkyle en C₁ à C₂), dans lequel la partie 2-oxo-morpholino peut être substituée par un ou deux groupes méthyle,
un groupe pipérazino substitué en position 4 par un groupe 2-oxo-morpholino-(alkylène en C₁ à C₂)-CO-, dans lequel la partie 2-oxo-morpholino peut être substituée par un ou deux groupes méthyle,
un groupe pipérazino substitué en position 4 par un groupe alkyle en C₂ à C₃, dans lequel la partie alkyle en C₂ à C₃ est substituée à partir de la position 2 respectivement par un groupe 2-oxo-morpholino éventuellement substitué par un ou deux groupes méthyle,
un groupe pipéridinyle substitué en position 1 par le radical R₆, par un groupe R₅-(alkyle en C₁ à C₂), R₅-CO-, R₅-(alkylène en C₁ à C₂)-CO-, (R₄NR₆)-(alkylène en C₁ à C₂)-CO-, R₆O-(alkylène en C₁ à C₂)-CO-, R₆S-(alkylène en C₁ à C₂)-CO-, R₆SO-(alkylène en C₁ à C₂)-CO-, R₆SO₂-(alkylène en C₁ à C₂)-CO-, ou 2-oxo-morpholino-(alkylène en C₁ à C₂)-CO-, sachant que R₄ à R₆ sont définis comme mentionné précédemment, et que la partie 2-oxo-morpholino peut être substituée par un ou deux groupes méthyle,
un groupe pipéridinyle substitué en position 1 par un groupe alkyle en C₂ à C₃, dans lequel la partie alkyle en C₂ à C₃ est substituée à partir de la position 2 respectivement par un groupe (R₄NR₆)-, R₆O-, R₆S-, R₆SO-, R₆SO₂- ou 2-oxo-morpholino, sachant que R₄ et R₆ sont définis comme mentionné précédemment, et que la partie 2-oxo-morpholino peut être substituée par un ou deux groupes méthyle,
un groupe pyrrolidin-3-yl-NR₄-, pipéridin-3-yl-NR₄- ou pipéridin-4-yl-NR₄- substitué au niveau de l'atome d'azote du cycle respectivement par le radical R₆, par un groupe R₅-(alkyle en C₁ à C₂), R₅-CO-, R₅-(alkylène en C₁ à C₂)-CO-, (R₄NR₆)-(alkylène en C₁ à C₂)-CO-, R₆O-(alkylène en C₁ à C₂)-CO-, R₆S-(alkylène en C₁ à C₂)-CO-, R₆SO-(alkylène en C₁ à C₂)-CO-, R₆SO₂-(alkylène en C₁ à C₂)-CO-, ou 2-oxo-morpholino-(alkylène en C₁ à C₂)-CO-, dans lesquels R₄ à R₆ sont définis comme mentionné précédemment, et la partie 2-oxo-morpholino peut être substituée par un ou deux groupes méthyle, ou
un groupe pyrrolidin-3-yl-NR₄-, pipéridin-3-yl-NR₄- ou pipéridin-4-yl-NR₄- substitué au niveau de l'atome d'azote du cycle respectivement par un groupe alkyle en C₂ à C₃, dans lequel la partie alkyle en C₂ à C₃ est substituée à partir de la position 2 respectivement par un groupe (R₄NR₆)-, R₆O-, R₆S-, R₆SO-, R₆SO₂- ou 2-oxo-morpholino, sachant que R₄ et R₆ sont définis comme mentionné précédemment, et que la partie 2-oxo-morpholino peut être substituée par un ou deux groupes méthyle,
leurs tautomères, leurs stéréoisomères et leurs sels.

3. Hétérocycles bicycliques de formule générale I selon la revendication 1, dans lesquels
X représente un atome d'azote,
Rₐ représente un atome d'hydrogène,
R_{b} représente un groupe phényle, benzyle ou 1-phényléthyle, dans lesquels le noyau phényle est substitué respectivement par les radicaux R₁ à R₃, sachant que
R₁ et R₂, qui peuvent être identiques ou différents, représentent respectivement un groupe méthyle ou un atome d'hydrogène, de fluor, de chlore ou de brome, et que
R₃ représente un atome d'hydrogène,
R_{c} représente un atome d'hydrogène,
R_{d} représente un atome d'hydrogène, un groupe méthoxy, éthoxy, cycloalcoxy en C₄ à C₆, (cycloalkyle en C₃ à C₆)-méthoxy, 2-méthoxy-éthoxy, 2-(cyclobutyloxy)-éthoxy, 2-(cyclopentyloxy)-éthoxy, 2-(cyclohexyloxy)-éthoxy, 2-(cyclopropylméthoxy)-éthoxy, tétrahydrofuran-3-yloxy, tétrahydropyran-4-yloxy, tétrahydrofuran-2-ylméthoxy, tétrahydrofuran-3-ylméthoxy, tétrahydropyran-2-ylméthoxy ou tétrahydropyran-4-ylméthoxy,
A représente un groupe 1,2-vinylène,
B représente un groupe méthylène ou éthylène, ou bien, dans le cas où B est relié à un atome de carbone du groupe C, encore une liaison,
C représente un groupe pipéridino, dans lequel les deux atomes d'hydrogène en position 4 sont remplacés par un pont -CH₂-O-CO-CH₂-, -CH₂CH₂-O-CO-, -CH₂CH₂-O-CO-CH₂-, -O-CO-CH₂-NCH₃-CH₂- ou -O-CO-CH₂-O-CH₂-,
un groupe pipérazino, dans lequel un atome d'hydrogène en position 3, conjointement avec l'atome d'hydrogène en position 4, sont remplacés par un pont -CO-O-CH₂-CH₂- ou -CH₂-O-CO-CH₂-, sachant que l'extrémité gauche respective des ponts précédents est reliée à la position 3 du cycle pipérazine,
un groupe pipéridino substitué par un groupe 2-oxo-tétrahydrofurannyle,
un groupe pipéridino, qui est substitué en position 4 par un groupe 2-oxo-morpholino ou 2-oxo-morpholinométhyle, sachant que la partie 2-oxo-morpholino peut être substituée respectivement par un ou deux groupes méthyle,
un groupe pipérazino, qui est substitué en position 4 par un groupe 2-oxo-tétrahydrofuran-3-yle ou 2-oxo-tétrahydrofuran-4-yle,
un groupe pipéridino, qui est substitué en position 4 par un groupe CH₃NR₆- ou R₆S-, sachant que
R₆ représente un groupe 2-oxo-tétrahydrofuran-3-yle ou 2-oxo-tétrahydrofuran-4-yle, un groupe pipérazino, qui est substitué en position 4 par un groupe 2-oxo-tétrahydrofurannylméthyle ou 2-oxo-tétrahydrofurannylcarbonyle,
un groupe pipérazino, qui est substitué en position 4 par un groupe alkyle en C₂ à C₃ à chaîne droite, sachant que la partie alkyle en C₂ à C₃ est substituée en position terminale respectivement par un groupe 2-oxo-tétrahydrofuran-3-ylsulfényle,
un groupe pipéridin-4-yle, qui est substitué en position 1 par un groupe 2-oxo-tétrahydrofuran-3-yle ou 2-oxo-tétrahydrofuran-4-yle, ou
un groupe pipéridin-4-yl-NCH₃-, qui est substitué au niveau de l'atome d'azote du cycle par un groupe 2-oxo-tétrahydrofuran-3-yle, 2-oxo-tétrahydrofuran-4-yle ou 2-oxo-tétrahydrofurannylcarbonyle,
leurs tautomères, leurs stéréoisomères et leurs sels.

4. Hétérocycles bicycliques de formule générale I selon la revendication 1, dans lesquels
X représente un atome d'azote,
Rₐ représente un atome d'hydrogène,
R_{b} représente un groupe 1-phényléthyle ou un groupe phényle substitué par les radicaux R₁ à R₃, sachant que
R₁ et R₂, qui peuvent être identiques ou différents, représentent respectivement un groupe méthyle ou un atome d'hydrogène, de fluor, de chlore ou de brome, et que
R₃ représente un atome d'hydrogène,
R_{c} représente un atome d'hydrogène,
R_{d} représente un atome d'hydrogène, un groupe méthoxy, éthoxy, 2-méthoxy-éthoxy, cyclobutyloxy, cyclopentyloxy, cyclopropylméthoxy, cyclobutylméthoxy, tétrahydrofuran-3-yloxy, tétrahydropyran-4-yloxy ou tétrahydrofuran-2-ylméthoxy,
A représente un groupe 1,2-vinylène,
B représente un groupe méthylène, ou bien, dans le cas où B est relié à un atome de carbone du groupe C, encore une liaison,
C représente un groupe pipéridino, dans lequel les deux atomes d'hydrogène en position 4 sont remplacés par un pont -CH₂-O-CO-CH₂-, -CH₂CH₂-O-CO-, -CH₂CH₂-O-CO-CH₂-, -O-CO-CH₂-NCH₃-CH₂- ou -O-CO-CH₂-O-CH₂-,
un groupe pipérazino, dans lequel un atome d'hydrogène en position 3, conjointement avec l'atome d'hydrogène en position 4, sont remplacés par un pont -CO-O-CH₂-CH₂- ou -CH₂-O-CO-CH₂-, sachant que l'extrémité gauche respective des ponts précédents est reliée à la position 3 du cycle pipérazine,
un groupe pipéridino substitué par un groupe 2-oxo-tétrahydrofurannyle,
un groupe pipéridino, qui est substitué en position 4 par un groupe 2-oxo-morpholino ou 2-oxo-morpholinométhyle, sachant que la partie 2-oxo-morpholino peut être substituée respectivement par un ou deux groupes méthyle,
un groupe pipérazino, qui est substitué en position 4 par un groupe 2-oxo-tétrahydrofuran-3-yle ou 2-oxo-tétrahydrofuran-4-yle,
un groupe pipéridino, qui est substitué en position 4 par un groupe CH₃NR₆- ou R₆S-, sachant que
R₆ représente un groupe 2-oxo-tétrahydrofuran-3-yle ou 2-oxo-tétrahydrofuran-4-yle, un groupe pipérazino, qui est substitué en position 4 par un groupe 2-oxo-tétrahydrofurannylméthyle ou 2-oxo-tétrahydrofurannylcarbonyle,
un groupe pipérazino, qui est substitué en position 4 par un groupe [2-(2-oxo-tétrahydrofuran-3-ylsulfényle) éthyle],
un groupe pipéridin-4-yle, qui est substitué en position 1 par un groupe 2-oxo-tétrahydrofuran-3-yle ou 2-oxo-tétrahydrofuran-4-yle, ou
un groupe pipéridin-4-yl-NCH₃-, qui est substitué au niveau de l'atome d'azote du cycle par un groupe 2-oxo-tétrahydrofuran-3-yle, 2-oxo-tétrahydrofuran-4-yle ou 2-oxo-tétrahydrofurannylcarbonyle,
leurs tautomères, leurs stéréoisomères et leurs sels.

5. Composés suivants de formule générale I selon la revendication 1 :
(a) 4-[(3-chlor-4-fluor-phényle) amino]-6-({4-[4-(2-oxo-tétrahydrofuran-3-yle)-pipérazin-1-yle]-1-oxo-2-butèn-1-yle} amino)-7-cyclopropylméthoxy-chinazoline,
(b) 4-[(3-chlor-4-fluor-phényle) amino]-6-({4-[4-(2-oxo-tétrahydrofuran-4-yle)-pipérazin-1-yle]-1-oxo-2-butèn-1-yle} amino)-7-cyclopropylméthoxy-chinazoline,
(c) 4-[(3-chlor-4-fluor-phényle) amino]-6-{[4-(4-{2-[(2-oxo-tétrahydrofuran-3-yle) sulfanyle]-éthyle}-pipérazin-1-yle)-1-oxo-2-butèn-1-yle] amino}-7-cyclopropylméthoxy-chinazoline,
(d) 4-[(3-chlor-4-fluor-phényle)amino]-6-{[4-(3-oxo-perhydro-pyrazino [2,1-c] [1,4] oxazin-8-yle)-1-oxo-2-butèn-1-yle]amino}-7-cyclopropylméthoxy-chinazoline,
(e) (S)-4-[(3-chlor-4-fluor-phényle)amino]-6-[(4-{4-[(5-oxo-tétrahydrofuran-2-yle) carbonyle]-pipérazin-1-yle}-1-oxo-2-butèn-1-yle)amino]-7-cyclopropylméthoxy-chinazoline,
(f) 4-[(3-chlor-4-fluor-phényle) amino]-6-{[4-(1-oxo-perhydro-pyrazino [2,1-c] [1,4] oxazin-8-yle)-1-oxo-2-butèn-1-yle]amino}-7-cyclopropylméthoxy-chinazoline, et
(g) 4-[(3-chlor-4-fluor-phényle) amino]-6-[(4-{4-[(2-oxo-tétrahydrofuran-3-yle) sulfanyle]-pipéridin-1-yle}-1-oxo-2-butèn-1-yle)amino]-7-cyclopropylméthoxy-chinazoline,
ainsi que leurs sels.

6. Sels physiologiquement acceptables des composés selon les revendications 1 à 5 avec des acides ou des bases inorganiques ou organiques.

7. Médicament, comprenant un composé selon les revendications 1 à 5 ou un sel physiologiquement acceptable selon la revendication 6, plus éventuellement un ou plusieurs supports et / ou agents de dilution inertes.

8. Utilisation d'un composé selon les revendications 1 à 6, pour la préparation d'un médicament qui est approprié au traitement des tumeurs bénignes ou malignes, à la prévention et au traitement des maladies des voies respiratoires et des poumons, au traitement des polypes, des maladies du tractus gastro-intesdnal, des canaux cholédoques et de la vésicule biliaire, ainsi que des reins et de la peau.

9. Procédé de préparation d'un médicament selon la revendication 7, **caractérisé en ce qu'**un composé selon les revendications 1 à 6 est inséré dans un ou plusieurs supports et / ou agents de dilution inertes par voie non chimique.

10. Procédé de préparation des composés de formule générale I selon les revendications 1 à 6, **caractérisé en ce que**
a. un composé de formule générale 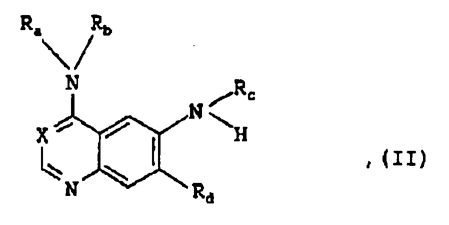 dans laquelle
Rₐ à R_{d} et X sont définis comme mentionné dans les revendications 1 à 5,
est mis à réagir avec un composé de formule générale
**HO-CO - A - B - C,** **(III)**
dans laquelle
A à C sont définis comme mentionné dans les revendications 1 à 5, ou avec des dérivés réactifs de celui-ci
b. un composé, éventuellement formé dans le mélange réactionnel, de formule générale 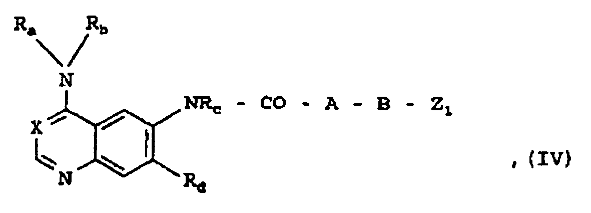 dans laquelle
Rₐ à R_{d}, A, B et X sont définis comme mentionné dans les revendications 1 à 5, et
Z₁ représente un groupe échangeable,
est mis à réagir avec un composé de formule générale
**H - G,** **(V)**
dans laquelle
G représente l'un des radicaux mentionnés pour C dans les revendications 1 à 5, qui est relié par l'intermédiaire d'un atome d'azote au radical B, ou
c. pour la préparation d'un composé de formule générale I, dans laquelle C représente l'un des groupes mentionnés pour C dans les revendications 1 à 5, qui comprend un groupe imino ou HNR₄- substitué par R₆ ou par un groupe R₅-(alkyle en C₁ à C₄), sachant que R₄ à R₆ sont définis comme mentionné dans les revendications 1 à 5, un composé de formule générale 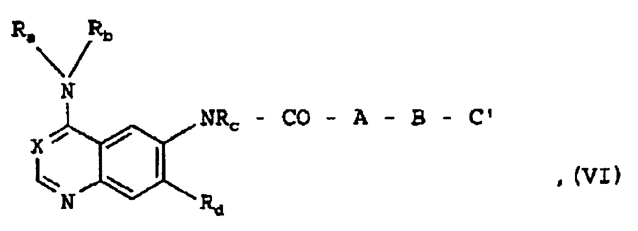 dans laquelle
Rₐ à R_{d}, A et B sont définis comme mentionné dans les revendications 1 à 5, et
C' représente l'un des groupes mentionnés pour C dans les revendications 1 à 5, qui comprend un groupe imino ou HNR₄- non substitué correspondant, sachant que R₄ est défini comme mentionné dans les revendications 1 à 5,
est mis à réagir avec un composé de formule générale
**Z₂ - U,** **(VII)**
dans laquelle
U représente le radical R₆ ou un groupe R₅-(alkyle en C₁ à C₄), sachant que R₅ et R₆ sont définis comme mentionné dans les revendications 1 à 5, et
Z₂ représente un groupe échangeable, ou
Z₂ et un atome d'hydrogène voisin représentent conjointement une autre liaison carbone - carbone, qui est reliée à un groupe carbonyle, ou
d. pour la préparation d'un composé de formule générale I, dans laquelle C représente l'un des groupes mentionnés pour C dans les revendications 1 à 5, qui comprend un groupe imino ou HNR₁- substitué par un groupe R₅CO-, R₅(alkylène en C₁ à C₄)-CO-, (R₄NR₆)-(alkylène en C₁ à C₄)-CO-, R₆O-(alkylène en C₁ à C₄)-CO-, R₆S-(alkylène en C₁ à C₄)-CO-, R₆SO-(alkylène en C₁ à C₄)-CO-, R₆SO₂-(alkylène en C₁ à C₄)-CO- ou 2-oxo-morpholino-(alkylène en C₁ à C₄)-CO-, sachant que R₄ à R₆ sont définis comme mentionné dans les revendications 1 à 5, et que la partie 2-oxo-morpholino peut être substituée par un ou deux groupes alkyle en C₁ à C₂, un composé de formule générale 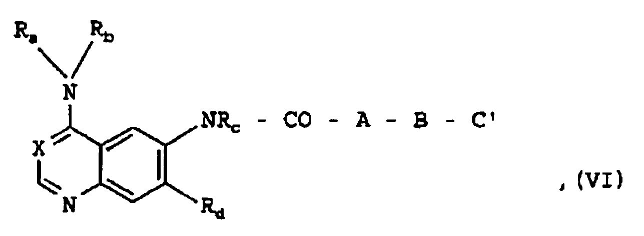 dans laquelle
Rₐ à R_{d}, A et B sont définis comme mentionné dans les revendications 1 à 5, et
C' représente l'un des groupes mentionnés pour C dans les revendications 1 à 5, qui comprend un groupe imino ou HNR₄- non substitué correspondant, sachant que R₄ est défini comme mentionné dans les revendications 1 à 5,
est mis à réagir avec un composé de formule générale
**HO-CO - W,** **(VIII)**
dans laquelle
W représente le radical R₅ ou un groupe R₅-(alkyle en C₁ à C₄), (R₄NR₆)-(alkyle en C₁ à C₄), R₆O-(alkyle en C₁ à C₄), R₆S-(alkyle en C₁ à C₄), R₆SO-(alkyle en C₁ à C₄), R₆SO₂-(alkyle en C₁ à C₄) ou 2-oxo-morpholino-(alkyle en C₁ à C₄), dans lesquels R₄ à R₆ sont définis comme mentionné dans les revendications 1 à 5, et la partie 2-oxo-morpholino peut être substituée par un ou deux groupes alkyle en C₁ à C₂, et
en cas de besoin, un groupement protecteur utilisé lors des réactions décrites précédemment est à nouveau éliminé, et / ou
dans le cas où cela est souhaité, un composé ainsi obtenu de formule générale I est décomposé en ses stéréoisomères, et / ou
un composé ainsi obtenu de formule générale I est converti en ses sels, particulièrement en ses sels physiologiquement acceptables pour l'application pharmaceutique.
